(19) Europäisches Patentamt / European Patent Office / Office européen des brevets

(11) **EP 4 403 184 A1**

(12) **EUROPEAN PATENT APPLICATION**
published in accordance with Art. 153(4) EPC

(43) Date of publication:
**24.07.2024 Bulletin 2024/30**

(21) Application number: **22870034.0**

(22) Date of filing: **15.09.2022**

(51) International Patent Classification (IPC):
**A61K 45/00** *(2006.01)*    **A61P 3/04** *(2006.01)*
**A61K 31/444** *(2006.01)*

(52) Cooperative Patent Classification (CPC):
**A61K 31/444; A61K 45/00; A61P 3/04**

(86) International application number:
**PCT/JP2022/034601**

(87) International publication number:
**WO 2023/042887 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.09.2021   JP 2021150271
12.08.2022   JP 2022128640**

(71) Applicant: **University of The Ryukyus
Nakagami-gun, Okinawa 903-0213 (JP)**

(72) Inventor: **ISHIUCHI Shogo
Nakagami-gun, Okinawa 903-0213 (JP)**

(74) Representative: **Marks & Clerk LLP
15 Fetter Lane
London EC4A 1BW (GB)**

Remarks:
The complete document including Reference Table(s) and the Sequence Listing(s) can be downloaded from the EPO website

(54) **PHARMACEUTICAL COMPOSITION FOR USE IN TREATING COGNITIVE DECLINE, EXCESS WEIGHT, OR OBESITY**

(57)    The present invention provides a pharmaceutical composition for use in treating cognitive decline. The pharmaceutical composition of the present invention comprises an antagonist of an AMPA receptor. The present invention also provides pharmaceutical compositions for use in treating a disease or condition associated with NMDA insensitivity in an NMDA-insensitive subject. The present invention can be useful, for example, in a subject in which intracellular calcium influx by stimulation with NMDA and glycine is inhibited in an NMDA receptor from which inhibition of activation by Mg ions has been released. The present invention further provides a pharmaceutical composition for use in treating overweight and obesity.

EP 4 403 184 A1

**Description**

Technical Field

The present invention relates to a pharmaceutical composition, containing an antagonist of an AMPA receptor, for use in treating cognitive decline. The present invention also relates to a pharmaceutical composition, containing an antagonist of an AMPA receptor, for use in treating a disease or condition associated with an NMDA-insensitive NMDA receptor in a subject having an NMDA-insensitive NMDA receptor. The present invention further provides a pharmaceutical composition for use in treating overweight and obesity.

Background Art

There is evidence that a high-fat diet (HFD) promotes obesity and adversely affects neurocognition due to the worldwide obesity epidemic (1, 2). However, evidence has accumulated that consuming a high-fat diet during adolescence and young adulthood can lead to changes in the function and morphology of the hippocampus, but the mechanism underlying this phenomenon has not yet been elucidated. Also, an attractive target for effective treatment for this phenomenon has not been established.

AMPA receptors (hereinafter also referred to as "AMPAR") are involved in fast-rate excitatory synaptic transmission and play important roles in learning and memory in cooperation with NMDA receptors. In particular, the NMDA receptor (hereinafter also referred to as "NMDAR") is a ligand-dependent ion channel belonging to the ionotropic glutamate receptor family. The NMDA receptor plays an important role in excitatory synaptic transmission, learning, and memory. The AMPA receptor is expressed on the postsynaptic membrane and induces hyperpolarization in the order of milliseconds in the presence of glutamic acid (or AMPA), but does not have calcium permeability. The NMDA receptor is expressed on the postsynaptic membrane and the non-synaptic region, and is activated in the presence of glycine and glutamic acid (or NMDA) accompanying depolarization of the cell membrane to allow cations including calcium ions to flow into the cell. The NMDA receptor normally binds magnesium ions and is inactivated. When the AMPA receptor is depolarized and the membrane potential of the membrane in which the NMDA receptor is present increases, the magnesium block of the NMDA receptor is released. This makes the NMDA receptor responsive to glycine and glutamic acid, and following the depolarization of the AMPA receptor, the NMDA receptor depolarizes and increases the intracellular calcium concentration, which can generate a signal in the postsynaptic membrane.

Hippocampus has the lowest firing threshold for stimulation in the brain, and plays an important role in acquisition of new memory and learning. For example, it is known that Alzheimer's dementia starts from a disorder of the entorhinal cortex (EC) and is caused by a decrease in hippocampal function. Also, the low firing threshold can cause epilepsy. Perampanel is commercially available as an AMPA receptor antagonist, and is used for treatment of epilepsy.

Summary of Invention

The present invention provides a pharmaceutical composition for use in treating cognitive decline.

The present inventors have found a new phenomenon in which calcium permeability of an AMPA receptor in the presence of AMPA increases, while activation of an NMDA receptor in the presence of NMDA is inhibited in the hippocampus. The inventors have also found that this new phenomenon occurs in overweight and obese subjects, as well as subjects with cognitive dysfunction. More specifically, in the hippocampus of a healthy subject, the AMPA receptor is non-permeable to calcium, whereas in Examples to be described later, in the hippocampus of a subject exhibiting overweight and obesity, it was revealed that the calcium permeability of the AMPA receptor in the presence of AMPA increased, and the activation of the NMDA receptor in the presence of NMDA was inhibited, and it was shown that the control of the AMPA receptor and the NMDA receptor in the hippocampus of the subject was abnormal. Subjects exhibiting overweight and obesity had certain cognitive decline. It was revealed that calcium permeability of the AMPA receptor in the presence of AMPA also increases in the hippocampus of a subject having Alzheimer's dementia, and activation of the NMDA receptor in the presence of NMDA was inhibited. It was revealed that the inhibition of the NMDA receptor from being activated under the conditions to be activated may cause cognitive dysfunction in overweight and obese subjects as well as subjects with cognitive dysfunction. The inventors also succeeded in restoring the NMDA-responsive channel activation capacity of NMDA receptors by applying AMPA receptor agonists to the hippocampus of these subjects. In the above subject, the control of the AMPA receptor and the NMDA receptor in the hippocampus is abnormal, and inhibition of the NMDA receptor due to hyperexcitability of the AMPA receptor (particularly, increase in calcium permeability) is involved in cognitive dysfunction. Therefore, by inhibiting intracellular influx of calcium via the AMPA receptor using the AMPA receptor antagonist, it is possible to induce release of the inhibition of the NMDA receptor (that is, NMDA receptor activation).

According to the present invention, for example, the following invention is provided.

[1] A pharmaceutical composition for use in treating cognitive dysfunction in a subject, containing an AMPA receptor antagonist.

[2] The pharmaceutical composition according to above [1], in which the subject has an NMDA receptor inhibited in at least one or more regions of a hippocampus body selected from the group consisting of an entorhinal cortex (EC), CA1, CA2, CA3, and a dentate gyrus (DG), thereby inhibiting NMDA receptor activity by NMDA in at least one or more regions of the hippocampus, and the subject has cognitive dysfunction.

[3] The pharmaceutical composition according to above [2], wherein the subject has a calcium-permeable AMPA receptor in at least one or more regions of the hippocampus body, whereby activation of an NMDA receptor is inhibited in at least one or more regions of the hippocampus.

[4] The pharmaceutical composition according to any of above [1] to [3], in which the subject is a subject having cognitive dysfunction.

[5] The pharmaceutical composition according to above [4], in which the cognitive dysfunction is Alzheimer's dementia.

[6] The pharmaceutical composition of any of above [1] to [5], in which the subject has a body mass index (BMI) of 25 or greater.

[7] The pharmaceutical composition according to any of above [1] to [6], in which the subject has a BMI of 30 or greater.

[8] The pharmaceutical composition according to any of above [1] to [7], in which the subject shows a change of 1.1 fold or more in blood oxygenation level dependent (BOLD) response during an event-related memory task, compared to that during resting state.

[9] A pharmaceutical composition for use in treating a disease or condition associated with NMDA insensitivity in an NMDA-insensitive subject or a subject with NMDA-insensitive NMDA receptors, the pharmaceutical composition containing an AMPA receptor antagonist.

[10] The pharmaceutical composition according to any of above [1] to [9], wherein the AMPA receptor antagonist is perampanel.

According to the present invention, for example, the following invention is provided.

[1A] A pharmaceutical composition for use in treating overweight or obesity in a subject with overweight or obesity, the pharmaceutical composition containing an AMPA receptor antagonist.

[2A] The pharmaceutical composition according to above [1A] for use in delaying, inhibiting, or stopping the development of overweight or obesity in a subject with overweight or obesity.

[3A] The pharmaceutical composition according to above [1A] for use in reducing body weight in a subject with overweight or obesity.

[4A] The pharmaceutical composition according to above [1A] or [3A] for use in reducing food intake in a subject with overweight or obesity.

[5A] The pharmaceutical composition according to any of above [1A] to [4A], for use in inhibiting the onset of diabetes, dyslipidemia, and cardiovascular disease in a subject with overweight or obesity.

[6A] The pharmaceutical composition according to any of above [1A] to [5A], wherein the AMPA receptor antagonist is perampanel.

Brief Description of Drawings

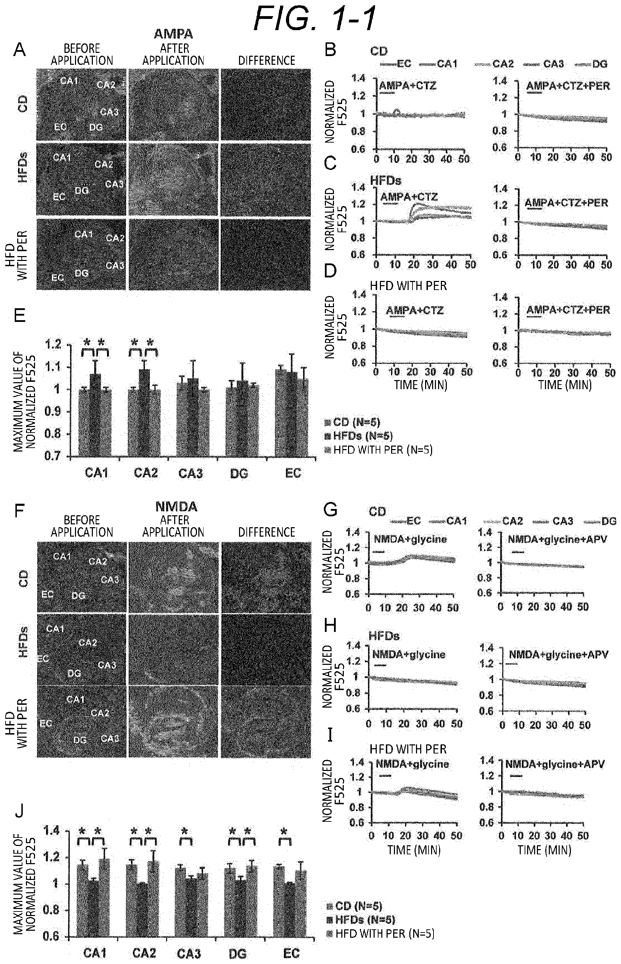

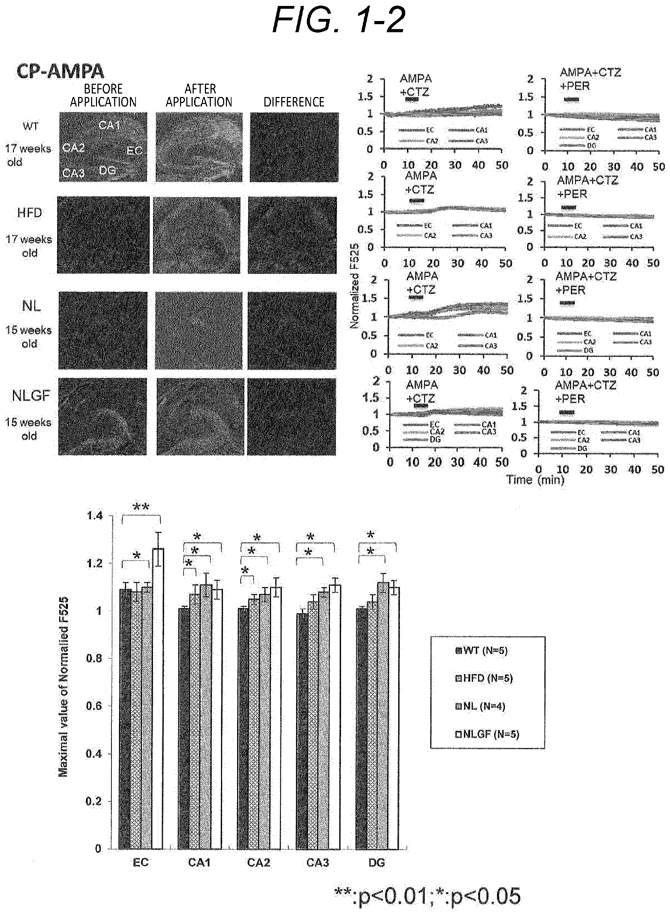

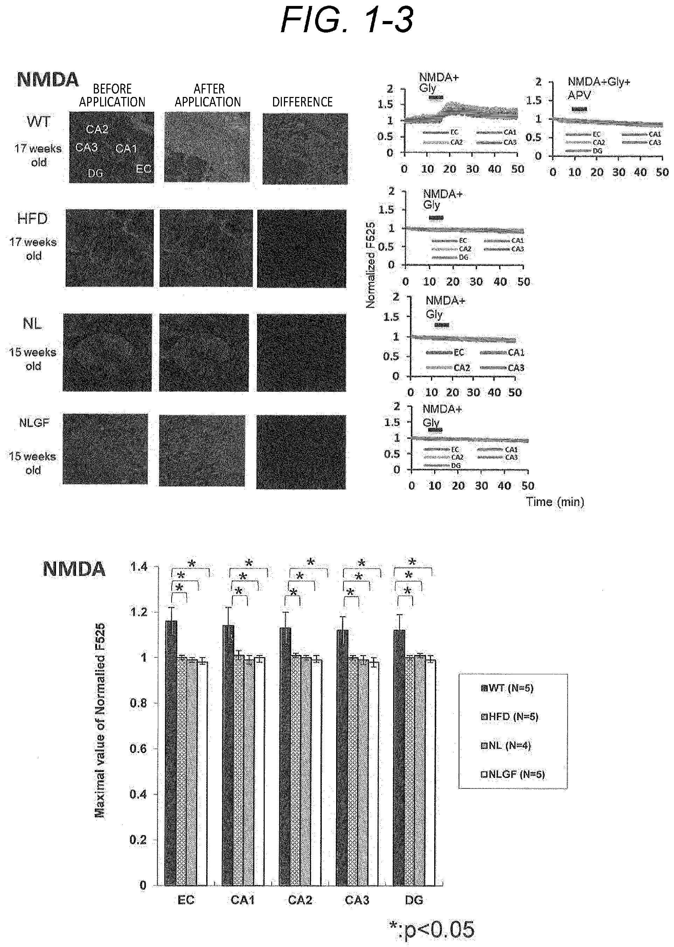

Fig. 1-1 shows that in a hippocampus of a model mouse having obesity-induced cognitive decline, NMDA receptors are inhibited even in the presence of an agonist, AMPA receptors have calcium permeability in the presence of an agonist, and the perampanel (PER), which is an AMPA receptor antagonist, restores the interaction of AMPAR and NMDAR in the hippocampus of an HFD-fed mouse. Panel A shows Fluo-3 fluorescence images of the hippocampus before and after administration when AMPA (AMPA receptor agonist) and CTZ (uncoupling inhibitor) are administered in combination in a CD-fed group, an HFD-fed group, and a PER administration + HFD-fed group (hereinafter may be referred to as an "HFD group with PER"). The right column of Panel A is an image showing the difference in the image after administration from the image before administration. Mice were 17 weeks old, and with respect to the HFD-fed group and the HFD-fed group with PER, HFD feeding or HFD feeding with PER was performed from 8 to 17 weeks of age. Panels B to D show AMPA-dependent changes in normalized F525 (NF525) in hippocampal CA1, CA2, CA3, DG, and EC regions. The F525 signal was measured under the conditions of the CD-fed group (Panel B), the HFD-fed group (Panel C) and PER-administered HFD-fed group (Panel D), respectively. Panel E shows the average values of the maximum values of NF 525 after application of AMPA obtained from different slice specimens (N = 5) (*: $p < 0.05$, two tailed t-test). Panel F shows Fluo-3 fluorescence images of the hippocampus before and after application of NMDA and glycine and the difference images thereof in the HFD-fed group administered with CD, HFD, and PER. Panels G to I show NMDA-dependent changes in NF525 in hippocampal CA1, CA2, CA3, DG, EC regions. The F525 signal was measured under each condition of CD (Panel G), HFD (Panel H), and HFD with PER treatment (Panel I). Panel J shows the average maximum value of NF525 after NMDA application obtained from different slice specimens (N = 5) (**: $p < 0.01$; *: $p < 0.05$, two tailed t-test).

Fig. 1-2 shows that in the hippocampus of Alzheimer's dementia model mice (NL and NLGF), the AMPA receptor has calcium permeability in the presence of an agonist, and that the perampanel (PER), an AMPA receptor antagonist, restores the calcium permeability of the AMPA receptor in the hippocampus of HFD-fed mice.

Fig. 1-3 shows that in the hippocampus of Alzheimer's dementia model mice (NL and NLGF), NMDA receptors are inhibited even in the presence of an agonist.

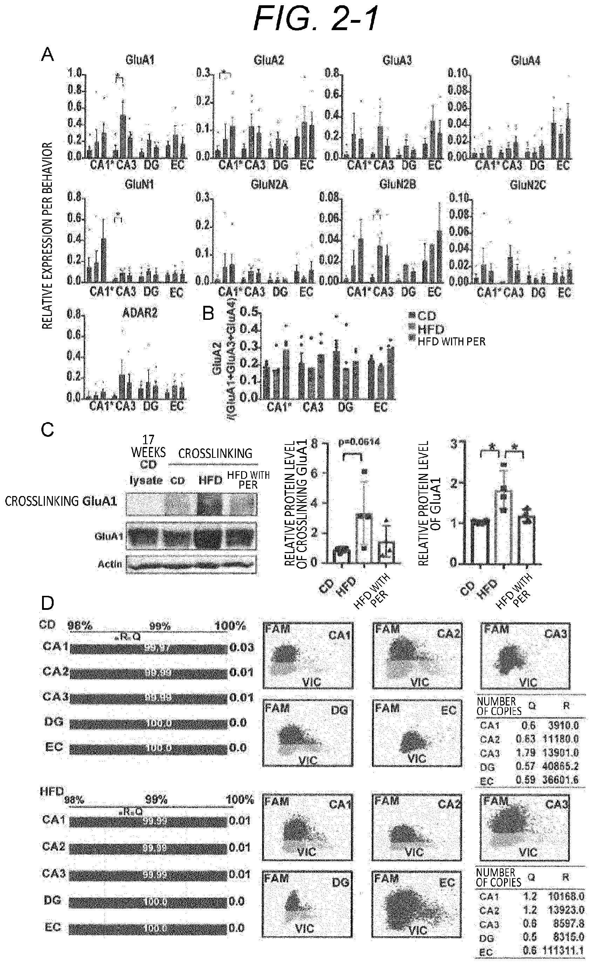

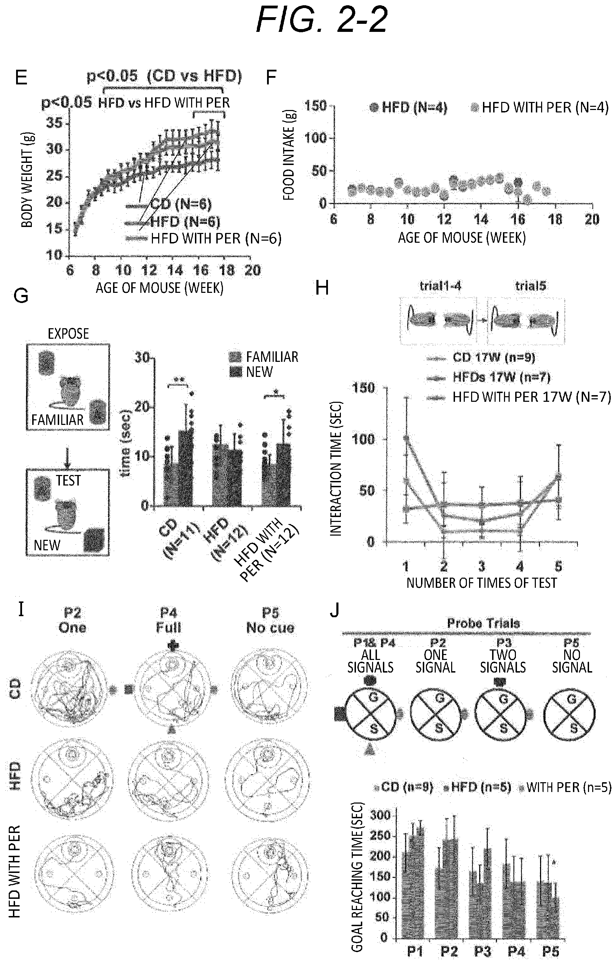

Fig. 2-1 shows the results of transcriptional profiling, expression, proportion of Q/R editing, and behavior analysis of AMPAR and NMDAR in the hippocampal region in the HFD-fed group. Panel A shows the expression levels by qRT-PCR of an AMPAR subunit, an NMDAR subunit, and ADAR2 in the hippocampal region (CA1, CA3, DG, EC) of the CD-fed group, the HFD-fed group, and HFD-fed group with PER. Mice were 17 weeks old, and with respect to the HFD-fed group and the HFD-fed group with PER, HFD feeding or HFD feeding with PER was performed from 8 to 17 weeks of age. Panel B shows the $Ca^{2+}$ permeability index of the hippocampal region (CA1, CA3, DG, EC) of the CD-fed group, the HFD-fed group, and the HFD-fed group with PER. Panel C shows that cell surface (middle column) and intracellular (right column) GluA1 protein expression in the hippocampus CA3 region of the HFD-fed group was increased and decreased in the HFD-fed group with PER, respectively. Panel D shows the analysis results of Q/R editing in the hippocampal region (CA1, CA2, CA3, DG, EC) of the CD-fed group and the HFD-fed group. Mice were 17 weeks old, and with respect to the HFD-fed group and the HFD-fed group with PER, HFD feeding or HFD feeding with PER was performed from 6 to 17 weeks of age. VIC: VIC-labeled probe detecting non-edited (Q) sequence, FAM: FAM-labeled probe detecting edited sequence (R). The left bar graph shows the ratio (%) of R-type and Q-type.

Fig. 2-2 is a diagram following Fig. 2-1. Panel E shows the change in body weight of the CD-fed group, the HFD-fed group, and the HFD-fed group with PER (N = 6). Mice were 6 to 17 weeks old, and with respect to the HFD-fed group and the HFD-fed group with PER, HFD feeding or HFD feeding with PER was performed from 6 to 17 weeks of age. Panel F shows the food intake of each of the HFD-fed group and the HFD-fed group with PER (N = 4). Panel G shows the average search time for with a familiar object and a new object in the CD-fed group (N = 11), the HFD-fed group (N = 12), and the fed group with PER (N = 12). The mark overlapping the bar graph indicates data of each individual. Mice were 12 weeks old, and with respect to the HFD-fed group and the HFD-fed group with PER, HFD feeding or HFD feeding with PER was performed from 4 to 12 weeks of age. Panel H shows the interaction time (seconds) in a five-trial social interaction test on each day of the test in the CD-fed group, the HFD-fed group, and the HFD-fed group with PER. Mice were 16 weeks old, and with respect to the HFD-fed group and the HFD-fed group with PER, HFD feeding or HFD feeding with PER was performed from 8 to 16 weeks of age. Data are shown as average values $\pm$ SD (*: $p < 0.05$; **: $p < 0.01$, two tailed t-test). Panels I and J show a representative example of the swimming path (the end of the track represents the starting point) to the phantom platform (left of the circle) of the probe trial (Panel I) and the results of the probe trial with the cue operation (Panel J). Panel K shows the goal reaching time of the Morris water maze pattern completion test by test day in the CD-fed group, the HFD-fed group, and the HFD-fed group with PER. Mice were 17 weeks old, and with respect to the HFD-fed group and the HFD-fed group with PER, HFD feeding or HFD feeding with PER was performed from 6 to 17 weeks of age.

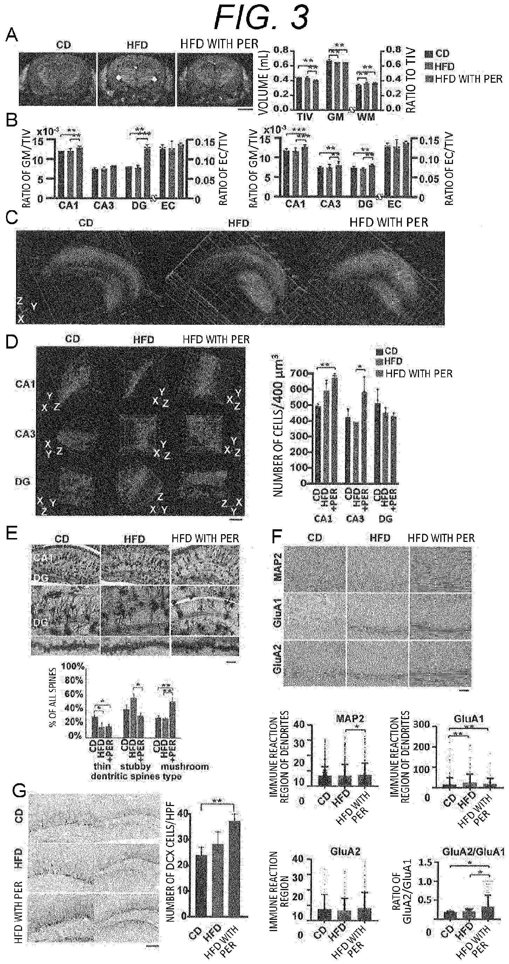

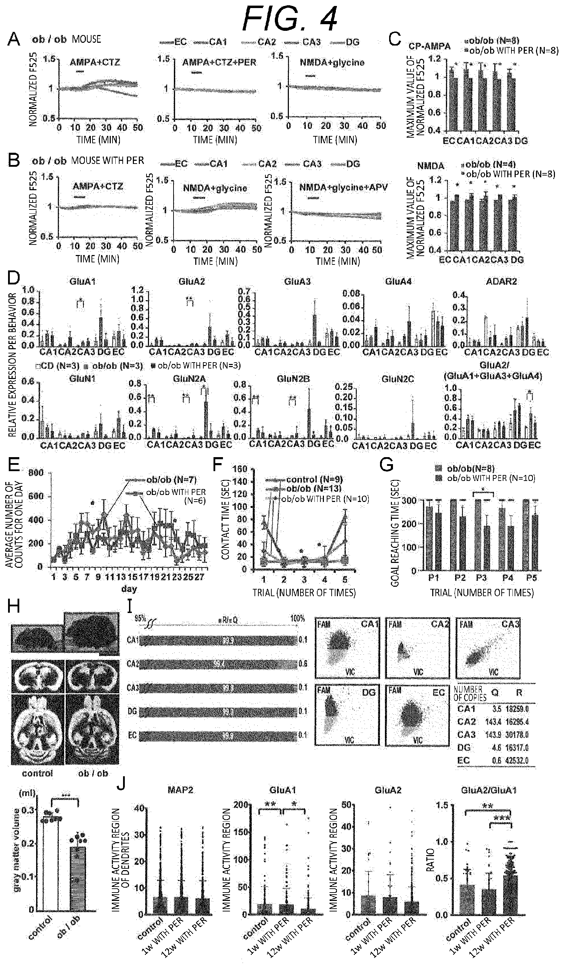

Fig. 3 shows morphological changes of hippocampus in HFD-fed group and HFD-fed group with PER. Panel A shows a coronal cross-sectional view of the brains of the BL/6 (16 weeks old), HFD-fed group and HFD-fed group with PER. Coronal cross-sectional views were acquired by MRI. White asterisks and diamonds indicate that the third lateral ventricle and bilateral lateral ventricles are dilated in the HFD-fed group. The right bar graph shows the mean volume of total intracranial volume (TIV), total gray matter (GM), and total white matter (WM), and the ratio of total grey matter (GM), and total white matter (WM) to TIV. The left Y axis shows the volume of TIV (mL), and the right Y axis shows the volume ratio of GM or WM to TIV, respectively. Panel B shows the average volume of GM per TIV of hippocampi CA1, CA3, DG, and EC. Left and right bar graphs show the results of left and right hippocampi, respectively. The left Y axis shows the ratio of GM to TIV of CA1, CA3, and DG (GM/TIV), and the right Y axis shows the ratio of GM to TIV of EC (GM/TIV). Panels C and D show 3D images of the hippocampus of Thy-1 mice (17 weeks old). The frame of the cubic box in Panel C has a length of 4000 $\mu$m, a width of 3000 um, and a height of 3000 um, and the length of each side of the small cubic is 400 $\mu$m. Panel D shows the results of observing CA1, CA3, and DG in the treatment conditions of the BL/6, the HFD-fed group (feeding from 4 to 17 weeks of age), and HFD-administered group with PER (PER administration and feeding from 4 to 17 weeks of age). The right bar graph shows the cell density of Thy-1[+]-FYP mouse hippocampus CA1, CA3, and DG regions. Panels E to G show Golgi staining of the CA1 and DG regions (Panel E, upper stage) and percentage of thin, stubby, and mushroom spine types (Panel E, lower stage); immunostaining with anti-MAP2 Ab (antibody), anti-GluA1 Ab, and anti-GluA2 Ab (Panel F, upper stage); immunoreaction area of anti-MAP2 antibody, anti-GluA1 antibody, and anti-GluA2 antibody in hippocampus DG region and GluA2/GluA1 ratio (Panel F, bottom column); anti-DCX+ staining (G, left field), DCX+ positive cell count/HFP (G, right column) in hippocampal DG region of BL/6, HFD-fed group, fed group with PER. Bars: 200 um (E up), 100 um (E in), 1 um (E down), 50 um (F), and 100 um (G left), 50 um (G left). Mice were 17 weeks old, and with respect to the HFD-fed group and the HFD-fed group with PER, HFD feeding or HFD feeding with PER was performed from 4 to 17 weeks of age. *, **, *** represent $p < 0.05$, $p < 0.01$, and $p < 0.001$, respectively. Fig. 4 shows the interaction of calcium-permeable (CP)-AMPA receptor and NMDA receptor, behavioral function, and brain volume in ob/ob mice. Panel A shows the function (calcium transport capacity) of an AMPA receptor and an NMDA receptor in the EC, CA1, CA2, CA3, and DG regions of obese mice (12 weeks old). An increase in normalized 525 by AMPA + CTZ administration means that the AMPA receptor has become calcium permeable, and no change in normalized 525 by NMDA + glycine administration means that the NMDA receptor is inhibited. Panel B shows the function of the AMPA receptor and the NMDA receptor in the EC, CA1, CA2, CA3, and DG regions of ob/ob mice (12 weeks old) receiving PER from 8 to 12 weeks of age. No variation in normalized 525 with AMPA + CTZ administration means that the AMPA receptor is calcium impermeable, and an increase in normalized 525 with NMDA + glycine administration means that inhibition of the NMDA receptor is released. Panel C is a graph of the function of the AMPA receptor and the NMDA receptor in the EC, CA1, CA2, CA3, and DG regions of ob/ob mice and ob/ob mice administered PER. In Fig. 4, the respective factors were used at the following concentrations. AMPA: 100 $\mu$M; CTZ: 100 $\mu$M; NMDA: 50 $\mu$M; PER: 100 $\mu$M; Glycine: 10 $\mu$M; and APV: 50 $\mu$M. Panel D shows RNA expression (qRT-PCR analysis results of AMPAR subunit, NMDAR subunit, ADAR, REST, RP 58) in BL6/J (N = 3, left bar) (12 weeks old), ob/ob (N = 3, middle bar) (12 weeks old), and ob/ob mice receiving PER (N = 3, right bar) (12 weeks old). The lower right Panel of Panel D shows the $Ca^{2+}$ permeability index of the hippocampal regions (CA1, CA2, CA3, DG, EC) of BL6/J mice, ob/ob mice, ob/ob mice receiving PER (the column at the lower right end). Panels E to G show the results of behavior analysis of ob/ob mice and ob/ob mice that received PER. Panel E shows the results of the running-wheel activity test, Panel F shows the results of the five-trial social memory assay, and Panel G shows the results of the pattern completion navigation task. Mice were 11 weeks old and PER administrations were performed from 6 weeks to 11 weeks. Data are presented as mean $\pm$ SE. Panel H shows the appearance of BL6 and ob/ob mice (top), grey matter images segmented from T1-weighted images of BL6 and ob/ob mice (middle), and grey matter volumes of BL6 mice and ob/ob mice (bottom). Panel I shows the results of a Q/R editing analysis of the hippocampal region (CA1, CA2, CA3, DG, EC) of ob/ob mice (mouse age: 12 weeks, PER administration: from 6 weeks to 12 weeks). The bar graph shows the percentage of molecules in the edited and non-edited GluA2 subunits. VIC: VIC-labeled probe detecting non-edited sequence (Q), FAM: FAM-labeled probe detecting edited sequence (R). Panel J shows the immune reaction area and GluA2/GluA1 ratio of anti-MAP2 Ab (antibody), anti-GluA1 Ab, anti-GluA2 Ab in the hippocampus DG region of ob/ob mice, ob/ob (1 w with PER) mice that received PER for 1 week, and ob/ob (12 w with PER) mice that received PER for 12 weeks. *: $p < 0.05$, ***: $p < 0.001$.

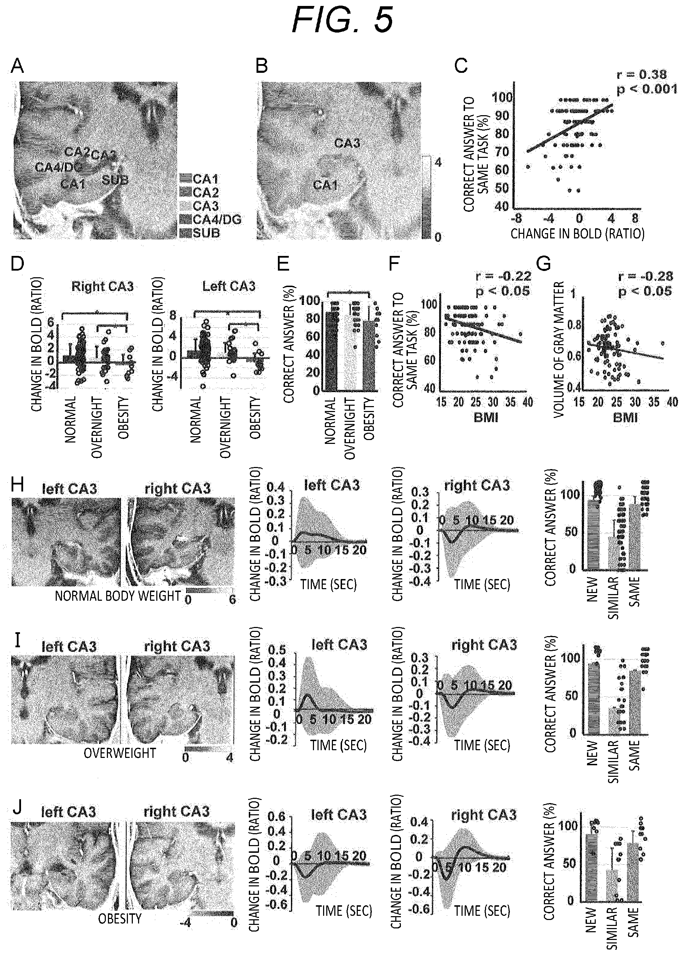

Fig. 5 shows an influence of an increase in BMI on fluctuations of human memory functions and synaptic functions. Panel A shows an anatomical view of a subregion of the right hippocampus in a T2-weighted image. CA1 to CA3, CA4/DG, and cerebellum (SUB) are displayed. Panel B shows the correlation of activation of CA3 and CA1 in the right hippocampus in the recognition task (normal body weight [n = 84], overweight [n = 27], obesity [n = 11]). Panels C and D show the correlation between the ratio value of the BOLD signal of the right hippocampus CA3 and the correct answer rate of the recognition task. Panel D shows percentage values of the BOLD signal in the bilateral hippocampus CA3 region (males: 52, females: 65, total: 117, average age = 37.8 $\pm$ 19.6 years, range: 18 to 86 years). A Panel E shows an average value of correct answer rates in the recognition task. The mark is data of each individual. Panel F shows the correlation between BMI and the correct answer rate in the recognition task (r = -0.22, $p < 0.05$), and Panel G shows the correlation between BMI and the whole brain grey matter volume (r = -0.28, $p < 0.05$). Panels H to J show the activation maps and the BOLD signal change of left and right CA3, and the results of the correct answer rate from left to right of the hippocampus memory task (New, Lure, Same) in the normal body weight group (H), the overweight group (I), and the obese group (J). Color bar: T value. *: $p < 0.001$.

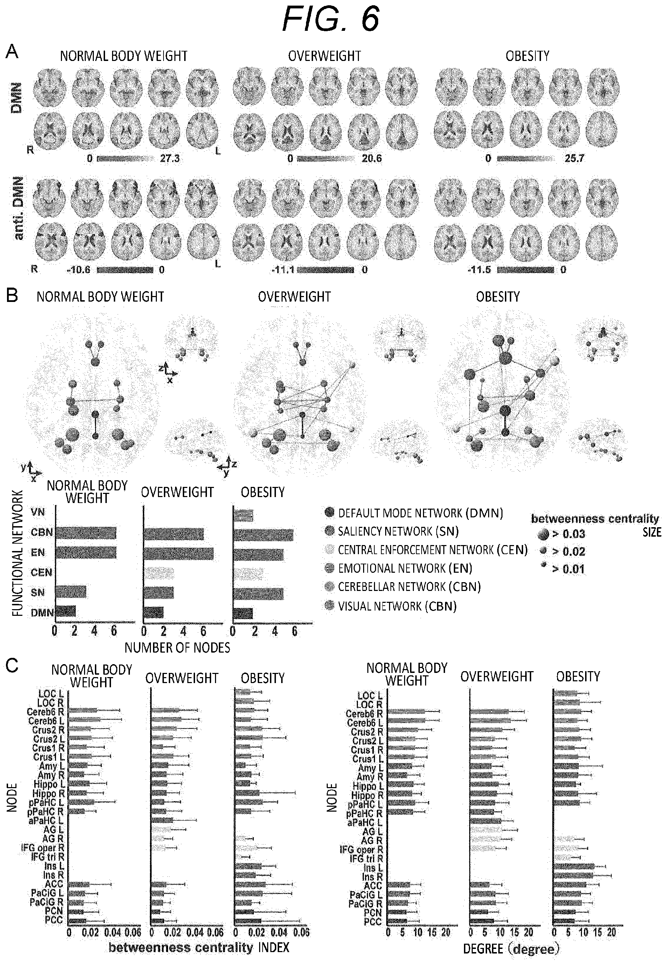

Fig. 6 shows a relationship between network connectivity and BMI in humans. Panel A shows DMN maps(top) and anti-correlation maps to DMN (bottom) of the normal body weight group, the overweight group, and the obese group . The color bar indicates the T value. Panel B shows functional network connectivity for the normal body weight group (left column), the overweight group (middle column), the obese group (right column). The diameter of each circle indicates the value of betweenness centrality. Panel C shows the average value and the standard deviation of betweenness centrality and degree of each node. a.u.: arbitrary unit.

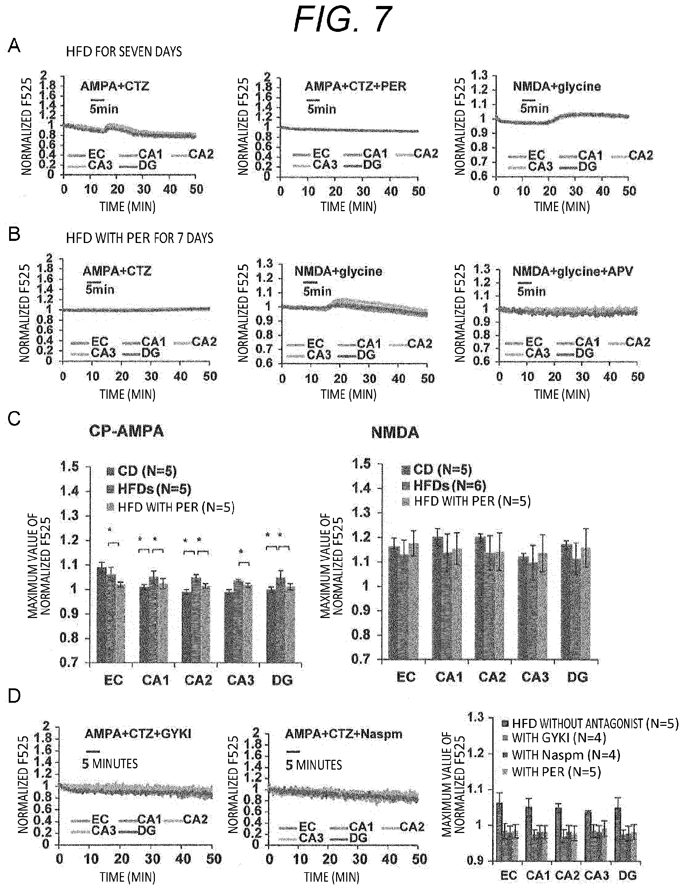

Fig. 7 shows functional changes of the AMPA receptor and the NMDA receptor in the HFD-fed group. Panel A shows functional changes (calcium transport capacity) of the AMPA receptor and the NMDA receptor in the EC, CA1, CA2, CA3, and DG regions in HFD-fed mice (7 days from 16 to 17 weeks of age). Panel B shows the function of the AMPA receptor and the NMDA receptor in the EC, CA1, CA2, CA3, and DG regions in HFD-fed group with PER (7 days from 16 to 17 weeks of age). Panel C is a graph showing the function of the AMPA receptor and the NMDA receptor in the EC, CA1, CA2, CA3, and DG regions in the CD-fed group, the HFD-fed group, and the HFD-fed group with PER. *: $p < 0.05$, t-test. Panel D shows the inhibitory effect of 1-naphthylacetylspermine (Naspm) (a selective antagonist of the AMPA receptor) and GYKI (a selective non-competitive AMPA receptor antagonist) in the HFD-fed groups (7 days of HFD feeding). AMPA: 100 $\mu$M, CTZ: 100 $\mu$M, NMDA: 50 $\mu$M, Glycine: 10 $\mu$M.

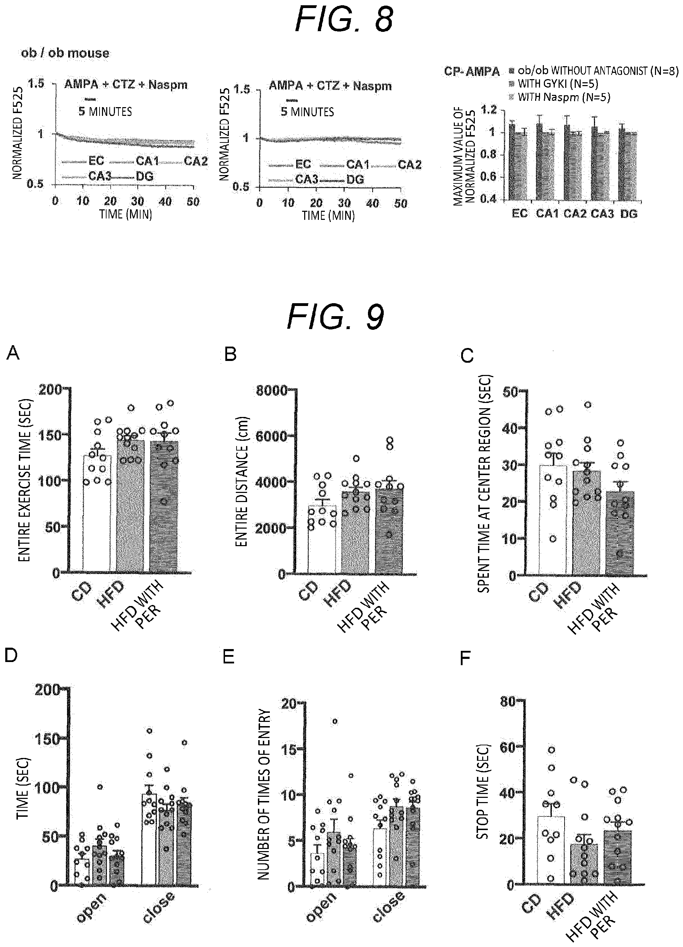

Fig. 8 shows the inhibitory effect of Naspm ($Ca^{2+}$-permeable AMPA receptor [CP-AMPAR] antagonist) and GYKI (selective non-competitive AMPAR antagonist) in the hippocampus of ob/ob mice (12 weeks old). AMPA: 100 $\mu$M; CTZ: 100 $\mu$M; Naspm: 20 $\mu$M; GYKI: 100 $\mu$M. *: $p < 0.05$, t-test.

Fig. 9 shows results of behavior analysis of an open field test (Panels A to C), an elevated plus maze test (Panels D and E), and a contextual fear conditioning test (Panel F). The total travel time (Panel A), the total distance (Panel B), the residence time in the middle (Panel C), the average value of the residence time (Panel D), the average value of the number of times of entry into the open arm and the closed arm (Panel E), and the average value of the stop time (F) for the CD-fed group (N = 11), the HFD-fed group (N = 12), the HFD-fed group with PER (N = 12) are shown. Mice were 12 weeks old, and with respect to the HFD-fed group and the HFD-fed group with PER, HFD feeding and the HFD feeding with PER are performed from 4 to 12 weeks of age. Data are shown as the average value $\pm$ SD. Marks overlapping the bar graph indicate individual data.

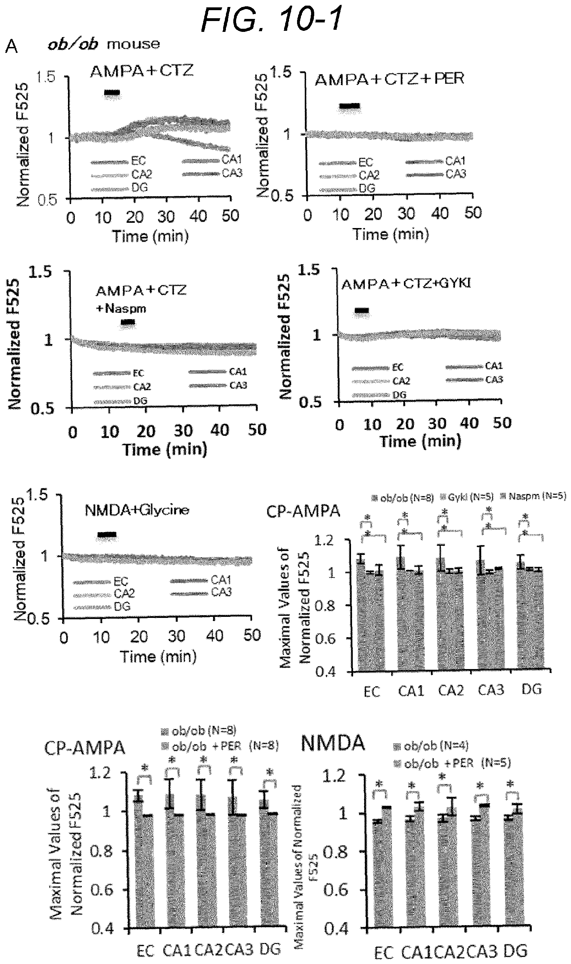

Fig. 10-1 shows (A) the effect of an AMPA receptor antagonist on the function of the CP-AMPA receptor and the NMDA receptor in the EC, CA1, CA2, CA3, and DG regions of ob/ob mice. AMPA: 100 $\mu$M; CTZ: 100 $\mu$M; NMDA:50 $\mu$M; PER: 100 $\mu$M; Glycine: 10 $\mu$M; APV: 50 $\mu$M; Naspm: 10 $\mu$M; GYKI: 100 $\mu$M; Leptin: 100nM *: $p < 0.05$, t-test.

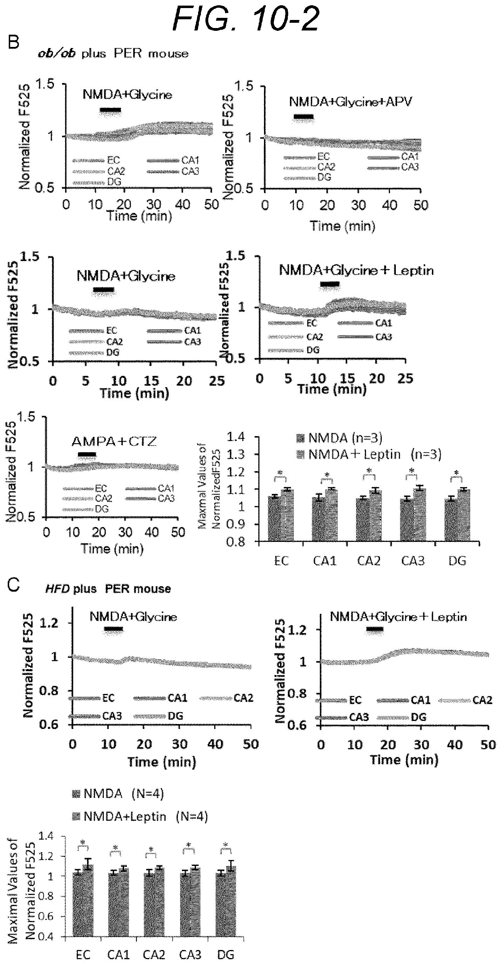

Fig. 10-2 shows (B) the effect of Leptin on NMDA receptor function in the EC, CA1, CA2, CA3, and DG regions of ob/ob mice with PER and (C) the effect of Leptin on NMDA receptor function in the EC, CA1, CA2, CA3, and DG regions of the HFD-fed group with PER. AMPA: 100 $\mu$M; CTZ: 100 $\mu$M; NMDA:50 $\mu$M; PER: 100 $\mu$M; Glycine: 10 $\mu$M; APV: 50 $\mu$M; Naspm: 10 $\mu$M; GYKI: 100 $\mu$M; Leptin: 100nM *: $p < 0.05$, t-test.

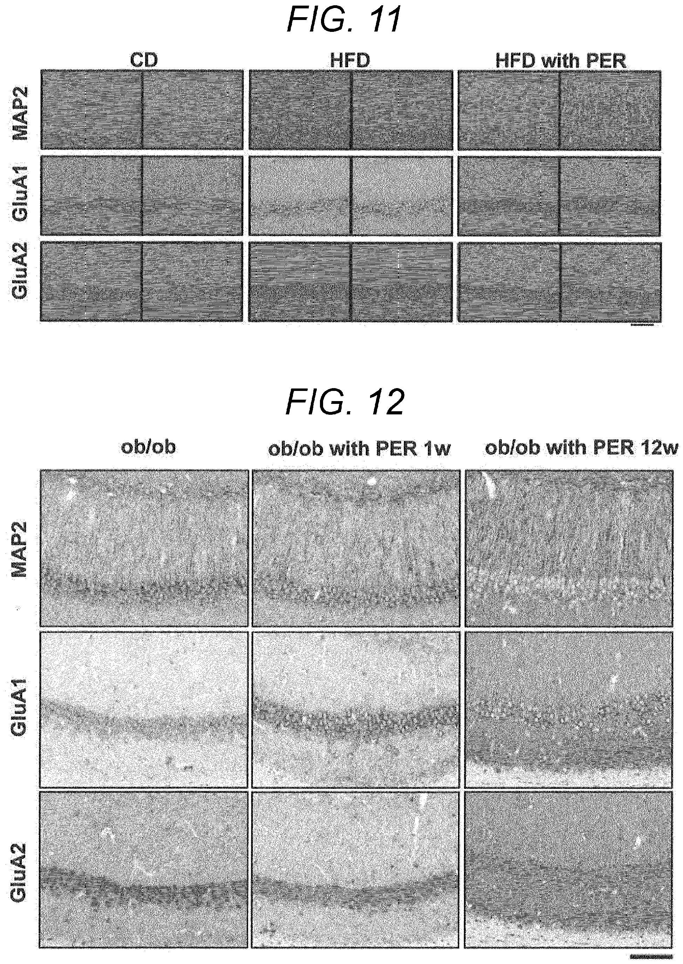

Fig. 11 shows the results of quantitative analysis of the expression levels of MAP2, GluA1, and GluA2 in the CD-fed group, the HFD-fed group, and the HFD-fed group with PER. The green-painted parts were segmented using Zeiss Intellisation image software to show immunopositive regions of MAP2, GluA1, and GluA2. Bar: 100 $\mu$m. Mice were 17 weeks old and PER administrations were performed between 6 and 17 weeks.

Fig. 12 shows immunostaining images of anti-MAP2 Ab, anti-GluA1 Ab, and anti-GluA2 Ab in ob/ob mice and ob/ob mice with PER administration for 1 week and 12 weeks. Bar: 100 $\mu$m. Mice were 17 weeks old and PER administrations were performed between 6 and 17 weeks.

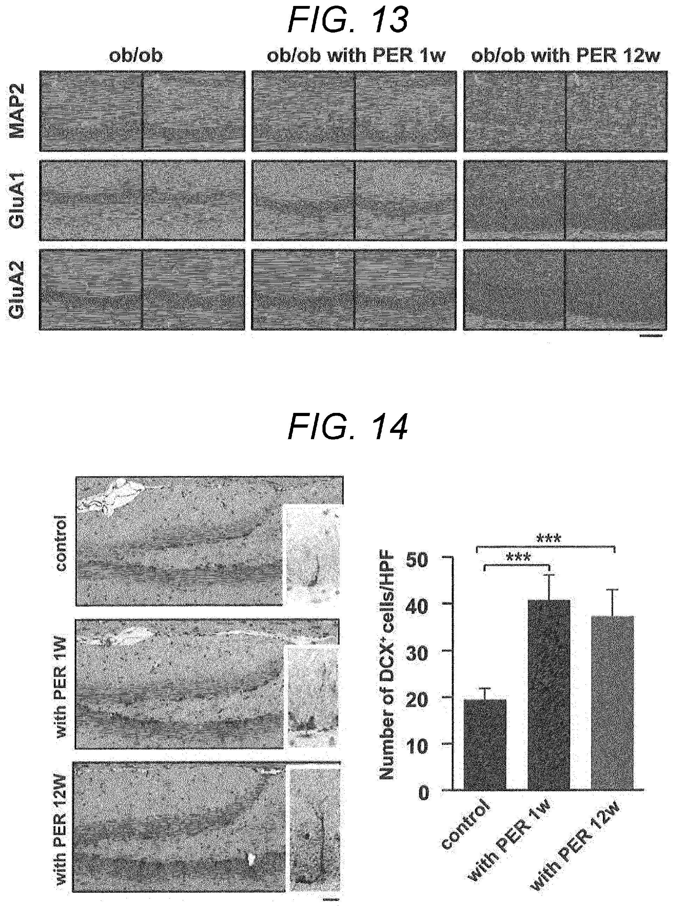

Fig. 13 shows the results of quantitative analysis of the expression of MAP2, GluA1, and GluA2 in ob/ob mice and ob/ob mice with PER administration for 1 week and 12 weeks. Bar: 100 $\mu$m. Mice were 17 weeks old and PER administrations were performed between 6 and 17 weeks.

Fig. 14 shows results of anti-DCX+ staining and DCX+ positive cell number/HFP in the hippocampus DG region of ob/ob mice and ob/ob mice with PER administration for 1 week and for 12 weeks. Inset shows DCX+ neuronal progenitor cells. Bar: 100 um and 20 um (inset). Mice were 17 weeks old and PER administrations were performed between 6 and 17 weeks.

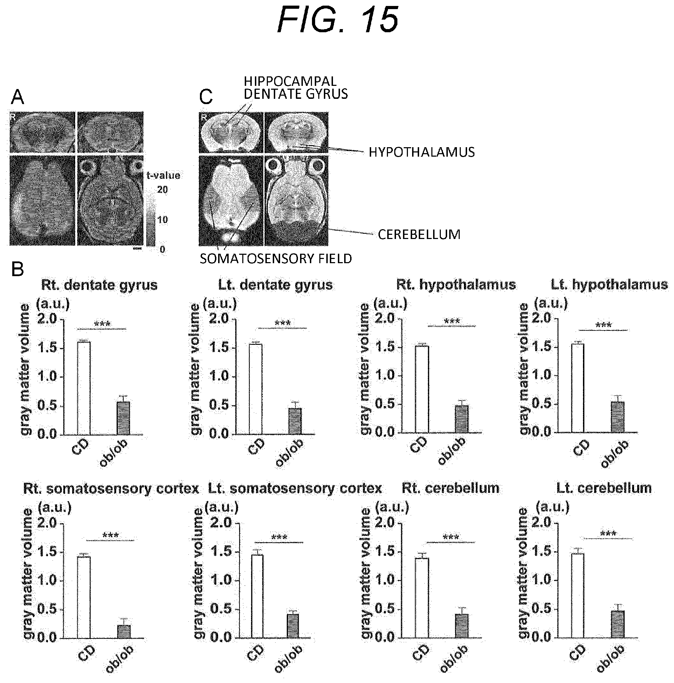

Fig. 15 shows a T-value map of grey matter (GM) volume and voxel intensity (Panels A and B) in GM region of the dentate gyrus, hypothalamus, somatosensory cortex, and the cerebellum of ob/ob mice (11 weeks old), and an anatomical ROI map (MRI image) of the hippocampal dentate gyrus, hypothalamus, somatosensory cortex, and cerebellum of CD mice in the MRI image (Panel C). Bar: 1 mm.

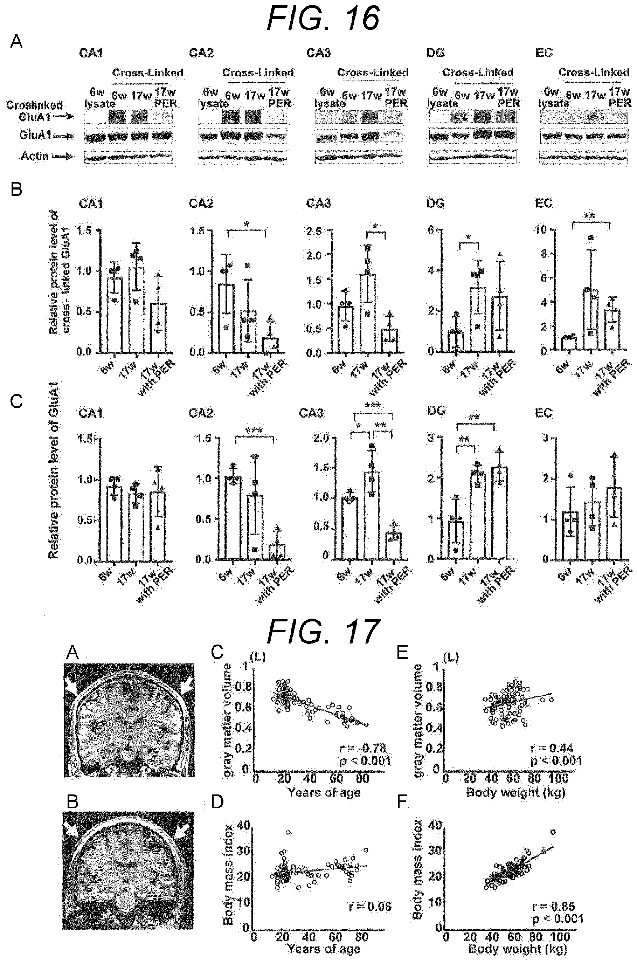

Fig. 16 shows a surface expression level and an intracellular pool amount of a hippocampus GluA1 subunit in ob/ob mice. Panel A shows the surface expression of GluA1 (top), the intracellular pool amount (middle), and the expression level of actin as an internal control (bottom) in ob/ob mice (6 or 17 weeks old or PER-administered group (PER administration from 11 weeks old). Panel B shows the relative expression level of GluA1 on the cell surface. Regarding the relative expression level, the expression level of GluA1 was normalized by the expression level of actin. Panel C shows the relative expression levels of GluA1 in the intracellular pool.

Fig. 17 shows a relationship among GM volume of whole human brain, body weight, BMI, and age. Panels A and B show representative T1-weighted coronal images of the normal body weight group (Panel A) and the obese group (Panel B). In the MRI image of the normal body weight, the parietal lobe and the temporal lobe bulge outward, but in the case of obesity, the parietal lobe and the temporal lobe are recessed without protruding. Panels C to F show results of partial correlation analysis of grey matter volume and age (Panel C), BMI and age (Panel D), grey matter volume and weight (Panel E), and BMI and weight (Panel F).

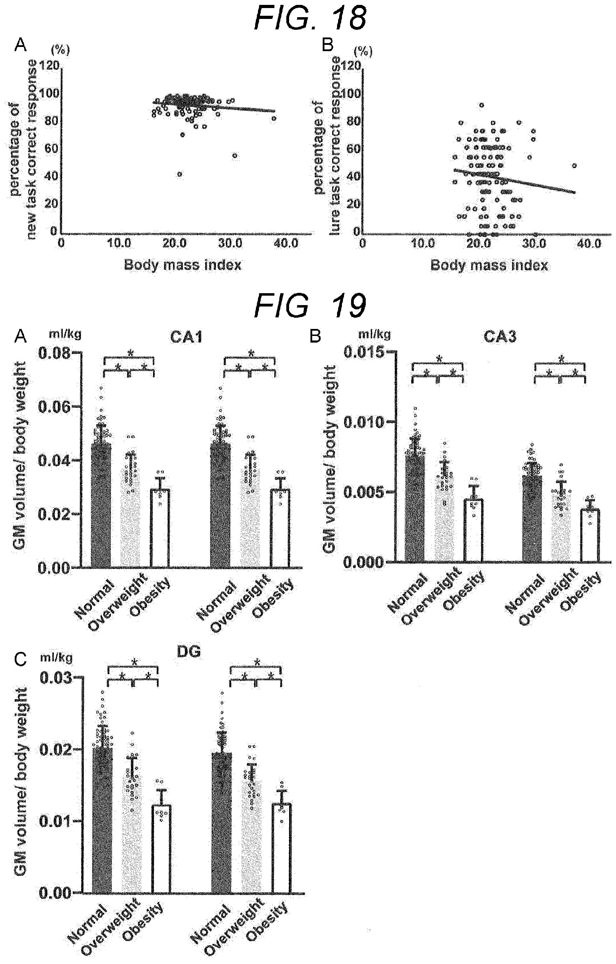

Fig. 18 illustrates a relationship between BMI and a correct answer rate in a new task and a Luer task. In the new task ($r = -0.05$, $p = 0.58$) and the Luer task ($r = -0.07$, $p = 0.43$), no significant negative correlation was found between BMI and the correct answer rate.

Fig. 19 shows a relationship between the volume of each region of the human hippocampus by MRI and overweight or obesity. Panels A to C show the average of the normal body weight group (Panel A), the overweight group (Panel B), and obesity (Panel C) (*: p < 0.05). Points in the graph are data of each individual.

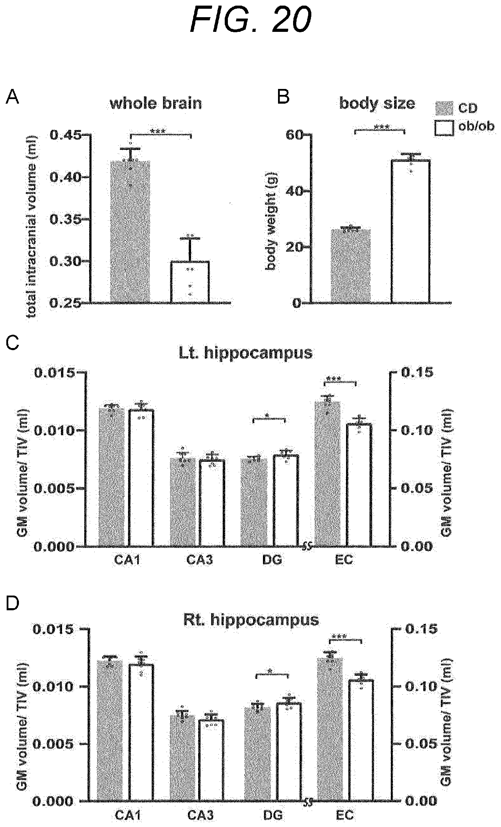

Fig. 20 shows the volume of each region of the hippocampus of CD-fed mice and ob/ob mice by volume analysis using MRI images. Panel A shows whole brain volumes of CD-fed mice and ob/ob mice (13 weeks old). Panel B shows the body weight of CD-fed mice and ob/ob mice (13 weeks old). Panel C shows the volume of each region of the left hippocampus of CD-fed mice and ob/ob mice (13 weeks old). Panel D shows the volume of each region of the left hippocampus of CD-fed mice and ob/ob mice (13 weeks old) .

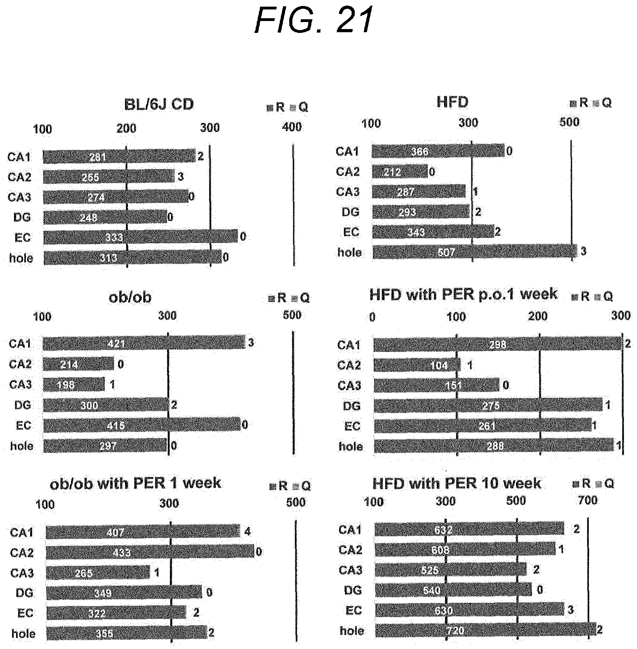

Fig. 21 shows the levels of R/Q editing of GluA2 in the CD-fed group, the HFD-fed group, the HFD-fed group with PER, ob/ob, PER-treated ob/ob. The level of R/Q editing was calculated as the ratio of the total number of reads aligned to the R/Q editing site of GluA2. Mice were 17 weeks old with BL/6J and fed HFD or HFD with PER from 16 to 17 weeks or from 6 to 17 weeks. PER treatment of ob/ob mice was from 16 to 17 weeks.

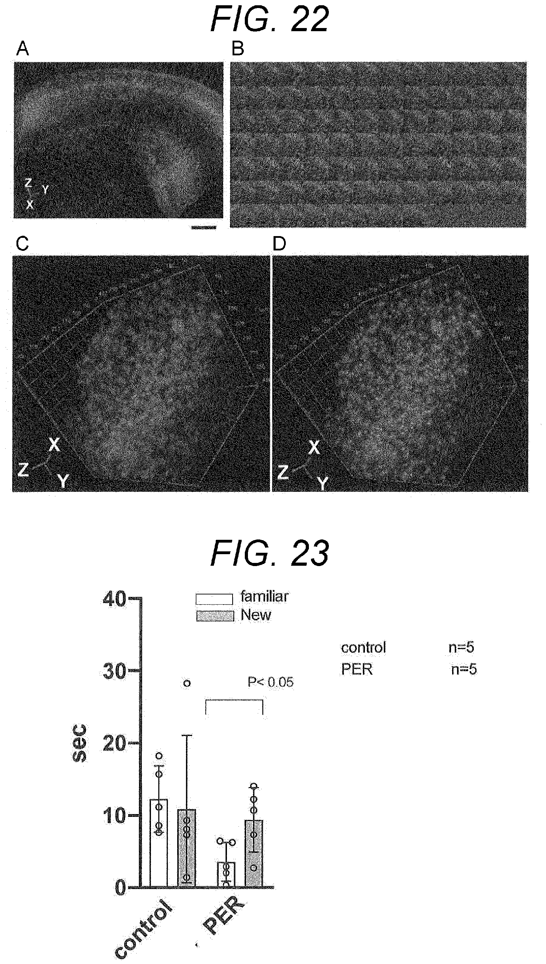

Fig. 22 shows the quantitative results of Thy1-YFPH[+] cells in the hippocampus CA1 region. Panel A shows an image of the entire hippocampus ($\times$ 10 fold water immersion objective) of the CD-fed group (17 weeks old). Voxel size was 4.013 um on the X axis, 4.013 um on the Y axis, 5.016 um on the Z axis. A part surrounded by a square is a cutout region. Bar: 400 $\mu$m. Panel B is a Panel display of selected cells. Panel C is a 3D reconstruction of the cutout region. Panel D is a Bob filter rendered image (average size 20 um, threshold 5 pm).

Fig. 23 shows the effect of perampanel administration on cognitive decline in Alzheimer's dementia model mice.

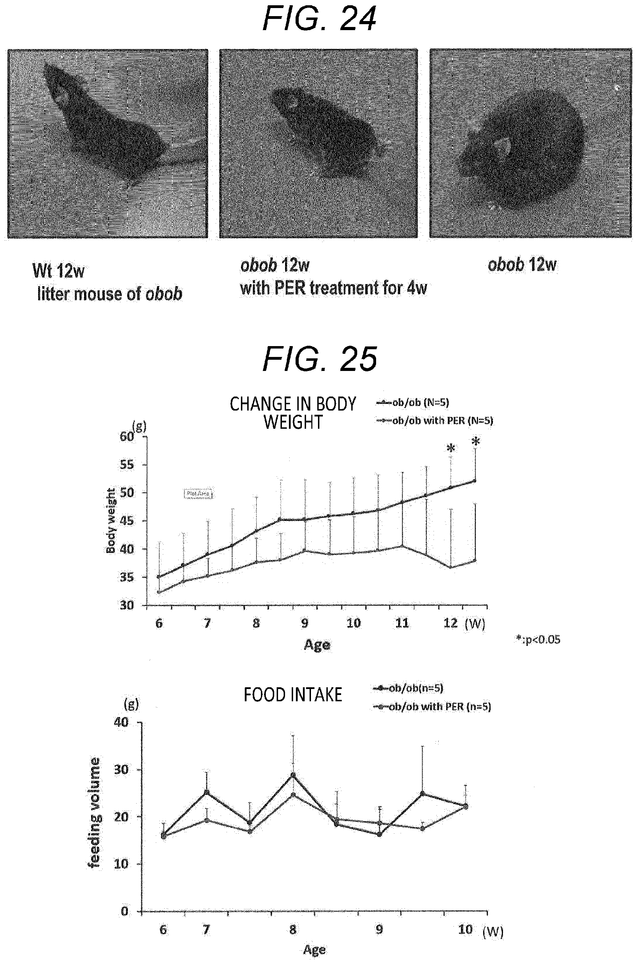

Fig. 24 shows photographs of appearances of wild type mice, ob/ob mice as an obesity model, and ob/ob mice to which an AMPA receptor antagonist was administered.

Fig. 25 shows the effect of administration of an AMPA receptor antagonist on body weight change in the HFD-fed group with comparable food intake.

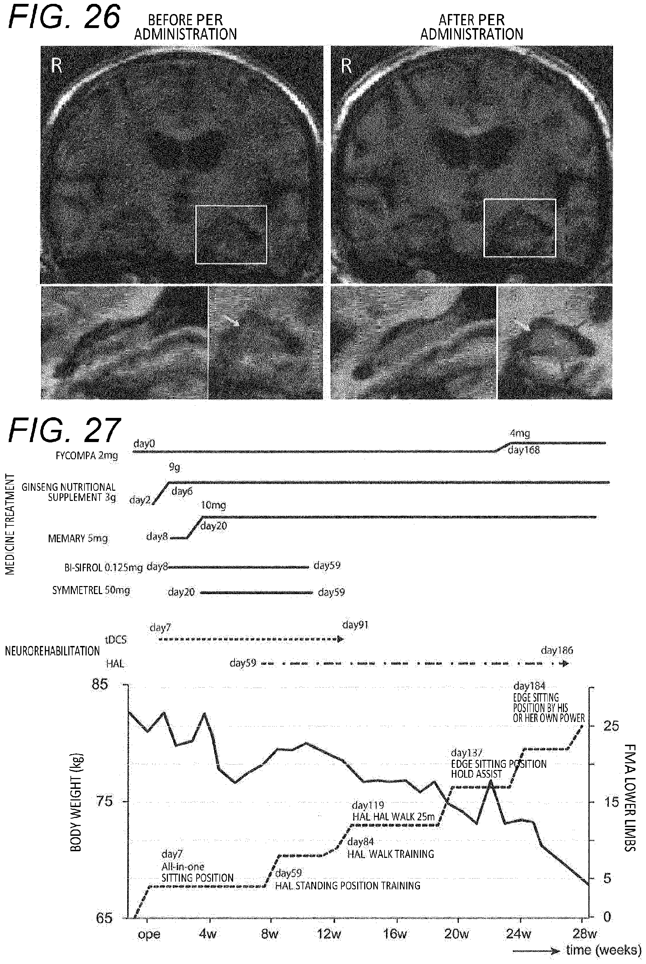

Fig. 26 shows the effect of anti-epileptic treatment on the hippocampus and body weight of a 70-year-old female with a BMI value of 38. The woman had a foramen magnum tumor and had quadriplegia and respiratory disorders. The MRI image of the brain after 26 weeks from administration (internal administration) of perampanel (PER) as an anti-epileptic treatment after tumor extraction is shown. The upper photograph before and after PER administration shows a T1-weighted coronal view, the lower left photograph shows a sagittal enlarged view of the left hippocampus, and the lower right photograph shows a coronal enlarged view of the left hippocampus. The upper left arrow in the lower right photograph indicates the CA3 region, the upper right arrow indicates the CA1 region, and the lower arrow indicates the DG/CA4 region.

Fig. 27 illustrates treatment for the 70-year-old female, transition of body weight, and transition of the score (FMA score) of lower limb Fugl-meyer assessment. After 26 weeks of PER administration, BMI values decreased to 31.

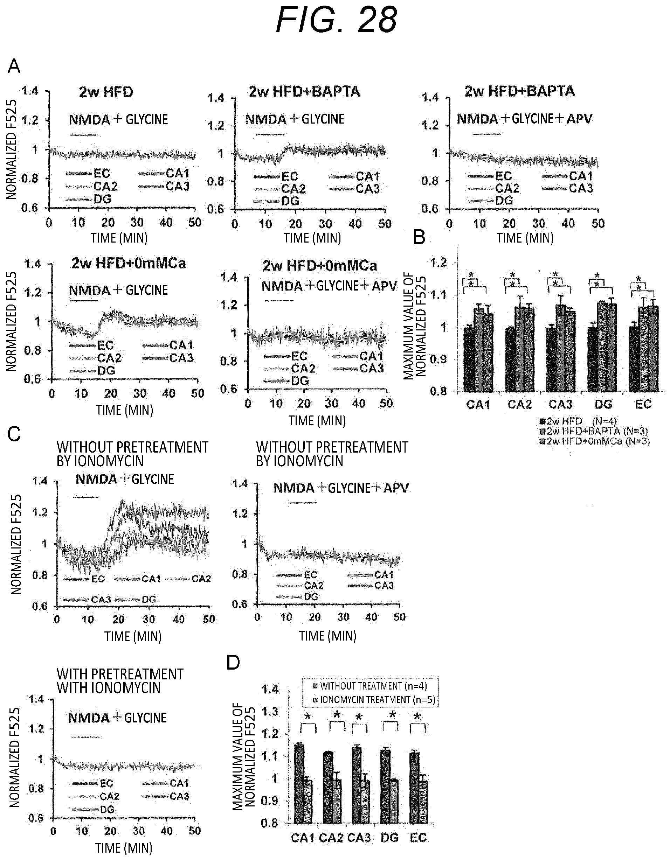

Fig. 28 shows that inactivation of the NMDA receptor in the HFD-fed groups is calcium ion-dependent.

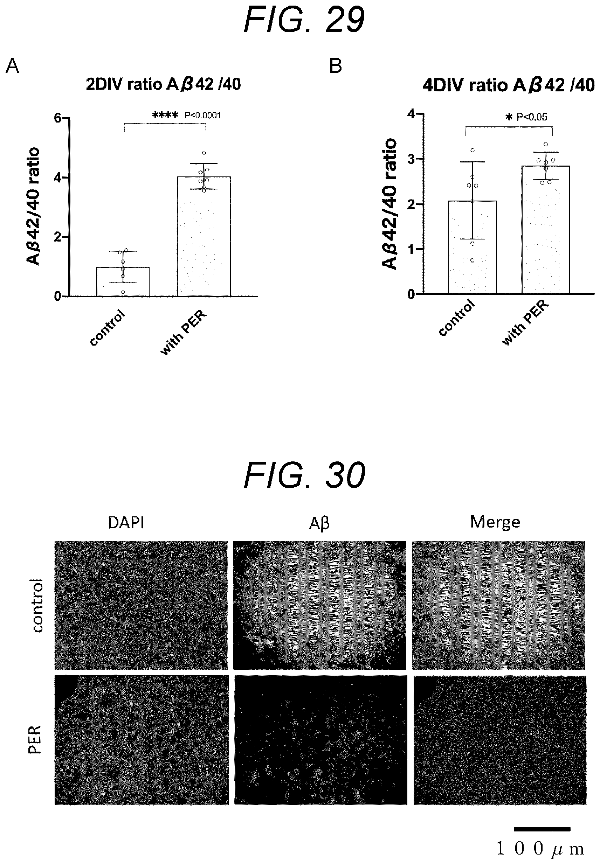

Fig. 29 shows the effect of perampanel (PER) administration on the A$\beta$42/A$\beta$40 ratio in neurospheres established from NL-G-F(-/-) mice, a model of Alzheimer's dementia.

Fig. 30 shows the effect of perampanel (PER) administration on accumulation of A$\beta$ in neurospheres established from NL-G-F(-/-) mice.

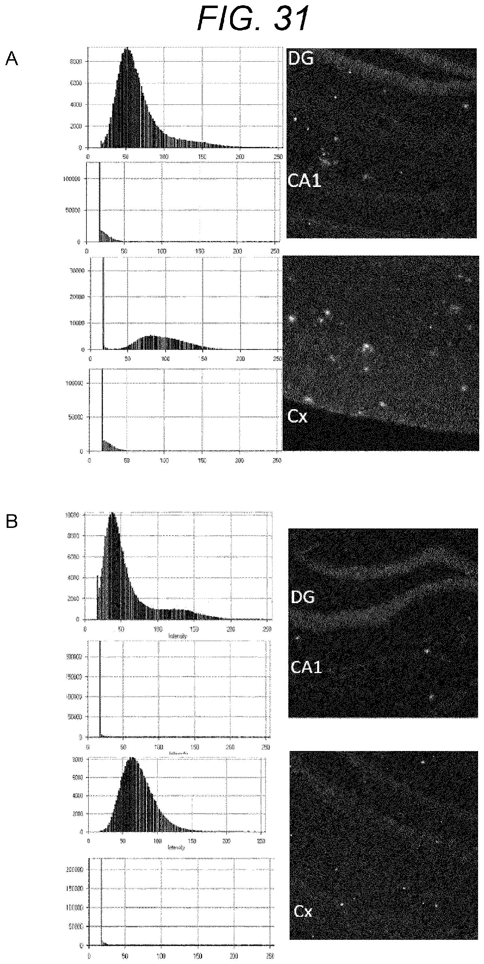

Fig. 31 shows the effect of perampanel administration on A$\beta$ accumulation in the hippocampal dentate gyrus (DG) CA1 and the cerebral cortex (Cx) in the Alzheimer's dementia model mouse (NL-G-F(-/-)).

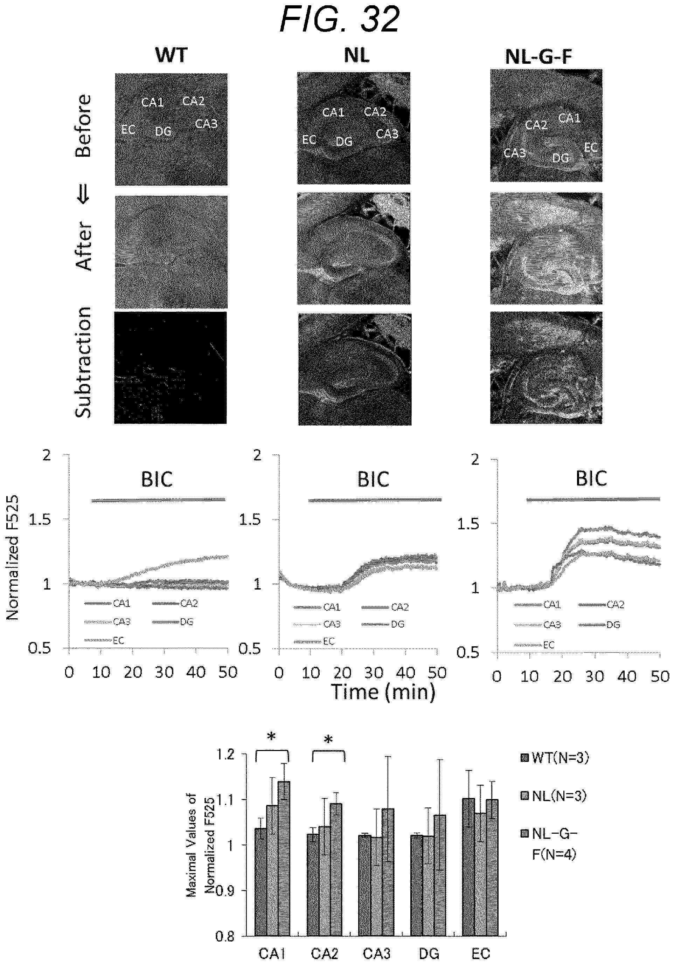

Fig. 32 shows significant enhancement of epileptiform reactions in the CA1 and CA2 regions by bicrine (BIC) administration to NL-G-F(-/-) mice.

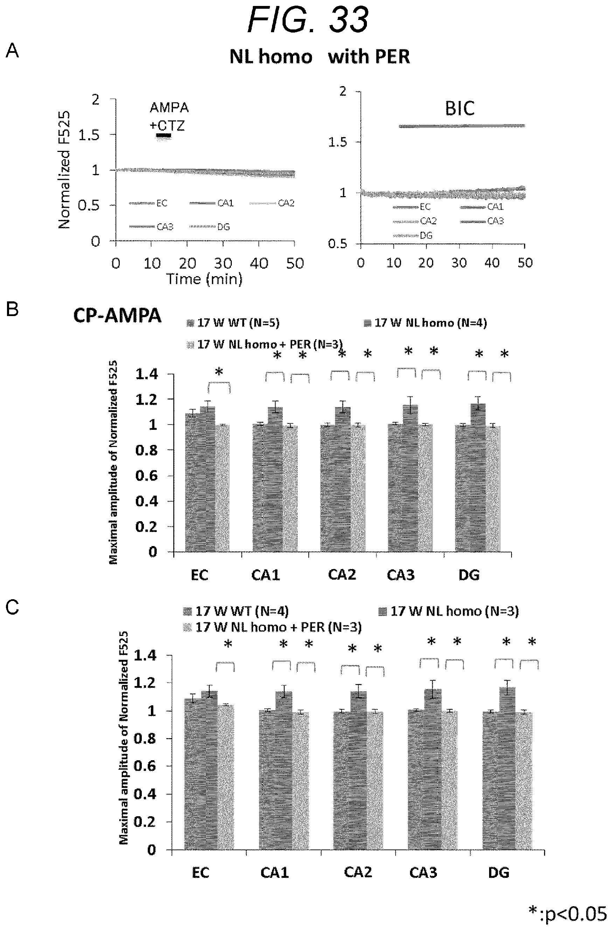

Fig. 33 shows the influence of administration of perampanel on epileptiform reactions induced by bicrine in NL(-/-) mice.

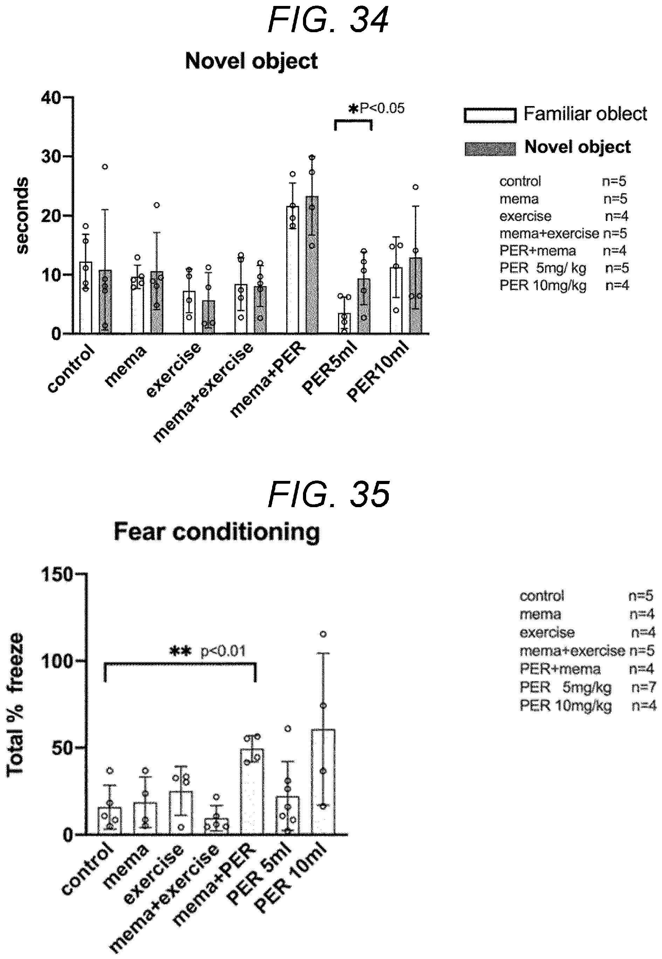

Fig. 34 shows the effects of memantine administration (mema), exercise tolerance (exercise), and perampanel administration (PER) in a new object recognition test in NL-G-F(-/-) mice.

Fig. 35 shows effects of memantine administration (mema), exercise tolerance (exercise), and perampanel administration (PER) in a fear conditioning test in NL-G-F(-/-) mice.

Description of Embodiments

As used herein, a "subject" is a mammal, for example, a primate, and for example, a human. A human can be, for example, from 0 to 10 years of age, from 10 to 20 years of age, from 20 to 30 years of age, from 30 to 40 years of age, from 40 to 50 years of age, from 50 to 60 years of age, from 60 to 70 years of age, from 70 to 80 years of age, and from 80 years of age and beyond, and at any age within the above ranges of age. A human may be male or female.

As used herein, "cognitive function" refers to an intellectual function such as understanding, judgment, and logic, and is understood as including elements such as perception, judgment, imagining, inference, decision, memory, and language understanding from a psychological perspective. As used herein, "cognitive dysfunction" is a disorder (or functional decline or dysfunction) in the higher functions of the brain, including judgment, calculation, understanding, learning, orientation, and memory. "Cognitive dysfunction" can be memory disorder and can manifest itself in dementia as memory disorder such as forgetfulness (for example, work memory degradation and work memory disorder). Cognitive dysfunction is diagnosed in an environment which is suitable for testing cognitive and memory functions or in which attention or concentration is possible.

As used herein, "treatment" refers to prophylactic and therapeutic treatment. As used herein, "therapeutic treatment" means treatment, cure, prevention, or amelioration of a disease or disorder, or reduction in the rate of progression of a disease or disorder. As used herein, "prophylactic treatment" means reducing the likelihood of developing a disease or condition, or delaying the onset of a disease or condition.

As used herein, a "therapeutically effective amount" means an amount by which the effect of therapeutic treatment or prophylactic treatment is obtained.

In the present specification, the "NMDA receptor" is a kind of ion channel type glutamate receptor present in the postsynaptic membrane, and is also called NMDA type glutamate receptor. The ion channel type glutamate receptors present in the postsynaptic membrane are roughly classified into an AMPA receptor, a kainate type receptor, and an NMDA receptor according to their pharmacological characteristics. The NMDA receptor is considered to be a receptor involved in memory, learning, cell death after cerebral ischemia, and the like. The NMDA receptor is activated by N-methyl-D-aspartate (NMDA; NMDA receptor agonists), which differs from other ionotropic glutamate receptors. The NMDA receptor binds to extracellular magnesium ions ($Mg^{2+}$) at a resting membrane potential of - 60 mV to -70 mV, and its channel activity is inhibited. When the postsynaptic membrane is depolarized (for example, the membrane potential may be positive or equal to or greater than -20 mV, such as between-20 mV and-10 mV), the inhibition by magnesium ions is released, and the NMDA receptor reacts with glutamic acid in the presence of glycine, and induces inflow of cations (in particular, calcium ions ($Ca^{2+}$), potassium ($K^+$) and sodium ($Na^+$) from the postsynaptic membrane into cells. In this manner, the NMDA receptor is depolarized (electrical signal; neural activity of the postsynaptic membrane) and stimulation with glutamic acid (biochemical signal; neural activity of presynaptic terminals) to express channel activity.

In the present specification, the "AMPA receptor" is a kind of ion channel type glutamate receptor present in the postsynaptic membrane, and is also called AMPA type glutamate receptor. The AMPA receptor is activated by α-amino-3-hydroxy-5-mesoxazole-4-propionic acid (AMPA; AMPA receptor agonists). The AMPA receptor is a tetramer containing any four of the four subunits GluA1, GluA2, GluA3, and GluA4. It is believed that each subunit has a glutamic acid receiving site and, when glutamic acid binds to two or more of the four, channel activity is induced. AMPA receptors with GluA2 are calcium-impermeable, whereas AMPA receptors without GluA2 are calcium-permeable (hereinafter the calcium permeable AMPA receptor may be referred to as a "CP-AMPA receptor"). The reason why the AMPA receptor including GluA2 is calcium impermeable is that glutamine (Q) in the M2 domain (second hydrophobic domain) of GluA2 is converted to arginine (R). The Q/R editing from the above Q to R of GluA2 above is the editing at the mRNA level (post-transcriptional regulation). Specifically, the conversion from the above Q to R occurs when the second base (adenine) of the codon specifying the glutamine of the GluA2 mRNA is deaminated by an adenine deaminase and converted to inosine, and the inosine is decoded as guanine by tRNA. Since the AMPA receptor containing unedited GluA2 is calcium permeable, the calcium impermeability of the AMPA receptor is believed to be due to the conversion of GluA2 from the above Q to R. An example of the nucleic acid sequence of human GluA2 is described in SEQ ID NO: 1, an example of the amino acid sequence of unedited human GluA2 is described in SEQ ID NO: 2, and an example of the amino acid sequence of edited human GluA2 is described in SEQ ID NO: 3. In the above Q to R editing of GluA2 from the Q to R, the 607th Q of SEQ ID NO: 2 is converted to R by editing. This site is referred to as a Q/R site of GluA2. Among glutamic acid responses, the AMPA receptor component is known to be faster than the NMDA receptor component. An example of the nucleic acid sequence of each of human GluA1, human GluA3, and human GluA4 is described in SEQ ID NO: 4, 6, and 8, and an example of the amino acid sequence is described in SEQ ID NO: 5, 7, and 9.

As used herein, the "hippocampus" is a cortex positioned in the bottom of the inferior horn of the lateral ventricle in the medial part of the cerebral temporal lobe. There is one hippocampus on each side of the brain. The hippocampus is a part of a limbic system called a hippocampus body, and the hippocampus body is divided into a dentate gyrus, a hippocampus, a subiculum, a presubiculum, a parasubiculum, and an entorhinal cortex. In addition, the hippocampus is roughly divided into CA1 to CA3 regions. The hippocampus is considered to be involved in various neuropsychiatric diseases such as epilepsy and Alzheimer's dementia. Hippocampus has the lowest seizure threshold in the brain, and in epileptic animal models, much of the electrical activity related to seizures is recorded from the hippocampus, starting in particular from the CA2 and CA3 fields. The initial symptom of Alzheimer's dementia is the lack of the ability to acquire new memory. The pathology of Alzheimer's dementia first appears in the entorhinal cortex. In Alzheimer's dementia, it is considered that the information processing ability of the hippocampus is deprived due to a disorder of the entorhinal cortex. As described above, the hippocampus has a low threshold of firing, and has an important function in saving and learning new memories. Hippocampus is vulnerable to hypoxia and ischemia and induces neuronal cell death under hypoxic conditions and ischemic conditions. Neuronal cell death is believed to be due to excitotoxicity via NMDA receptors. The hippocampus is believed to be essential for the formation of manifest memory, such as episodic memory. Temporal lobe cortical sites involved in memory formation have the entorhinal cortex, parasubiculum, anterior subiculum, subiculum, hippocampus (Ammons horn), and dentate gyrus. Inactivation of NMDA receptors in the hippocampus causes cognitive dysfunction, including memory disorder and learning disorder. Alzheimer's disease includes a state where the disease eventually transitions to Alzheimer's dementia but there are no clinical symptoms (preclinical stage Alzheimer's disease), mild cognitive dysfunction (MCI) that transitions to Alzheimer's dementia, and Alzheimer's dementia. In Alzheimer's disease, a decrease in Aβ42 levels, an increase in total tau levels, and an increase in phosphorylated tau levels in cerebrospinal fluid can be observed from the time of pre-clinical Alzheimer's disease. The reduction in Aβ42 levels in cerebrospinal fluid is believed to reflect the accumulation of Aβ42 in the brain. Accumulation of tau, changes in neurofibrils, shedding of nerve cells, and the like may increase and lead to transition to mild cognitive dysfunction. In mild cognitive dysfunction, a lesion is observed in a region around the hippocampus. In Alzheimer's dementia, the lesion also extends to the cerebral neocortex. Alzheimer's dementia is characterized, for example, by senile plaques and/or neurofibrillary tangles. Alzheimer's dementia may be diagnosed, for example, based on diagnostic criteria according to the American Society for Psychiatry Diagnostic and Statistical Manual, 5th Edition (DSM-5). That is, Alzheimer's dementia can be diagnosed when the following are satisfied:

A: meeting the criteria for major neurocognitive disorder;
B: at least two or more cognitive dysfunction develop in a latent manner and gradually progress;
C: there is evidence of the presence of a mutation in the causative gene of Alzheimer's dementia from a family history or genetic testing, or all of the following are met:

· Detailed history and neuropsychological tests over time provide clear evidence of decline in memory, learning, and other cognitive functions.
· Cognitive function is not stable for a long time, and is certainly getting worse gradually.
· No other neurodegenerative disease, cerebrovascular disease, nerve/mental/systemic disease or condition which are likely to cause cognitive dysfunction; and

D: there is clear evidence of decline in memory, learning and other cognitive functions in detailed medical history and neuropsychological tests over time.

Cognitive function is not stable for a long time, and is certainly getting worse gradually.

No other neurodegenerative disease, cerebrovascular disease, nerve/mental/systemic disease or condition which are likely to cause cognitive dysfunction.

In the present specification, the term "overweight" means that a body mass index (BMI) is 25 or more and less than 30 (particularly men and women of 18 years old or more). BMI is calculated by weight (kg)/{height (m)}$^2$. The standard BMI value is approximately 22 for both adult men and women. In the present specification, "obesity" refers to a human (particularly, adult men and women) having a BMI of 30 or more. Obesity is roughly classified into obesity class I with a BMI of 30 or more and less than 35, obesity class II with a BMI of 35 or more and less than 40, and obesity class III with a BMI of 40 or more according to the criteria (WHO criteria) by the World Health Organization (WHO). In the present specification, "metabolic syndrome" refers to a case where BMI is 25 or more, an abdominal circumference is 85 cm or more for men and 90 cm or more for women, and 2 or more of the following (1) to (3) are satisfied.

(1) The neutral fat is 150 mg/dL or more, or the HDL is less than 40 mg/dL. (2) The systolic blood pressure is 130 mmHg or more, or the diastolic blood pressure is 85 mmHg or more. (3) Fasting blood glucose is 110 mg/dL or more.

Obesity in a child can be determined by the degree of obesity. Specifically, the obesity of a child refers to a state where the degree of obesity is +20% or more, and the body fat percentage increases by 25% or more for males, increases by 30% or more for females under 11 years old, and increases by 35% or more for females over 11 years old. The degree of obesity (%) is calculated by (current body weight - standard body weight at that age)/standard body weight at that age × 100.

In the present specification, the term "dyslipidemia" refers to a state where lipid metabolism such as neutral fat and cholesterol is abnormal. Dyslipidemia is roughly classified into hyper LDL cholesterolemia, hypo HDL cholesterolemia, and hypertriglyceridemia. In hyper LDL cholesterolemia, whether a blood LDL cholesterol level is 140 mg/dL or more may be a diagnostic criterion. In hypo HDL cholesterolemia, whether a blood LDL cholesterol level is less than 40 mg/dL may be a diagnostic criterion. In hypertriglyceridemia, whether a blood neutral fat is 150 mg/dL or more may be a diagnostic criterion.

In the present specification, the "cardiovascular disease" refers to a disease group in which the inner wall of a blood vessel is narrowed by arteriosclerosis and the supply of blood to an organ is insufficient. Cardiovascular diseases include coronary artery disease, stroke, cerebral infarction, myocardial infarction, and peripheral arterial disease. When a state such as hyperglycemia continues, the inner wall of the blood vessel is damaged, and cholesterol accumulates in the blood vessel wall. Accumulation increases over time, narrowing the vascular lumen and reducing the supply of blood. A decrease in blood flow to the brain can cause cerebral infarction, and a decrease in blood volume to the heart can cause myocardial infarction. Other coronary artery disease and peripheral arterial disease may occur. Risk factors for cardiovascular disease include dyslipidemia and arteriosclerosis.

As used herein, "diabetes" is a disease in which hyperglycemia chronically continues due to insufficient action of insulin (for example, lack of insulin or lack of responsiveness to insulin). Diabetes is often associated with three major complications: retinopathy, nephropathy, and neuropathy. Diabetes mellitus is diagnosed by satisfying any one of the following (1) to (3) and (4): (1) a fasting blood glucose in the morning is 126 mg/dL or more, (2) a 2 hour fluorescent glucose tolerance test value of 75 g is 200 mg/dL or more, (3) a blood glucose value measured regardless of time is 200 mg/dL or more, and (4) HbA1c is 6.5% or more.

According to the present specification, a phenomenon was found in which in the hippocampus of a subject, calcium permeability of an AMPA receptor in the presence of glutamic acid (or in the presence of AMPA) increased, while activation of an NMDA receptor in the presence of glutamic acid (or in the presence of NMDA) was inhibited. This inhibition of NMDA receptor activation was resolved by AMPA receptor antagonists.

According to the present disclosure, there is provided a pharmaceutical composition for use in treating a disease or condition associated with glutamic acid (or NMDA) insensitive NMDA receptor in a subject (hereinafter also simply referred to as "NMDA unresponsiveness") having the glutamic acid (or NMDA) insensitive NMDA receptor, the pharmaceutical composition containing an AMPA receptor antagonist. As will be described later, the hippocampus of an NMDA-insensitive subject has an NMDA receptor that is inhibited regardless of the membrane potential of the postsynaptic membrane in the presence of glycine and NMDA. As a result, in the hippocampus of the NMDA-insensitive subject, the NMDA receptor is completely or partially inhibited regardless of the membrane potential of the postsynaptic membrane in the presence of glycine and NMDA. As described below, inhibition of the NMDA receptor may be inhibition of calcium influx into cells via the NMDA receptor (and preferably inhibition of increase in intracellular calcium concentration). As described later, in the above subject, the AMPA receptor has acquired agonist-dependent calcium permeability. As described below, in certain embodiments, the disease or condition associated with NMDA insensitivity may be cognitive dysfunction, including cognitive decline. As described below, cognitive dysfunction can be derived from overweight or obesity. As described below, cognitive dysfunction may be derived from Alzheimer's dementia. In an NMDA-insensitive subject, the ratio of blood oxygen level-dependent (BOLD) responses when working on a hippocampal memory task to the resting case (when not doing anything) can be equal to or greater than a first predetermined ratio as described below. In an NMDA-insensitive subject, the ratio of the BOLD response when working on the event-related memory task to the resting case at rest (when not doing anything) can be equal to or less than a second predetermined ratio as described below. An increase in BOLD response is indicative of overactivity of synaptic function and a decrease is indicative of synaptic dysfunction. NMDA insensitivity may be assessed by unresponsiveness of brain activity upon NMDA administration. In a living body, when working on a hippocampus functional problem using functional magnetic resonance imaging (fMRI), a task correct answer rate decreases, and a synaptic function decrease in which a BOLD signal is continuously low, or an overactive state of a synaptic function in which an initial dip disappears and a peak of a first positive wave increases can be used as an index to evaluate functional decrease and overactive state.

In addition, as suggested from Examples to be described later, in the hippocampus of a subject exhibiting overweight and obesity, the calcium permeability of the AMPA receptor in the presence of glutamic acid (AMPA) increases, while the activation of the NMDA receptor in the presence of glutamic acid (NMDA) is inhibited, and abnormality in the control of the AMPA receptor and the NMDA receptor may be observed. In addition, as suggested by Examples to be described later, in the hippocampus of a subject exhibiting overweight and obesity, inhibition of the NMDA receptor not to be activated under the conditions under which the NMDA receptor should be activated damages plasticity of synapses, leading to long-term potentiation (LTP) and long term depression (LTD) and may cause cognitive dysfunction. Furthermore, as suggested by Examples to be described below, the inhibition of the NMDA receptor can be released by inhibition of calcium entering from the AMPA receptor by the AMPA receptor antagonist (in particular, calcium permeable AMPA receptors in the hippocampus). Without wishing to be bound by theory, subjects exhibiting overweight and obesity may have certain cognitive dysfunction due to the aforementioned abnormalities in the control of the AMPA receptor and the NMDA receptor in the hippocampus. Calcium permeability of the AMPA receptor in the presence of AMPA also increased in the hippocampus of a subject having Alzheimer's dementia, and activation of the NMDA receptor in the presence of NMDA was inhibited.

According to the present disclosure, there is provided a pharmaceutical composition for use in treating cognitive dysfunction (including cognitive decline, the same applies hereinafter) in a subject, the pharmaceutical composition containing an AMPA receptor antagonist.

AMPA receptor antagonists include competitive and non-competitive antagonists (for example, allosteric antagonists). In certain embodiments, as the AMPA receptor antagonist, a non-competitive antagonist can be preferably used. Examples of the AMPA receptor antagonist include AMPA receptor-selective antagonists (for example, AMPA receptor-selective competitive antagonists and AMPA receptor-selective non-competitive antagonists). The AMPA receptor-selective antagonist means that the inhibitory effect on the AMPA receptor is greater (for example, the $IC_{50}$ for the AMPA receptor is 2 times or less smaller, 3 times or less smaller, 5 times or less smaller, 10 times or less smaller, 30 times or less smaller, 50 times or less smaller, 100 times or less smaller, 300 times or less smaller, 500 times or less smaller, 1000 times or less smaller, 3000 times or less smaller, 5000 times or less smaller, or 10,000 times or less smaller than $IC_{50}$ for other ion channel type glutamic acid receptors) than that on other ion channel type glutamic acid receptors such as the NMDA receptor and the kainate receptor. As the AMPA receptor antagonist, an AMPA receptor-selective antagonist can be preferably used.

The AMPA receptor antagonist is not particularly limited, and examples thereof include 2,3-benzodiazepine compounds, 4-(8-chloro-2 methyl-11H-imidazo[1,2-c][2,3]benzodiazepine-6-benzenamine (GYKI 47261), 1-(4-aminophenyl)-4 methyl-7,8-methylenedioxy-5H-2,3-benzodiazepine (GYKI 52466), and 1-(4-aminophenyl)-3 methylcarbamyl-4 methyl-7,8-methylenedioxy-3,4-dihydro-5H-2,3-benzodiazepine (GYKI 53655) (Paternain et al., Neuron 1995, 14: 185-189), 2,3-dioxo-6-nitro-1,2,3,4-tetrahydrobenzo[f]quinoxaline-7-sulfonamide (NBQX), 6-cyano-7-nitroquinoxaline-2,3-dione (CNQX), 6,7-dinitroquinoxaline-2,3-dione (DNQX), 1,4-dihydro-6-(1H-imidazo-1-yl)-7-nitro-2,3-quinoxalinedione (YM90K), (3S,4aR,6S,8aR)-6-(([1H]1,2,4-triazole-5-yl-sulfonyl)methyl)-1,2,3,4,4a,5,6,7,8,8a-decahydroisoquinoline-3-carboxylic acid (LY302679), LY292025, LY307190, LY280263, LY289178, LY289525, (3S,4aR,6R,8aR)-6-[2-([1H tetrazole-5-yl)ethyl]-1,2,3,4,4a,5,6,7,8,8a-decahydroisoquinoline-3-carboxylic acid (LY293558), (3SR,4aRS,6SR,8aRS)-6-((1H-tetrazole-5-yl)methyloxymethyl)-1,2,3,4,4a,5,6,7,8,8a-decahydroisoquinoline-3-carboxylic acid (LY294486) (Neuropharmacology, 37: 1211-1222, 1998), 9-(1H-imidazole-1-yl)-8-nitro-[1,2,4]triazolo[1,5-c]quinazoline-2,5(3H,6H)-dione(Ro48-8587), N-[3-[[4-[(3-aminopropyl)amino]butyl]amino]propyl]-1-naphthaleneacetamide (Naspm), Joro spider venom, 8-methyl-5((4-(N,N-dimethylsulfamoyl)phenyl)6,7,8,9-tetrahydro-1H-pyrrolo[3,2-h]-isoquinoline-2,3-dione-3 O-(4-hydroxybutyrate-2-yl)oxime (SPD-502), NS-1209 (Neuropharmacology. 61(5-6): 1033-47, 2011), talamPanel, 1,2-dihydropyridine compound, for example, 3-(2-cyanophenyl)-5-(2-pyridyl)-1-phenyl-1,2-dihydropyridine-2-one (perampanel; E2007) (US6,949,571B), 2,3-dihydroxy-6-nitro-7-sulfamoylbenzo (f) quinoxaline, [1,2,3,4,-tetrahydro-7-morpholin-yl-2,3-dioxo-6-(trifluoromethyl)quinoxaline-1-yl]methylphosphonate, 1-(4-aminophenyl)-4 methyl -7,8-methylene-dioxy-5H-2,3-benzodiazepine, or (-)1-(4-aminophenyl)-4-methyl-7,8-methylene-dioxy-4,5-dihydro-3 methylcarbamoyl-2,3-benzodiazepine, [7-(1H-Imidazole-1-yl)-6-nitro-2,3-dioxo-3,4-dihydroquinoxaline-1(2H)-yl]acetic acid (ZonamPanel) (WO 96/10023), and 2-[N-(4-chlorophenyl)-N-methylamino]-4H-pyrido[3,2-e]-1,3-thiazine-4-one, and the compounds disclosed in WO 2003/082332 (for example, CX516, LU-73068, LU115445, Aloracetam, compound described in WO95/12594, IramPanel, compound described in WO98/17652, LY215490, LY-215490, LY-293558, LY-311446, LY326325, LY-377770, LY-404187, 2-methylsulfonylamino-6,7-dinitro-2(1H)-quinoxalinone, compound described in WO97/32858, GYKI-47261, compound described in WO99/06408, GYKI52466, GYKI-53655, GYKI152466, TalamPanel, compound described in US5639751, RWJ37947, KRP-199, NS-1029, NS229, PNQX, COMP, AMP-397, NNC-07-0775, compound described in WO96/15100, NNC-07-9202, compound described in EP283959, 2-carboxy-1-methyl-7-trifluoromethylimidazo[1,2-a]quinoxalin-4(5H)-one, compound described in WO95/21842, CNDQ, PD-159265, PD160725, CP-465022-27, NS-257, PNQX,1-(4-aminophenyl)-7,8-(methylenedioxy)-3,5-dihydro-4H-2,3-benzodiazepine-4-one, SH-608, ZK-200.755-2, ZK-200775, compound described in WO99/07707, EGIS-9637, EGIS7444, S-17625, S-347301, S-1746, SYM2189, SYM-2207, SYM2229, SYM2259, SYM2267, YM90K, compound described in WO92/07847, TQX173, and Kaitocephalin) and their pharmaceutically acceptable salts (salts may include solvates such as hydrates). As the AMPA receptor antagonist, one or more selected from the group consisting of GYKI 47261, Naspm, and perampanel can be used.

In certain embodiments, the pharmaceutical compositions of the present disclosure contain a therapeutically effective amount of an AMPA receptor antagonist. In certain embodiments, the pharmaceutical compositions of the present disclosure contain a therapeutically effective amount of an AMPA receptor-selective antagonist. In certain embodiments, the pharmaceutical compositions of the present disclosure contain a therapeutically effective amount of one or more selected from the group consisting of GYKI 47261, Naspm, and perampanel.

In certain embodiments, the subject is a human. In certain embodiments, the subject is a human over the age of 18 (for example, male or female).

In certain embodiments, the subject is overweight. In certain embodiments, the subject is obese. In certain embodiments, the subject has obesity class I. In certain embodiments, the subject has obesity class II. In certain embodiments, the subject has obesity class III.

In certain embodiments, the subject has cognitive dysfunction (including cognitive decline). In certain embodiments, the subject has dementia and is overweight. In certain embodiments, the subject has cognitive dysfunction and obesity selected from the group consisting of obesity classes I to III. In certain embodiments, the subject is overweight or obese, and hippocampal memory function of pattern completion is lower than in healthy subjects. In certain embodiments, the subject has cognitive dysfunction, but is not overweight or obese.

In certain embodiments, the cognitive dysfunction is memory disorder (including a decline in memory function). In certain embodiments, the memory disorder may be social memory disorder. In certain embodiments, the memory disorder can be a decrease in pattern completion ability. In certain embodiments, the cognitive dysfunction is a learning disorder (including a decline in learning function). In certain embodiments, the memory disorder can be memory disorder in social memory.

In certain embodiments, the subject has dementia. In certain embodiments, the subject has dementia and is overweight. In certain embodiments, the subject has dementia and obesity selected from the group consisting of obesity classes I to III. In certain embodiments, the subject has dementia but is not overweight or obese.

In certain embodiments, the subject has Alzheimer's dementia. In certain embodiments, the subject has Alzheimer's dementia and is overweight. In certain embodiments, the subject has Alzheimer's dementia and obesity selected from the group consisting of obesity classes I to III. In certain embodiments, the subject is Alzheimer's dementia but is not overweight or obese. In certain embodiments, the patient with Alzheimer's dementia may be a patient with epilepsy. In certain embodiments, the patient with Alzheimer's dementia may be a patient without epilepsy. Epilepsy is a disease that causes epileptic seizures repeatedly such as sudden loss of consciousness and loss of response.

In certain embodiments, a subject with cognitive dysfunction has a reduced grey matter volume than that of a healthy subject.

In certain embodiments, in subjects with cognitive dysfunction, activation of the central enforcement network is observed in resting brain activity. In certain embodiments, in subjects with cognitive dysfunction, connectivity to a default mode network of a central enforcement network is permitted in resting brain activity. In certain embodiments, in subjects with cognitive dysfunction, activation of the visual network is observed in resting brain activity. In certain embodiments, in subjects with cognitive dysfunction, there is an activation of the central enforcement network and an activation of the visual network. In certain embodiments, in subjects with cognitive dysfunction, there is a connection between the saliency network and the cerebellar network in resting brain activity. The subject with cognitive dysfunction may be a subject with, in particular, obesity or overweight. The network has been developed as a biomarker of human cognitive function (35). Specifically, the network can be obtained by recording resting brain activity of a subject by fMRI and analyzing connectivity of activation of the brain (35).

In one embodiment, in the subject having cognitive dysfunction, the change ratio of the BOLD response in the CA3 region of the hippocampus is equal to or greater than the first predetermined ratio. The first predetermined ratio may be 1.1 times or more, 1.11 times or more, 1.12 times or more, 1.13 times or more, 1.14 times or more, 1.15 times or more, 1.16 times or more, 1.17 times or more, 1.18 times or more, 1.19 times or more, or 1.2 times or more. The upper limit of the first predetermined ratio may be, for example, either 1.3 times or a scale factor for the first predetermined value indicated above. In certain embodiments, in the subject having cognitive dysfunction, the change ratio of the BOLD response in the CA3 region of the hippocampus is less than the second predetermined ratio. The second predetermined ratio may be equal to or less than 0.9 times, equal to or less than 0.89 times, equal to or less than 0.88 times, equal to or less than 0.87 times, equal to or less than 0.86 times, equal to or less than 0.85 times, equal to or less than 0.84 times, equal to or less than 0.83 times, equal to or less than 0.82 times, equal to or less than 0.81 times, or equal to or less than 0.8 times. The lower limit of the second predetermined ratio may be, for example, either equal to or greater than 0.7 times or a scale factor for the second predetermined value indicated above. The change ratio of the BOLD response is determined by measuring the BOLD response by functional MRI (fMRI) under the condition of the presence or absence of a behavioral task. Examples of the behavioral task include an event-related memory task. In the event-related memory task, for example, it is possible to confirm whether the subject can correctly answer the same image, the similar image, and the new image as the same image, the similar image, and the new image, respectively. In the event-related memory task, a correct answer rate can be measured.

In certain embodiments, the subject has an NMDA receptor inhibited in at least one or more regions of a hippocampus body selected from the group consisting of an entorhinal cortex (EC), CA1, CA2, CA3, and a dentate gyrus (DG), thereby inhibiting NMDA receptor activity by NMDA in at least one or more regions of the hippocampus, and the subject has cognitive dysfunction. Inhibition of NMDA receptors is inhibition of NMDA receptor activation in the presence of glutamic acid and glycine (and preferably magnesium ion) (that is, under the condition that the NMDA receptor is activated in a physiological environment). When the NMDA receptor is not activated at the time to be activated, memory disorder and learning disorder may occur.

In the present disclosure, the inhibition of the NMDA receptor is based on the acquisition of calcium permeability of the AMPA receptor. In the present disclosure, the AMPA receptor antagonist is effective for releasing the inhibition of the NMDA receptor. Then, by releasing the inhibition of the NMDA receptor, the NMDA receptor can restore its original function and improve cognitive dysfunction. In certain embodiments, a pharmaceutical composition containing an AMPA receptor antagonist of the present disclosure may be combined with an NMDA receptor agonist. NMDA receptor agonists include Rapastinel (PCT/US2017/015851).

Therefore, the overweight and obese subject and the subject with cognitive dysfunction has an NMDA receptor inhibited in at least one or more regions of a hippocampus body selected from the group consisting of an entorhinal cortex (EC), CA1, CA2, CA3, and a dentate gyrus (DG), thereby inhibiting NMDA receptor activity by NMDA in at least one or more regions of the hippocampus, and the subject has cognitive dysfunction.

The calcium permeability of the AMPA receptor may increase by decreasing the expression level of GluA2 and/or decreasing the Q/R editing of GluA2. In GluA2, glutamine at the Q/R site is converted to arginine by post-transcriptional translation (subject to Q/R editing). Q/R-edited GluA2, when incorporated into the AMPA receptor, renders the AMPA receptor calcium-impermeable. When the uptake of GluA2 subjected to Q/R editing into the AMPA receptor is inhibited, the AMPA receptor acquires calcium permeability and allows calcium to flow into the cell in response to glutamic acid.

In certain embodiments, in the hippocampus of the subject, the ratio of GluA2/AMPA receptor is lower than the ratio in a healthy subject. In certain embodiments, in the hippocampus of the subject, the ratio of the unedited type of GluA2 to the total number of GluA2 is increased more than the ratio in the healthy subject. In certain embodiments, in the CA2 region of the hippocampus of the subject, the ratio of the unedited type of GluA2 to the total number of GluA2 is increased by a predetermined ratio or more than the ratio in the healthy subject. The predetermined ratio may be 2 times or more, 3 times or more, 4 times or more, 5 times or more, 6 times or more, 7 times or more, 8 times or more, 9 times or more, 10 times or more, 15 times or more, 20 times or more, 25 times or more, 30 times or more, 35 times or more, 40 times or more, 45 times or more, or 50 times or more.

In certain embodiments, in any region (for example, CA3 region) selected from the group consisting of CA1, CA2, and CA3 of the hippocampus of the subject, the number of Thy-1 positive nerve cells is smaller than the number of cells in a healthy subject. In certain embodiments, in any region (for example, the CA1 region) selected from the group consisting of CA1, CA2, and CA3 of the hippocampus of the subject, integrity of the dendritic arbor (specifically, integrity to CA1) is lower than integrity in a healthy subject. In certain embodiments, in any region of the hippocampus body of the subject (for example, DG region), the ratio of the immature spine is increased more than the ratio in the healthy subject. AMPA receptor antagonists can improve these situations.

According to the present disclosure, there is provided a pharmaceutical composition for use in inhibiting suppression of activation of an NMDA receptor in a subject having a hippocampus expressing a calcium-permeable AMPA receptor, the pharmaceutical composition containing an AMPA receptor antagonist.

In a subject having a hippocampus expressing a calcium-permeable AMPA receptor, the intracellular calcium concentration of nerve cells is increased. Activation of the NMDA receptor is inhibited in the hippocampus of a subject having a hippocampus expressing a calcium-permeable AMPA receptor. In contrast, the AMPA receptor antagonist can inhibit glutamate-dependent calcium transport of the AMPA receptor into cells and inhibit suppression of activation of the NMDA receptor. By inhibiting the suppression of NMDA receptor activation, the NMDA receptor can induce activation dependent on glutamate and membrane potential.

Pharmaceutical compositions of the present disclosure may contain an AMPA receptor antagonist in the form of a pharmaceutically acceptable salt. Pharmaceutically acceptable salts include acid addition salts. Examples of the acid addition salt include inorganic acid salts such as hydrochloride, hydrobromide, sulfate, hydroiodide, nitrate and phosphate; and organic acid salts such as citrate, oxalate, phthalate, fumarate, maleate, succinate, malate, acetate, formate, propionate, benzoate, trifluoroacetate, methanesulfonate, benzenesulfonate, para-toluenesulfonate, and camphorsulfonate. Pharmaceutical compositions of the present disclosure may contain an AMPA receptor antagonist in the form of a prodrug. Prodrugs are metabolized in the body to exhibit drug efficacy in the body. Often, prodrugs are made by esterifying their carboxyl group or hydroxy group. The ester bond is broken down by an esterase present in the body, and the active ingredient is released from the prodrug. Pharmaceutical compositions of the present disclosure may further contain a pharmaceutically acceptable additive in addition to the AMPA receptor antagonist. As the pharmaceutically acceptable additive, an excipient, a disintegrant, a binder, a fluidizing agent, a lubricant, a coating agent, a dissolving agent, a solubilizing agent, a thickener, a dispersing agent, a stabilizing agent, a sweetening agent, a flavoring agent, and the like can be used depending on the purpose. Specific examples thereof include lactose, mannitol, crystalline cellulose, low substituted hydroxypropylcellulose, corn starch, partially gelatinized starch, carmellose calcium, croscarmellose sodium, hydroxypropylcellulose, hydroxypropylmethylcellulose, polyvinyl alcohol, magnesium stearate, sodium stearyl fumarate, polyethylene glycol, propylene glycol, titanium oxide, and talc. Pharmaceutical compositions of the present disclosure may be formulated for oral or parenteral administration (for example, intravenous administration or the like). The dosage form is not particularly limited, and examples thereof include tablets, capsules, powders, granules, solutions, suspensions, injections, patches, and cataplasms. Administration can be by, for example, oral administration or parenteral administration (for example, intravenous administration, intracerebroventricular administration, intracerebral intrathecal administration, etc.). The dosage can be, for example, 100 to 2,000 mg per adult.

In an embodiment of the present disclosure, there is provided a method of treating a disease or condition associated with NMDA insensitivity in an NMDA-insensitive subject or a subject having an NMDA-insensitive NMDA receptor , the method including administering to the subject a therapeutically effective amount of an AMPA receptor antagonist. The subject, the disease or condition associated with NMDA insensitivity, and the AMPA receptor antagonist may each be as described above.

In an embodiment of the present disclosure, there is provided a method of treating cognitive dysfunction in a subject in need thereof, the method including administering to the subject a therapeutically effective amount of an AMPA receptor antagonist. The subject, cognitive dysfunction, and AMPA receptor antagonist may each be as described above.

According to an embodiment of the present disclosure, there is provided a method for inhibiting suppression of activation of an NMDA receptor in a subject having a hippocampus expressing a calcium-permeable AMPA receptor, the method including administering an effective amount of an AMPA receptor antagonist to the subject. The subject, cognitive dysfunction, and AMPA receptor antagonist may each be as described above.

In an embodiment of the present disclosure, there is provided a method of treating a subject having obesity or overweight, the method including administering to the subject an effective amount of an AMPA receptor antagonist. An effective amount can be an amount that can delay and stop the development of obesity or overweight, or ameliorate obesity or overweight. In certain embodiments of the present disclosure, the subject may be a subject subject to dietary restrictions. In certain embodiments of the present disclosure, the subject may be a subject that is not subject to dietary restrictions. In certain embodiments of the disclosure, the subject does not receive or is not receiving a diet. In certain embodiments of the disclosure, the subject receives or is receiving a diet. In certain embodiments of the present disclosure, cognitive dysfunction may be improved in a subject. In certain embodiments of the present disclosure, obesity or overweight may be ameliorated in a subject.

In certain embodiments of the present disclosure, there is provided a method of treating a subject having Alzheimer's dementia, the method including administering to the subject a therapeutically effective amount of an AMPA receptor antagonist. In certain embodiments of the present disclosure, there is provided a method of treating Alzheimer's dementia in a subject in need thereof, the method including administering to the subject a therapeutically effective amount of an AMPA receptor antagonist. The subject may be, for example, a subject having pre-clinical Alzheimer's disease. The subject may be, for example, a subject having an accumulation of Aβ in the brain. Accumulation of Aβ can be assessed by Positron Emission Tomography (PET). The subject may be, for example, a subject having any of reduced Aβ42 levels, elevated total tau levels, and elevated levels of phosphorylated tau in the cerebrospinal fluid. These levels can be examined using, for example, an antibody. The subject may be, for example, a subject with mild cognitive dysfunction, or moderate cognitive dysfunction, or severe cognitive dysfunction. The subject may be a subject having neurodegeneration in the hippocampus. The subject may be a subject having neurodegeneration in the cerebral neocortex. The subject may be, for example, a subject having an accumulation of tau in the brain. Accumulation of tau in the brain can be assessed by PET.

In an embodiment of the present disclosure, an AMPA receptor antagonist for use in the method is provided. In an embodiment of the present disclosure, a pharmaceutical composition, for use in the above method, including an AMPA receptor antagonist may be provided.

In an embodiment of the present disclosure, there is provided a use of an AMPA receptor antagonist in the manufacture of a medicament for use in treating a disease or condition associated with NMDA insensitivity in an NMDA-insensitive subject. In addition, in an embodiment of the present disclosure, there is provided a use of an AMPA receptor antagonist in the manufacture of a medicament for use in treating cognitive dysfunction in a subject in need thereof. In an embodiment of the present disclosure, there is provided a use of an AMPA receptor antagonist in the manufacture of a medicament for use in inhibiting suppression of activation of an NMDA receptor in a subject having a hippocampus expressing a calcium-permeable AMPA receptor. The subject, cognitive dysfunction, and AMPA receptor antagonist may each be as described above.

The AMPA receptor antagonist can be used in combination with memantine. Memantine may be in the form of an addition salt, for example, in the form of an acid addition salt, preferably in the form of a hydrochloride salt. Therefore, memantine can be used in combination, for example, as memantine hydrochloride. In certain embodiments of the present disclosure, a combination pharmaceutical product containing an AMPA receptor antagonist and memantine is provided. In certain embodiments of the present disclosure, there is provided a combination pharmaceutical product including a pharmaceutical composition containing an AMPA receptor antagonist and a pharmaceutical composition containing memantine. The combination pharmaceutical product can be used for the treatment of cognitive dysfunction.

Examples

Animals

Male C57BL/6J background and *ob/ob* mice were obtained from CLEA Japan Inc. and Japan SLC, respectively (3). The reason why male mice were selected is that it has been reported that male mice are susceptible to HFD, such as weight gain, metabolic changes, and disorder of learning and hippocampal synaptic plasticity. These animals were housed 3 to 4 per cage in a standard 12 hour dark-light cycle room at 25°C in an environment with freely available food and water. All animal experiments were performed in accordance with the guidelines of the Animal Experiment Ethics Committee of the University of the Ryukyus. Time series changes in average food intake and average body weight were monitored in each condition.

Human amyloid precursor protein knock-in (APP-KI) mice are provided by Dr. Takaomi Saido of the RIKEN, Japan. This mouse was obtained by introducing mutations of Swedish KM670/671NL mutation (NL), the Artic E693G mutation (G), and the Iberian I716F mutation (F) into a human APP gene, and an NL mouse having one mutation and an NL-G-F mouse having all mutations were used for the experiment. All of these mice produce human amyloid beta (Aβ)40 and Aβ42 oligomers (Saito et al., Single App knock-in mouse models of Alzheimer's disease. Nat. Neurosci.

2014 17, 661-4). In the knock-in mouse, accumulation of Aβ in the brain is observed after 2 months (8 weeks).

Control and high-fat diet feed

As a control diet (CD) for mice, CE-2 feed (CLEA Japan, Inc., Tokyo) was used. The composition table of the CE-2 feed was as shown in Table 1. CE-2 feed had a total energy of 339.1 kcal/100 g and a crude fat of 4.61%. On the other hand, as a high-fat diet (HFD), F2HFD2 feed (Oriental Yeast Co., Ltd., Tokyo, Japan) was used. The total energy of F2HFD2 is 640 kcal and is composed of 58% lard (wt/wt), 30% fish meal, 10% skim milk, and 2% vitamin mineral mixture (equivalent to carbohydrate 7.5%, protein 24.5%, and fat 60%) (7). The other components of the F2HFD were similar to those of the CE-2. HFD was administered to a 4-week-old mouse group (F2HFD2 feed). Control mice were fed a low fat diet (CE-2 feed).

[Table 1]

| Table 1: Composition table of CE-2 feed (per 100g) | |
|---|---|
| | **CE-2 ( CD )** |
| Moisture (%) | 8.84 |
| Crude protein (g) | 25.48 |
| **Crude fat (%)** | **4.61** |
| Crude fiber (%) | 5.14 |
| Crude ash (%) | 7.01 |
| NFE (%) | 48.62 |
| **Energy (kcal)** | **339.1** |
| Ca (g) | 1.18 |
| P(g) | 1.1 |
| Mg (g) | 0.34 |
| K(g) | 1.07 |
| Mn (mg) | 1 0.67 |
| Fe (mg) | 30.1 |
| Cu (mg) | 0.85 |
| Zn (mg) | 6.87 |
| Na (g) | 0.34 |
| Ca/P | 1.07 |
| Ca/Mg | 3.47 |
| K/Na | 3.18 |
| Retinol (IU) | 1515 |
| Vitamin B1 (mg) | 1.7 |
| Vitamin B2 (mg) | 1.4 |
| Vitamin B6 (mg) | 1.3 |
| Vitamin B12 (μg) | 6 |
| Total vitamin C (mg) | 22.5 |
| Vitamin D3 (IU) | 260 |
| Vitamin E (mg) | 7.1 |
| Pantothenic acid (mg) | 2.7 |
| Niacin (mg) | 18.5 |

(continued)

| Table 1: Composition table of CE-2 feed (per 100g) | |
|---|---|
| | **CE-2 ( CD )** |
| Folic acid (mg) | 0.2 |
| Choline (mg) | 185 |
| Biotin ($\mu$g) | 54.2 |
| Inositol (mg) | 626 |

Perampanel (PER) oral administration

HFD mice were orally administered with HydroGel (Clear $H_2O$, Portland, ME 04101, USA) at a dose of 5 mg/kg body weight (HFD treated group with PER). For *ob/ob* mice, CE-2 feed (CLEA Japan, Inc., Tokyo) was used. HFD mice and *ob/ob* mice were orally administered one per cage with PER at a dose of 5 mg/kg body weight (HFD mice and *ob/ob* mice PER-administered group), and body weight and HydroGel intake were monitored twice a week for both groups to adjust the dose of PER to 5 mg/kg body weight.

Wheel-running activity

Wheel cages (MELQUEST, Japan, Model RWC-15) were used to monitor the activity of individual mice. The wheel rotation was monitored and recorded every 10 minutes over a 14 day period as previously reported (45).

Open field test and elevated plus-maze

The open field test was performed as follows. Mice were allowed to move freely for 5 minutes in a space enclosed by 50 cm square 40 cm high walls (Muromachi Kikai Co., Ltd., Japan) and their trajectories were analyzed using the CompACT VAS/DV video tracking system (Muromachi Kikai Co., Ltd., Japan) (46).
The elevated plus-maze test was performed as follows. The space installed 50 cm above the floor consisted of 2 open arms, 2 closed arms (30 cm $\times$ 6 cm each) and a neutral zone. Mice were disposed centrally in the neutral zone, facing the closed arms, and allowed to move freely for 3 minutes. Time spent in the open and closed arms and frequency of visits to different arms were recorded and scored using the CompACT VAS/DV video tracking system (Japan, Muromachi Kikai Co., Ltd.).

Novel object recognition test

Mice of CD, HFD, and HFD with PER treatment were handled daily for 5 minutes for 5 days prior to the start of the novel object recognition test. PER means the perampanel, which is an AMPA receptor antagonist. The mice were habituated to a 35 cm square, 25 cm high box for 10 minutes on the first day, and were given 2 identical objects (familiar objects) for 10 minutes on the second day. On the third day, one of the familiar objects was replaced with a novel object of different shape and color (Fig. 2F). The behavior of the mice was observed with a video camera for 5 minutes, and the videos were digitized and stored in a personal computer. The search time for each object was measured by offline analysis (47).

Morris water maze

Memory impairment was assessed using the Morris water maze test as previously described (48). In summary, the 120 cm diameter water maze pool (Muromachi Kikai Co., Ltd., Japan, Tokyo) contained opaque water (room temperature) and a platform (10 cm diameter) submerged 2 cm below the water surface. The hidden platform task was performed for 4 to 7 days (twice daily, 3 hour interval), during which two trials were performed each day (15 minute intervals). The platform location was kept constant and the entry points changed semi-randomly between the trials. A 1 minute probe trial was performed without the platform 24 hours after the last day of the hidden platform task. The entry point of the probe trial was set in the quadrant opposite to the target quadrant. Memory retention was evaluated at the time the escape platform was located in the correct quadrant in the hidden platform trial. Performance was monitored using a CompACT VAS/DV video tracking system.

Contextual and spatial recall mediated by pattern completion test

The Morris water maze task was performed as previously described using mice with CD, HFD, and HFD with PER treatment (49). All experiments were performed at approximately the same time of day. Mice were transported from the colony to the holding area and kept undisturbed for 30 minutes prior to the experiment. The tests were performed in a rectangular dimly lit room with a circular pool (Muromachi Kikai Co., Ltd., Tokyo, Japan) of 120 cm diameter filled with opaque water with skim milk (Morinaga, Japan) kept at room temperature. Four large objects illuminated by floor lamps were hung on the black curtains surrounding the pool as cues outside the maze. A hidden circular platform with a diameter of 10 cm was placed 1 cm below the surface of the water and the mice were trained to find the platform 4 times per day at intervals of approximately 60 minutes for 12 days. During training, the mice were released from 4 start points (N, S, E, and W) assigned in a pseudo-random manner and allowed to swim for 300 seconds. Mice that did not find the platform within 300 seconds were manually guided to the platform and rested on the platform for 15 seconds.

On the thirteenth day, a probe trial was performed under the condition of full-cue (P1). Mice were released at the center of the pool and allowed to swim in the absence of a platform for 300 seconds. The probe trial was followed by four training trials in the presence of the platform to avoid memory extinction that may have occurred during the probe trial. Thereafter, once a day, four probe trials with extra maze cue manipulations were performed and no retraining was performed during the probe trials. In one-cue probe trial (P2), one cue that was located more distantly from the platform was left and the other three cues were removed from the surrounding curtains. In two-cue probe trial (P3), one cue positioned near the platform and the one used in the one-cue probe trial were left, and the other two cues were removed from the surrounding curtains. In the no-cue probe trial (P5), all four extra maze cues were removed. Training and probe trial data were collected and analyzed using CompACT VAS/DV video tracking system software. The escape latency to the hidden platform (goal reaching time) was measured.

Five-trial social memory assay

Five-trial social tests were performed as previously described (19). In summary, subject mice with CD, HFD, and HFD with PER treatment were housed individually starting 7 days before the study to establish territory dominance. On the day of the test, a female mouse was presented in the cages of subject male mice, which is a subject (Fig. 2G), and 5 min trials were performed 4 times in succession at 10 min intervals. In the fifth trial, a novel female mouse was presented (Fig. 2G) and the duration of the social investigation was recorded.

Contextual fear conditioning

Contextual fear conditioning was applied with slight modifications to published protocols (50). Mice were transferred to the animal experimental room and allowed to acclimatize for at least 30 minutes prior to contextual fear conditioning training. Next, the mice were placed in a foot shock system model MK-450 MSQ (Muromachi Kikai Co., Ltd., Japan), searching was performed for 2 minutes, and then 3 electric foot shocks (0.8 mA, 2 sec, 2 min interval) were performed. After mice were left in the apparatus for an additional 1 minute, the animals were removed.

Obtainment of MRI data of mouse

Anatomical brain images of 8 ex vivo mice with CD, HFD, PER treatment were acquired using a Bruker BioSpec 117/11 11.75 Tesla MRI scanner (Bruker BioSpin GmbH, Ettlingen, Germany). MPRAGE (three-dimensional (3D)-prepared rapid gradient-echo) sequences were acquired as high resolution 100 um iso-voxel images for voxel-based morphometry (matrix size: $280 \times 220 \times 220$; field of view: $28 \times 22 \times 22$ mm, repetition time: 2000 ms, echo time: 1.78 ms, flip angle: 12 degrees, inversion time: 800 ms, echo train length: 13, average number of times: 2).

A region of interest was drawn in the acquired data image using a rapid acquisition protocol by relaxation enhancement (RARE) sequence (matrix size: $280 \times 220 \times 220$, field of view: $28 \times 22 \times 22$ mm; repeat time: 1500 ms; echo time: 25 ms; flip angle: 180°). The MPRAGE image and the RARE image were simultaneously acquired by one single scan.

MRI data acquisition of ex vivo mice was acquired by simultaneously placing the head parts of four mice fixed with PBS into an MRI coil. Brains of four mice were confirmed using raw MRI data.

Image preprocessing and estimation for voxel-based morphometry analysis

Voxel-based morphometry (VBM) analysis and preprocessing of MPRAGE and RARE images were performed using the SPM8 analysis tool (Wellcome Department of Clinical Neurology, London; http://www.fil.ion.ucl.ac.uk) and SPMMouse toolbox (http://www.spmmouse.org/).

The raw data were divided into four mouse head part MRI images and stored separately. The 3D (x-y-z-) coordinates of the divided images were transformed into the SPM standard coordinate system, and the origin of the 3D coordinates was defined as a bregma point. The brain images were then segmented into grey matter (GM), white matter, and cerebrospinal fluid using a segmentation tool (installed in the SPM8 system). Dividing into GM images was performed using the tissue probability map in the SPMMouse toolbox. These divided GM images improved contrast and were normalized to deformation of the images. Finally, these images were smoothed using a 200 um isotropic Gaussian kernel method (installed in the SPM8 system). The smoothed images were applied for VBM analysis.

The volume of subfields of the hippocampus (CA1, CA2, CA3, DG, EC) was calculated using the ROI file (31, 33, 51, 52). Differences between the average values of whole brain volumes of CD, HFD, and HFD with PER treatment were tested by one-way analysis of variance (ANOVA). When there was a significant difference between the 3 groups by ANOVA ($p < 0.05$), Scheffe post hoc analysis (Scheffe) was performed between the CD group and the HFD group; between the CD group and the HFD group with PER treatment; and between the HFD group and the HFD group with PER treatment.

Human studies

The participants of the study were 117 healthy volunteers (average age $37.8 \pm 19.6$ years, 65 females, 52 males) and 5 patients with benign tumors (average age $55.5 \pm 9.6$ years, 2 females and 3 males), all agreed in writing to participate in the study, and event-related memory tasks and T1-weighted images by 3T MRI were taken. The participants were divided into three groups based on body mass index (BMI) according to WHO criteria: normal body weight (BMI < 25), overweight (BMI $\geq$ 25 and < 30), and obesity (BMI $\geq$ 30). There were 84 participants in the normal body weight group (average BMI $20 \pm 1.8$), 27 participants in the overweight group (average BMI $26 \pm 1.2$), and 11 participants in the obese group (average BMI $32 \pm 2.3$). All experiments were carried out with the approval of the Ethics Committee on Medical and Health Research on humans at the University of the Ryukyus.

Behavioral task paradigm

Details of the fMRI experiment of the event-related memory task used in this study are described in the previous report (32). The memory task consisted of 108 photos of 16 luer sets (similar images), 16 repeat sets (identical images), and 44 novel items (new images). Participants were instructed to respond by pressing a button regarding whether the picture stimulus displayed on the display was a novel item (new), a repeated identical picture (same), or a picture that was similar but not the same as the previous picture (similar; lure). The button operation of the subject during the memory task was recorded in a personal computer, and the correct answer rate of new, same, and lure was calculated. The correct answer rate of each task (new task, similar task, same task) was calculated by the following equation.

Correct answer rate (%) = number of correct answers of presented tasks/total number of presented tasks $\times$ 100

In this experiment, the total number of tasks presented for a new stimulus was set to 76, and the total number of tasks presented for the same stimulus and a Luer stimulus was set to 16.

Acquisition of functional MRI data for behavioral task

Functional and structural images of the brain were acquired using 3T-MRI (Discovery MR 750; General Electric, Milwaukee, WI). As a functional image for measuring the BOLD contrast, echo planar imaging (EPI, repetition time: 1500 ms, echo time: 25 ms, flip angle: 70°, matrix size: 128 $\times$ 128, field of view: 192 $\times$ 192, in-plane resolution: 1.5 $\times$ 1.5 mm$^2$, 23 slices, 3 mm thickness, 0 mm space) was used. Anatomical brain images were acquired using a three-dimensional (3D) spoiled gradient recoil echo (SPGR) sequence (slice thickness in sagittal section: 1 mm, matrix size: 256 $\times$ 256, field of view: 256 $\times$ 256 mm, repetition time: 6.9 ms, echo time: 3 ms, flip angle: 15°). High-resolution T2-weighted fast spin echo sequences (matrix size: 512 $\times$ 512, field of view: 192 $\times$ 192 mm, repetition time: 4300 ms, echo time: 92 ms, in-plane resolution: 0.375 $\times$ 0.375 mm$^2$, 23 slices, thickness: 3 mm, space: 0 mm) were acquired to visualize the structure of the hippocampus and to perform co-registration of 3D SPGR images and EPI functional images.

Image processing of behavioral task fMRI

Using the SPM12, preprocessing of realignment, temporal correlation, spatial normalization, and spatial smoothing of the functional image was performed and analyzed. Based on the hippocampus atlas (Duvernoy), a lower region of the hippocampus (CA3, CA1, DG) and a region around the hippocampus (parahippocampal gyrus, perirhinal cortex, entorhinal cortex) were drawn by handwriting using a pen tablet on a high-resolution coronal T2-weighted image. The percentage of signal change in the BOLD response in the hippocampal region of each subject was extracted using MarsBar toolbox. 3D-SPGR images were used for analysis of voxel-based morphometry. The T1-weighted image was segmented into GM, white matter, and cerebrospinal fluid images using the SPM12 segmentation tool. The volume of GM throughout the brain was calculated after spatial normalization and modulation of the GM image.

Image acquisition for functional connectivity analysis

MRI data was acquired using a GE Medical Discovery MR 750 3T scanner (with 32 channel head coil). Subjects lying on the scanner bed in a supine position had their head and neck clamped with foam pads and Philadelphia neck collar to minimize head movement. Resting-state fMRI images were acquired using a single shot EPI sequence covering the whole brain (42-axis slice, thickness 4 mm, no gap between slices, repetition time 2000 ms, echo time 30 ms, flip angle 70°, matrix size 64 × 64, field of view 256 × 256). A total of 150 volumes were captured in one session. Anatomical brain images were acquired using a T1-weighted sagittal 3D SPGR sequence.

Preprocessing and analysis of data for functional network

Image preprocessing and functional network analysis were performed using SPM12 and CONN toolbox 18.b (www.nitrc.org/projects/conn, RRID: SCR_009550 [Whitfield-Gabrieli and Nieto-Castanon, 2012]). Details are as described in the previous report (32). The image was preprocessed in the order of realignment, slice-timing correction, coregistration, normalization, smoothing, and segmentation. The noise of the BOLD signal was removed by linear regression of potential confounding effects contained in the BOLD signal and by temporal bandpass filtering (time frequency was 0.008 Hz or lower or 0.09 Hz or higher).

In the functional network analysis using the CONN toolbox, the default mode network (DMN) and the functional connectivity were analyzed. First, as an individual analysis, regions that had a positive correlation with BOLD fluctuations in the posterior cingulate cortex (PCC) and precuneus were calculated as a DMN map. Next, a region having a negative correlation with the DMN map was calculated as an anti-correlation DMN map. The average images of the DMN map and the anti-correlation DMN map of the normal body weight group, the overweight group, and the obese group were calculated using the one-sample t-test (the threshold of the voxel level was $p < 0.001$ (without correction), and the threshold of the cluster level of the false discovery rate [FDR] was $p < 0.05$ (with correction).).

In the functional combination analysis, nodes and edges of graph theory parameters were calculated. The correlation coefficient of the fluctuation of the BOLD signal between each ROI was calculated with a seed-based connectivity measurement method (53) using a 132 × 132 ROI map (default ROI atlas of CONN toolbox). The ROI-to-ROI degree (the number of edges of any node between nodes) and betweenness centrality (the number of shortest paths for a vertex through any two pairs of nodes $(j, i)$ in the graph) were calculated by ROI-to-ROI correlation 132 × 132 matrix established using our 132 × 132 ROI map.

Degree is defined as the number of edges from/to each node at each node.

The degree (di) is defined by the following formula.

[Mathematical formula 1]

$$ di = \sum_j Ai,j $$

Here, $A_{i,j}$ represents the correlation between ROI and ROI in a 132 × 132 matrix.

$$ i = 1,2,3....., 132;\ j = 1,2,3...., 132 $$

Betweenness centrality (BCi) represents a hub that connects other functional modules and nodes (54), and $BC_i$ is defined as follows.

[Mathematical formula 2]

$$BC_i = \frac{\sum_{j,k \neq i}[i \in P_{j,k}]}{(N-1)(N-2)}$$

Here, $P_{j,k}$ is the number of nodes in the shortest path between each pair of nodes $(k, j)$ (the shortest path when passing through any node defined with $i$ as a variable), and $N$ is the number of all nodes in the graph of the ROI map.

[Mathematical formula 3]

$$j,\ k \neq i\ ;\ N = i \times j = 132^2$$

The average value of each node and the number of edges were calculated for each of the normal body weight group, the overweight group, and the obese group. In addition to PCC and precuneus in DMN, anterior cingulate cortex, cerebellar lobules *CrusI,* and hippocampus related to memory function of hippocampus were set as a region of interest as described in the previous report (32), and functional connectivity of 3 groups was analyzed. Functional connectivity maps for each group were shown using a BrainNet viewer (https://www.nitrc.org/projects/bnv/) (55).

$Ca^{2+}$ imaging of brain slices

Hippocampal brain slices were prepared from 17 to 18 week old male C57BL/6J background mice, and according to previous reports (56, 57), mice were divided into the CD group, the HFD group, and the HFD group with PER treatment (HFD with PER), or 12 to 13 week old ob/ob mice were divided into ob/ob and ob/ob groups with PER treatment. After cutting out slices (thickness, 300 to 400 $\mu$m) using a Linear Slicer (PRO 7N, Dosaka EM, Japan), hippocampal brain slices were incubated in normal Krebs Ringer (125 mM NaCl, 2.5 mM KCl, 10 mM D-glucose, 1.25 mM $NaH_2PO_4$, 26 mM $NaHCO_3$, 2 mM $CaCl_2$, 1 mM $MgCl_2$, with continuous bubbling of mixed gas [95% $O_2$]; 5% $CO_2$]) for 60 min at room temperature to restore cutting damage. The $Ca^{2+}$ indicator Fluo-3 AM (excitation wavelength: 508 nm, emission wavelength: 525 nm, Kd, 0.4 $\mu$mol/L) (5 $\mu$M) (Dojindo, Kumamoto, Japan) was loaded into brain slice specimens for 90 minutes. To monitor $[Ca^{2+}]_i$, the fluorescence intensity (F525) of Fluo-3 was monitored using a photomultiplier tube under a confocal microscope (excitation wavelength: 488 nm; LSM5 PASCAL, Carl Zeiss, Germany). In this experiment, Ca imaging of the entire hippocampal slice was performed using a low magnification (2.5 times) objective lens (FLUAR 2.5×, NA = 0.12, Carl Zeiss, Germany) (Fig. 1). Fluo-3 fluorescence images (512 × 512 pixels) were digitized and stored on a personal computer every 10 seconds for 50 minutes. F525 in each region of CA1, CA2, CA3, DG, and EC was normalized by an average value of F525 (NF525) (value 0 to 5 minutes before application of AMPA or NMDA) under offline analysis (Microsoft Excel, Microsoft Corporation, WA). During Ca imaging, brain sections were perfused with an extracellular solution (35 mm $\mu$-dish, Ibidi GMBH, Graefelfing, Germany) containing 125 mM NaCl, 2.5 mM KCl, 10 mM D-glucose, 1.25 mM $NaH_2PO_4$, 26 mM $NaHCO_3$, 2 mM $CaCl_2$, 1 mM $MgCl_2$, and 1 $\mu$M tetrodotoxin at 2 mL/min with continuous bubbling of a mixed gas (95% $O_2$; 5% $CO_2$) in a perfusion chamber. In the case of applying NMDA, the concentration of $MgCl_2$ was set to 0 mM in order to prevent $Mg^{2+}$ dependent inhibition of the NMDAR (58). The maximum value of NF525 was estimated as the maximum value of NF525 within 5 minutes after application of AMPA (Tocris, Bristol, UK) (100 $\mu$M) or NMDA (Tocris, Bristol, UK) (50 $\mu$M). Cyclothiazide (CTZ) (100 $\mu$M) was used to prevent desensitization of AMPAR, glycine (Tocris, Bristol, UK) (10 $\mu$M) was used to activate NMDAR, respectively, and perampanel (Eisai Co, Ltd., Tokyo, Japan) (100 $\mu$M), GYKI 47261 (Tocris, Bristol, UK; hereinafter also simply referred to as "GYKI") (100 $\mu$M), or (2R)-amino-5-phosphovaleric acid (APV) (Tocris, Bristol, UK) (50 $\mu$M) was used as an antagonist of AMPAR or NMDAR, respectively. As an antagonist of $Ca^{2+}$ permeable AMPAR, 1-naphthylacetylspermine (NASPM) (Tocris, Bristol, UK) (20 $\mu$M) was used (59).

Surface AMPAR subunits of cross-linking with $BS^3$

Crosslinking using the $BS^3$ procedure carried out here was carried out based on previously described methods (60). Hippocampal slices of 17 to 18 week old male C57BL/6J background mice (CD, HFD, and HFD with PER treatment) or 12 to 13 week old *ob/ob* mice (*ob/ob,* and *ob/ob* with PER treatment) were prepared as previously reported (56, 57). Hippocampal slices were prepared and collected, and then CA1, CA2, CA3, DG, and EC regions were separated from the hippocampal slices using a dissection knife. CA1, CA2, CA3, DG, and EC regions were added to an Eppendorf tube (Eppendorf, Hamburg, Germany) containing ice-cold artificial cerebrospinal fluid (ARTCEREB; Otsuka Pharmaceutical Co., Ltd., Tokyo, Japan) supplemented with 2 mM BS³ (Thermo Fisher Scientific, Wilmington, DE, USA). Incubation was performed on ice for 30 minutes. Crosslinking was terminated by quenching the reaction with 100 mM glycine (10 min at 4°C). Hippocampal subregions were resuspended in ice-cold lysis buffer (25 mM HEPES, pH 7.4, 500 mM NaCl, 2 mM EDTA, 1 mM DTT, 1 mM phenylmethylsulfonyl fluoride, 20 mM NaF, 1 mM sodium orthovanadate, 10 mM sodium pyrophosphate, 1x protease inhibitor mixture [Sigma-Aldrich, St Louis, MO], and 0.1% Nonidet P-40 [v/v]) containing protease and phosphatase inhibitors and rapidly homogenized with 5 sec ultrasonication. The total protein concentration of the lysate was measured using the Lowry method (61). Samples were dispensed (divided into approximately 15 per mouse) and stored at -80°C for later analysis. BS³ samples were analyzed by SDS-PAGE directly without purification, and surface and intracellular bands were measured in the same lane, thus no normalization was required and sample throughput was improved. Total protein lysate (40 μg) was loaded and electrophoresed on 5% to 10% Tris-HCl gel (Thermo Fisher Scientific, Wilmington, DE) under reducing conditions and proteins were transferred to nitrocellulose membranes (Thermo Fisher Scientific, Wilmington, DE) for immunoblotting. The membrane was blocked at room temperature for 30 minutes using a blocking reagent Blocking One (Nacalai Tesque, Tokyo, Japan). The membrane was then incubated overnight at 4°C with anti-GluA1 (1:1,000, Merck Millipore, Burlington, MA) and actin (1:5,000, Protein Teck, Chicago, IL, USA). Membranes were incubated with HRP-labeled anti-rabbit IgG (1:3,000, Cell Signaling Technology, Danvers, MA, USA) for 30 min and extensively washed again with TBS-T. After soaking the membrane in the chemiluminescence detection substrate Chemi-Lumi One (Nacalai Tesque, Tokyo, Japan) for 1 min, luminescence was detected using a LuminoGraph1 imaging system (Atto, Tokyo, Japan). The surface and intracellular bands of each lane were analyzed using a CS Analyzer (Atto, Tokyo, Japan).

RT-PCR

Hippocampal slices of 17 to 18 week old male C57BL/6J background mice (CD, HFD, and HFD with PER treatment) or 12 to 13 week old *ob/ob* mice (*ob/ob,* and *ob/ob* with PER treatment) were prepared as previously reported (56, 57). After preparing and collecting hippocampal slices, CA1, CA2, CA3, DG, and EC regions were separated from hippocampal slices using a dissection knife from 57BL/6J background mice and *ob/ob* mice, respectively. The CA1, CA2, CA3, DG, and EC regions of mice were separated and immediately lysed in disposable homogenization tube BioMasher2 (trademark) (Nippi, Tokyo, Japan) using 0.5 mL of TRIzol RNA isolation reagent (Thermo Fisher Scientific, Wilmington, DE). Total RNA was individually extracted from each region according to the remaining protocol of the TRIzol RNA isolation reagent provided by the manufacturer (Thermo Fisher Scientific, Wilmington, DE). 1 μg of total RNA was reverse transcribed using PrimeScript RT Reagent Kit (Takara, Shiga, Japan). An aliquot of the obtained cDNA was diluted 1:10 and added to a master mix of TB Green Premix ExTaq (Takara, Shiga, Japan), and real-time PCR was performed using a 7500 Fast Real-Time PCR system (Applied Biosystems, Foster City, CA, USA) according to the manufacturer's instructions. The conditions for real-time PCR were as follows. 40 cycles of 95°C for 30 seconds, then 95°C for 5 seconds and 60°C for 34 seconds. The fluorescence intensity was measured for each annealing step and the threshold cycle time was determined using 7500 software. In addition, relative expression levels of β-actin were normalized to respective controls (vehicle group). The relative expression level of the target gene in each sample was determined using a standard curve and further normalized to the expression level of β-actin of the same sample. The sequences of primers and probes for these genes are shown in Supplemental Table 2. The "ratio of GluA2 mRNA amount/(GluA1 + GluA3 + GluA4 mRNA amount" was used as a $Ca^{2+}$ permeability index.

[Table 2]

| Table 2: Mouse primer set for RT-PCR | | |
| --- | --- | --- |
| Gene name | Forward primer | Reverse primer |
| β-actin | ACAGCTTCACACAGCT (SEQ ID NO: 10) | TGCGAGTGGCCATCTCCT (SEQ ID NO: 11) |
| GluAl | Ctgtgaatcagaacgcctca (SEQ ID NO: 12) | ggaaacctgtcacattggct (SEQ ID NO: 13) |
| GluA2 | ccctggagcacacagcqa (SEQ ID NO: 14) | ctgcttccgaagattgcgga (SEQ ID NO: 15) |
| GluA3 | gtgcagttatacaaccaacca (SEQ ID NO: 16) | gagcagaaagcattagtcacaga (SEQ ID NO: 17) |
| GluA4 | GGCTCAGTGCACTCA (SEQ ID NO: 18) | CTCTGCAGTGCGACACA (SEQ ID NO: 19) |
| GluN1 | CTCGCATGCAAGAC (SEQ ID NO: 20) | tgtcttatccaggtcttcca (SEQ ID NO: 21) |
| GluN2A | gggtcacagagcaacatgct (SEQ ID NO: 22) | agctcttttgggtgagtcca (SEQ ID NO: 23) |
| GluN2B | gtgagagctcctttgccaac (SEQ ID NO: 24) | TCAGAGAGCGAACTGCT (SEQ ID NO: 25) |

(continued)

| Table 2: Mouse primer set for RT-PCR | | |
|---|---|---|
| Gene name | Forward primer | Reverse primer |
| GluN2C | aaggcctttgtcattgtgg (SEQ ID NO: 26) | cttgaggatgtcgatgcaga (SEQ ID NO: 27) |
| GluN2D | gactactccttcaatgagga (SEQ ID NO: 28) | gttcccagctgcccaccact (SEQ ID NO: 29) |
| GluN3A | ccagtcagggtttcacaga (SEQ ID NO: 30) | CCATCTTCCATCTGACTACT (SEQ ID NO: 31) |
| GluN3B | cttggctaggttctgagca (SEQ ID NO: 32) | GCGTAAGCTCCATACCTTGA (SEQ ID NO: 33) |
| ADAR1 | AAGACTCGGGAAACAGCA (SEQ ID NO: 34) | CCTTCTTGGACAGAGACG (SEQ ID NO: 35) |
| ADAR2 | TGCTGGACTACTGTACTTCA (SEQ ID NO: 36) | ccagagcactttccttcg (SEQ ID NO: 37) |
| ADAR3 | ccaggaaagtctccactt (SEQ ID NO: 38) | $\alpha$cgtgtgctagcctaacat (SEQ ID NO: 39) |
| REST | acatgcggactcattcaggt (SEQ ID NO: 40) | gaggtttaggcccgttgtga (SEQ ID NO: 41) |
| RPSB | gctttcgctctcctgcttga (SEQ ID NO: 42) | T GT GGGAAC T GGCAGC T (SEQ ID NO: 43) |

Sanger sequence

A template for Sanger sequencing of the GluA2 editing site was prepared by amplifying cDNA using a forward primer (GAGGAATTTGAAGATGGAAGAGA: SEQ ID NO: 44) and a reverse primer (AGGAGGAGATGATGATGAGGGT: SEQ ID NO: 45). PCR products (10%) were confirmed by agarose gel electrophoresis. After confirming the appropriate expected size, the PCR product was purified according to the manufacturer's instructions using innuPREP PCRpure Lite Kit (Analytik Jena, Jena, Germany) prior to direct sequencing. The cycle sequence was performed using the BigDye Terminator v 3.1 Cycle Sequencing Kit (Applied Biosystems, CA, USA) and each primer described above. Sanger sequence analysis was performed using the ABI 3500 (Applied Biosystems, CA, USA) and sequence viewer software 4 Peaks.

Editing assay

The Q/R editing status of GluA2 was analyzed using a Quant Studio™ 3D digital PCR system equipped with a TaqMan (trademark) custom SNP genotyping assay (Thermo Fisher Scientific, Wilmington, DE). The TaqMan probe was synthesized by Thermo Fisher Scientific Inc., and a VIC-labeled probe was used for detection of the non-edited (Q) sequence, and a FAM-labeled probe was used for detection of the edited (R) sequence (5'CATCCTTGCTGCATAAA3': SEQ ID NO: 46, 5'ATCCTTGCCGCATAAA3': SEQ ID NO: 47, respectively).

The following amplification primer pairs were used for the PCR reaction:

mouse forward primer:
5'TTGGGATTTTTAATAGTCTCTGGTTTTCCTT3' (SEQ ID NO: 48) and
mouse reverse primer: 5'GACCAACCTTGGCGAAATATCG3' (SEQ ID NO: 49). 5 to 50 ng of cDNA was mixed with a digital PCR master mix (Thermo Fisher Scientific, Wilmington, DE) and the mixed TaqMan probes were loaded onto a QuantStudio™ 3D digital PCR 20 K chip (Thermo Fisher Scientific, Wilmington, DE, USA) using an automated chip loader according to the manufacturer's instructions. The loaded chips were amplified using Gene Amp 9700 PCR system (Thermo Fisher Scientific, Wilmington, DE, USA) under the following conditions. A cycle of 96°C for 10 minutes, 56°C for 2 minutes, and 98°C for 30 seconds was repeated 39 times, followed by a final extension step of 60°C for 2 minutes. After amplification, the chips were imaged using QuantStudio 3D Instrument (Thermo Fisher Scientific, Wilmington, DE, USA). For analysis of chip data, relative and quantitative data analysis was performed using QuantStudio 3D Analysis Suite Cloud Software (Thermo Fisher Scientific, Wilmington, DE).

Golgi staining and image acquisition of entire hippocampus

Coronal sections of mouse brains from Bregma -0.94 mm to Bregma -4.04 mm (5 mm wide, including the entire hippocampus from dorsal to ventral, medial to lateral) were fixed with 4% paraformaldehyde for 2 days, and then stained with the Golgi Cox staining system (FD Rapid GolgiStain™ Kit, MD 21041, USA). The impregnated tissues were cut into 100 um sections and counterstained with crystal violet, and the total number of 15 um spines on the apical dendrites of CA1 and DG and the percentage of morphologic spines (thin, stubby, mushroom) were examined using an Axio Observer Z1 (Carl Zeiss, Germany). Another set of paraformaldehyde-fixed 5 mm wide coronal sections of the brain, including the entire hippocampus, were processed using the passive CLARITY technique (PACT) (62) and examined through Lightsheet Z.1 (Carl Zeiss, Germany) using a 10x clear objective lens, and 3D images of Thy1-YFPH transgenic mice (the Jackson Laboratory, stock number: 003709, strain name: B6.Cg-Tg. (Thy1-YFP) 16Jrs/J) (63, 64), HFD-fed mice, and whole hippocampus of the HFD-fed mice with PER treatment. were acquired respectively. Maximum intensity projection images were acquired along the Z dimension, time dimension, and channel dimension to create an output image with the pixel containing the maximum value for all images in the stack at a particular pixel position. With the Z-stack function, a series of XY images at different focus positions can be acquired to create Z-stack. In this manner, a 3D dataset of 400 $\mu$m $\times$ 400 um was obtained from the specimens, dorsal to lateral and medial to lateral for each of CA1, CA3, and DG. In the gallery view, the images from the Z-stack are displayed in chronological order.

Quantitative analysis of number of Thy1-YFPH[+] cells

3D reconstruction was performed using Arivis Vision 4-dimensional (4D) software. Blob finder (filter) was used for rounded 2D and 3D segments, close to spherical shape, out of noisy images. The Gaussian scale was used to find the seeds of the object and specify the boundaries of the object with the watershed algorithm. The average size of the structure of interest was set to 20 um and the threshold was set to 5. High resolution rendering is an approach that visualizes the current view with higher image and data resolution.

Immunohistochemical analysis

Mouse brains were perfusion-fixed with 4% paraformaldehyde, embedded in paraffin, and cut into 4 micrometer thick sections to be used for immunohistochemical analysis of monoclonal antibodies against DCX (E-6 monoclonal, 1:50, Santa Cruz Biotechnology Inc, Dallas, USA), MAP2ab (AP-20 monoclonal, 1:100, SIGMA-ALDRICH, St. Louis, USA), GluA1 (AB1540 polyclonal, 1:100, EMD Millipore Corp., Burlington, USA), and GluA2 (AB 1768-1 polyclonal, 1:5, EMD Millipore Corp., Burlington, USA).

Quantitative immunostained regions by ZEISS ZEN Intellization software

The immunostained region in each of the expression regions of MAP2, GluA1, and GluA2 was acquired using a ZVI format AxioVision (Carl Zeiss, Germany) microscope (objective lens 20X). Generation of data image processing with image segmentation was performed using machine learning with ZEN Intelliesis software. 1000 or more cells were examined in each group. One-way variance analysis was used for testing variance among the three groups excluding data of 3SD or higher. Then, the following number of samples was used.

HFD series

MAP2 CD n = 731, HFDs n = 288, HFD+PER n = 225; GluA1 CD n = 220, HFD n = 156, HFD+PER n = 94; GluA2 CD n = 78, HFD n = 114, HFD+PER n = 182; GluA2/GluA1 CD n=78, HFD n=94, HFD+PER n=94

*ob/ob* series

MAP2 *ob/ob* control n = 849, *ob/ob* PER 1 w n = 967, *ob/ob* + PER 12 w n = 511; GluA1 ob/ob control n = 163, *ob/ob* PER 1 w n = 124, *ob/ob* + PER 12 w n = 242. GluA2 *ob/ob* control n = 39, *ob/ob* PER 1 w n = 44, *ob/ob* + PER 12 w n = 369, GluA2/GluA1 *ob/ob* control n = 54, *ob/ob* PER 1 w n = 41, *ob/ob* + PER 12 w n = 234.
Bonferroni method was used for post hoc test to analyze significant differences between groups.

RNA-seq analysis

Sequenced raw RNA-seq fastq reads were aligned to the genome of mouse GRCm 38 (Ensemble Release 104, http://ftp.ensembl.org/pub/release-104/fasta/mus_musculus/dna/) using HISAT2 (v.2.2.0) (65). The average mapping rate of all samples was 94.21% (range 88.70 to 96.30%). Aligned reads were quantified using Salmon (v.0.14.2) (66) and TPM values were calculated using StringTie (v.2.1.2) (67). Variant cools were performed using GATK package (v.3.8) (70) according to GATK Best Practice for RNAseq short variant discovery (68, 69). The addition of the read group information, the sorting, the creation of the duplication mark, and the creation of the index were performed using a tool of Picard. GATK tool Split N Cigar Reads were used to split reads into exon segments and hard clip sequences that protrude into intron regions. Variant coating and filtration were performed using GATK HaplotypeCaller and VariantFiltration, respectively. The functional annotation of the output variants was performed using SnpEff (v.4.3) (71). RNA-editing levels were calculated as the ratio of the total number of reads aligned to the R/Q editing site of GluA2 (chr3: 80706912) to the number of reads that were T-C-converted at this site.

Statistical analysis

In animal model experiments, statistical analysis was performed using one-way ANOVA and Bonferroni's multiple comparison test and/or two-tailed t-test. Statistical significance was $p < 0.05$. In the human experiment, the relationship between BMI, GM volume of whole brain, and body weight, and the correct answer rate for the task condition (new, similar, same) was analyzed by partial correlation analysis. Furthermore, the difference between the average brain activity and the correct answer rate under the three task conditions (new, similar, same) was analyzed by one-way analysis of variance.

Results

HFD stimulates calcium influx via AMPAR in mouse hippocampus

First, the intracellular calcium concentration ($[Ca^{2+}]i$) was directly measured in an acute brain slice of the hippocampus having a perihippocampal region using a mouse obesity model associated with HFD feeding (composition of diet (7) shown in Table 1) for 12 weeks after weaning to track calcium signaling (Fig. 1A: supplementary data moving image 1) in the hippocampus. HFD-fed mice received 100 $\mu$M AMPAR containing 100 $\mu$M CTZ (cyclothiazide; channel desensitizer) applied to EC (entorhinal cortex), CA1 (cornu ammonis 1), CA2, CA3, and DG (dentate gyrus). The F525 normalized before and after application was compared. Evaluating the normalized maximum value of F525 (NF525) as an indicator, a marked activation of calcium signal transduction via AMPA type receptors was observed in the HFD group (Fig. 1B, C, D, and E and Table 3).

[Table 3]

Table 3: Comparison of CP-AMPA and NMDA reaction in regions of hippocampus of mice of CD, HFD, and HFD with PER

| CP-AMPA | CA1 | CA2 | CA3 | DG | EC |
|---|---|---|---|---|---|
| CD | 1.00 ± 0.01 (N=5)) | 1.00 ± 0.01 (N=5) | 1.03 ± 0.03 (N=5) | 1.01 ± 0.03 (N=5) | 1.09 ± 0.02 (N=5) |
| HFDs | 1.07 ± 0.06 (N=5) | 1.09 ± 0.04 (N=5) | 1.05 ± 0.08 (N=5) | 1.04 ± 0.07 (N=5) | 1.08 ± 0.08 (N=5) |
| HFDs with PER | 1.00 ± 0.01 (N=5) | 1.00 ± 0.02 (N=5) | 1.00 ± 0.01 (N=5) | 1.02 ± 0.01 (N=5) | 1.05 ± 0.05 (N=5) |

| NMDA | CA1 | CA2 | CA3 | DG | EC |
|---|---|---|---|---|---|
| CD | 1.18 ± 0.02 (N=5) | 1.18 ± 0.02 (N=5) | 1.17 ± 0.01 (N=5) | 1.17 ± 0.02 (N=5) | 1,18 ± 0.01 (N=5) |
| HFDs | 1.20 ± 0.08 (N=5) 1 | 1.01 ± 0.01 (N=5) | 1.04 ± 0.02 (N=5) | 1.03 ± 0.03 (N=5) | 1.02 ± 0.01 (N=5)) |
| HFDs with PER | 1.20 ± 0.09 (N=5) | 1.01 ± 0.01 (N=5) | 1.12 ± 0,04 (N=5) | 1.17 ± 0.04 | (N=5) 1.16 ± 0.06 (N=5) |

Inactivation of NMDA type receptors elicited by intracellular $Ca^{2+}$ via AMPA type receptors

In contrast, calcium signal transduction via 50 $\mu$M NMDA (N-methyl-D-aspartic acid) with 10 $\mu$M glycine (channel coactivator) was inactivated in hippocampi derived from HFD-fed mice, whereas mice fed CD (control diet: normal diet but not high-fat diet) showed activation via the hippocampus (Fig. 1F, G, H, I, and J and Table 3).
In hippocampal slices from mice fed HFD and perampanel (PER) (Fycompa (trademark), Eisai, Japan) (5 mg/kg/day orally administered by hydrogel), mice treated with a new non-competitive AMPAR antagonist (8) were identified to normalize transmission via AMPAR (Fig. 1D, Table 3). In addition, signal transduction via the NMDAR was simultaneously restored (Fig. 1I, Table 3, and Supplementary Movie 3). Induction of elevation of $[Ca^{2+}]i$ via AMPAR in acute slices from HFD-fed mice completely disappeared by 100 $\mu$M PER (Fig. 1B-D). These facts suggest that calcium influx via AMPAR induced inactivation of NMDA receptor (NMDAR). As a result, a significant increase in $[Ca^{2+}]i$ by application of 100 $\mu$M AMPAR and 100 $\mu$M CTZ was observed in EC, DG, CA3, CA2, and CA1 in HFD-fed mice compared to CD fed mice, while a significant inactivation by 50 $\mu$M NMDA and 10 $\mu$M glycine was also observed (Fig. 1E, J). These results strongly suggested that HFD alters the expression of glutamate receptor subunits. Changes in $[Ca^{2+}]i$ via AMPAR were observed in mice fed HFD for at least 7 days (Figs. 7A, 7B, and 7C). Interestingly, in these mice, NMDAR-mediated activation was maintained in EC, DG, CA3, CA2, and CA1 even when 50 $\mu$M NMDA added 10 $\mu$M glycine was applied in the bath. Again, the increase in $[Ca^{2+}]i$ via these pathological AMPARs completely disappeared by addition of 100 $\mu$M PER, as well as other AMPAR antagonists, such as 100 $\mu$M GYKI 47261 (Tocris, UK, Bristol) and 20 $\mu$M NASPM (Tocris, UK, Bristol), which is a selective antagonist of $Ca^{2+}$ permeable AMPAR (CP-AMPAR), similarly, the increase in $[Ca^{2+}]i$ via pathological AMPAR disappeared (Fig. 7A-D). In an *in vitro* artificial system, it has been proposed that entry of $Ca^{2+}$ via calcium-permeable AMPAR results in inhibition of NMDAR by $[Ca^{2+}]i$ (9), but its physiological significance and association with diseases have been unknown. The above results show that in overweight or obese subjects, inhibition of NMDAR by a calcium-permeable AMPA receptor can be found and resolved by an AMPA receptor antagonist.

Permeable AMPAR upregulation in Alzheimer's disease model

Experiments similar to the above were performed on Alzheimer's disease models. As the Alzheimer's disease model, NL mice and NLGF mice were used. NL and NLGF mice were fed CD. The results were as shown in Fig. 1-2 (CP-AMPA) and Fig. 1-3 (NMDA). As shown in Fig. 1-2, in the Alzheimer's disease model (NL and NLGF), application of AMPA + CTZ induced elevation of $[Ca^{2+}]i$. This suggests that the AMPA receptor is a calcium-permeable AMPA receptor. In contrast, as shown in Fig. 1-3, in the Alzheimer's disease model (NL and NLGF), NMDA receptor was not activated and did not induce an increase in $[Ca^{2+}]i$ even by NMDA + glycine. As described above, also in the Alzheimer's disease model, it was found that the inhibition of NMDAR by the calcium-permeable AMPA receptor can be eliminated by the AMPA receptor antagonist.

Upregulation of calcium permeable AMPAR by HFD

$Ca^{2+}$-permeable AMPAR (CP-AMPAR) is associated with pathophysiology underlying various neurological diseases, such as brain tumors (10), amyotrophic lateral sclerosis (ALS) (11), cocaine addiction (12), neuropathic pain (13), and epilepsy (14). The molecular diversity behind pathophysiology is different in each disease and is not well understood. However, based on the above results, altered calcium dynamics in $[Ca^{2+}]i$ via AMPAR and NMDAR may play an important role in obesity with cognitive decline.

AMPAR mediates the fastest rate of excitatory neurotransmission. AMPAR consists of four subunits GluA1-4 that determine the functional properties of AMPAR channels, and the diversity of AMPAR depends on the abundance of these subunits. Specifically, other subunits of GluA1, GluA3, and GluA4 are $Ca^{2+}$ permeable, whereas the GluA2 subunit is essentially $Ca^{2+}$ impermeable. In addition, the $Ca^{2+}$ permeability of AMPAR is regulated by the relative amount of GluA2 expression. That is, it is considered that the $Ca^{2+}$ permeability of AMPAR decreases as the relative amount of GluA2 subunit increases, and the $Ca^{2+}$ permeability of AMPAR increases as the relative amount decreases.

GluA2 is edited at the Q/R site within the reentry M2 membrane loop region by adenosine deaminase (ADAR2), which acts on RNA type 2 involved in editing double-stranded RNA from adenosine (A) to inosine (I) (review, refer to 15, 16 and 17). Attempts were made to determine real-time quantitative reverse transcription polymerase chain reaction (qRT-PCR), subunit composition of AMPAR and NMDAR. Total RNA in hippocampal subregions (CA1, CA3, DG, EC) was separated, and quantitative analysis of mRNA of GluA1, GluA2, GluA3, and GluA4 for AMPAR, and GluN1, GluN2A, GluN2B, and GluN2C for NMDAR was performed by qRT-PCR. As a result, a significant increase in GluA1, GluN1, and GluN2B expression in the CA3 region associated with HFD-fed mice, an increase in GluN2B expression in DG ($p < 0.05$, respectively), and a significant increase in GluA2 in CA1 in mice fed HFD with PER ($p < 0.05$) (Fig. 2A) were found. Based on the mRNA amount of each subunit, the ratio of GluA2 to other GluA subunits (Fig. 2B) was determined. The ratio was defined as a $Ca^{2+}$ permeability index.

[Table 4]

| Table 4: Ca2+ permeability index of the hippocampal regions CA1, CA3, DG, and EC of CD-fed mice, HFD-fed mice, and HFD-fed mice with PER | | | | |
|---|---|---|---|---|
| $Ca^{2+}$-pormeability index | CA1 | CA3 | DG | EC |
| CD | $0.17 \pm 0.01$ (N=5) | $0.21 \pm 0.14$ (N=5) | $0.21 \pm 0.14$ (N=5) | $0.21 \pm 0.14$ (N=5) |
| HFDs | $0.17 \pm 0.10$ (N=5) | $0.18 \pm 0.10$ (N=5) | $0.19 \pm 0.17$ (N=5) | $0.19 \pm 0.08$ (N=5) |
| HFDs with PER | $0.29 \pm 0.11$ (N=5) | $0.26 \pm 0.10$ (N=5) | $0.22 \pm 0.06$ (N=5) | $0.30 \pm 0.05$(N=5) |

The reduction in $Ca^{2+}$ permeability index (Fig. 2B, Table 4) by HFD-fed mice via CA1, CA3, DG, and EC (N = 5) returned to CD-fed mouse levels (N = 5) upon PER (N = 5) administration (Fig. 2B, Table 4). These data indicate that the extent of GluA2 expression of AMPAR in the hippocampus is reduced under HFD feeding conditions compared to CD, suggesting that this reduction is restored by PER administration. Downregulation of GluA2 content in AMPAR may play an important role in upregulating AMPAR $Ca^{2+}$ permeability in HFD mice (Fig. 1A, C). Western blot analysis of GluA1 subunits showed remodeling in hippocampal neuronal circuits of ionotropic glutamate receptor expression from CP-AMPAR to $Ca^{2+}$ impermeable receptors by PER administration in HFD-fed mice. Crosslinking assays using BS3, a crosslinker capable of determining surface receptors and intracellular receptors, showed a 3-fold increase in GluA1 in the HFD group (n = 4) compared to the CD group, and treatment with PER (n = 4) reduced GluA1 to basal levels in CA3. (Fig. 2C). In editing analysis at residue 607 of GluA2, genomic glutamine (Q607) had been converted to the arginine (R) codon, with editing rates > 99.9% for both CD-fed mice and HFD-fed mice via CA1, CA2, CA3, DG, and EC (Fig. 2D).

Structural rearrangement of HFD-fed mouse

Post-weaning (6 weeks) food intake and body weight of mice maintained on control diet (CD), high-fat diet (HFD), or HFD containing PER (Fycompa (trademark), Eisai, Japan) (5 mg/kg/day) was monitored up to 18 w (Fig. 2, E and F). Although the caloric content of the diet did not significantly differ between the three groups, significant inhibition of the body weight of HFD by the PER-administered group started from 16 w compared to HFD, and significant body weight gain of HFD started from 8 w compared to CD. To determine the effect of HFD on cognitive behaviors, the open field test (Fig. 9, AC), elevated plus maze test (Fig. 9, DE), the contextual fear conditioning test (Fig. 9, F), the novel object recognition test (Fig. 2G), and a five-trial social interaction test (Fig. 2H) were performed. Among these tests, the latter two tests were found to have significant differences between the CD group and the HFD group, respectively (p < 0.005). Since hippocampal NMDARs have been reported to play an important role in the retrieval of spontaneous object recognition memory (18, 19, 20), behavioral analysis was performed. As a result, in the HFD group, the retrieval ability of the object recognition memory was deteriorated (Fig. 2G). This result was well consistent with the change in hippocampus NMDA receptor function of the HFD mouse. In addition, elevated NR2B RNA levels were seen in CA1 and CA3 of HFD mice (Fig. 2A). This may be a rebound phenomenon due to inhibition of NMDAR by AMPAR. The hippocampus CA2 region has been reported to play an essential role in social memory (21). As described above, it was found that the induction from calcium-impermeable AMPAR to permeable AMPAR by a high-fat diet causes abnormalities in social memory (Fig. 2H), and the NMDA receptor function in the hippocampus CA2 region is inhibited. (Fig. 1). Indeed, PER treatment restored social memory disturbance in HFD-fed mice with the normalized $[Ca^{2+}]i$ signal of CA2. It was found that the decrease in pattern completion ability (ability to retrieve the entire stored memory from partial cues) observed in HFD-fed mice was restored by PER administration (Fig. 2I, J, K). Activation of the NMDA receptor is not necessary for pattern completion during association in memory recall (22), and our data suggested that changing the AMPA receptor to a calcium-permeable type form plays an important role in impairing the pattern completion ability of HFD mice.

Quantitative analysis of the brain by 11.7 T MRI (Bruker BioSpec 117/11, Bruker BioSpin GmbH, Germany) showed hydrocephalus, a sign of brain atrophy caused by a decrease in brain volume, in all mice fed HFD after weaning (n = 6) (Fig. 3A). Furthermore, a significant reduction in left CA1 ($0.14 \pm 0.01$ μL/g) ($0.14 \pm 0.01$ μL/g (n = 8) for CD mice), bilateral CA3 (left, $0.09 \pm 0.01$ μL/g) ($0.11 \pm 0.003$ μL/g (n = 8) for CD mice), and bilateral ECs was observed for HFD mice (n = 8) compared to CD-fed mice (left CA1, 0.17) (Fig. 3B). By volumetric analysis, when HFD-fed mice (n = 8) were treated with PER, brain weight was significantly restored in the regions of the left side CA1 and DG and the right side CA1, CA3 and DG, respectively, of the hippocampus (Fig. 3B). CLARITY (24) was applied to Thy1-eYFP transgenic mice (23), and the number of neurons in the hippocampus subregion was quantified with a Z1 fluorescence microscope (Carl Zeiss). The reduction in the number of Thy-1$^+$-eYFP neuronal cells in CA3 of HFD mice was significantly restored by treatment of PER (p < 0.001) (n = 4) (Fig. 3C, D). By Golgi staining, complete loss of integrity of dendritic arbors in CA1 of HFD mice was restored by PER (Fig. 3E, upper left Panel). Similarly, in the staining with the anti-MAP2 antibody, the integrity of the dendritic arbors disappeared in the CA1 region, and the integrity of the dendritic arbors was completely restored by PER (Fig. 3E, upper left Panel). Immunostaining reaction of GluA1 in CA1 was significantly enhanced in HFD mice, but decreased with PER treatment, and the GluA2/GluA1 ratio increased. In DG, Golgi staining positive cells reduced by HFD were restored by PER (Fig. 3E, top and bottom), while immature spines became mature (Fig. 3E, bottom). In addition, an increase in double cortin positive cells (Fig. 3G), which are markers of neurogenic cells, was observed (Fig. 3E). These data interestingly suggest that immature synapses, as determined by the increase in the stub spine in the DG region under HFD conditions, restored maturation by PER treatment (Fig. 3E).

Effects of AMPA receptor antagonists in Alzheimer's <u>dementia (AD) model mice (NL-GF)</u>

NL-GF mice begin to accumulate in the brain of Aβ from 8 weeks of age (Nat. Neurosci. 2014 17,661-4). In this example, the AD model mouse (n = 5) was fed a CD diet containing a perampanel at a dose of 5 mg/kg body weight/day from 8 to 13 weeks of age, and the mouse was subjected to a novel object recognition test. In the negative control (n = 5), a normal CD diet without perampanel was taken. The results of the novel object recognition test of mice at 13 weeks of age were as shown in Fig. 23. As shown in Fig. 23, the AD model mouse could not distinguish between the mouse in the same room and the mouse for the first time, whereas in the perampanel (PER) administration group, mice significantly distinguish between the mouse in the same room and the mouse for the first time. This result indicates that CA induced dysfunction in NL-GF mice, whereas AMPA receptor antagonists inhibited the development of this dysfunction. Thus, the enhancement of the calcium permeability of the AMPA receptor in Alzheimer's dementia and thereby the inhibition of the NMDA receptor can be treated with an AMPA receptor antagonist, and the treatment restored brain dysfunction in Alzheimer's dementia.

Functional changes of AMPA and NMDA glutamate receptors in ob/ob mice

Leptin (25, 26), a hormone that regulates energy expenditure stimulated by anorexigenic eating behaviors, also plays an important role in synaptic transmission via NMDAR (27). HFD-fed Mice generally first show elevated leptin, one of the hallmarks of obesity, gradually chronic obesity forms leptin resistance and fails to suppress appetite and promote energy expenditure (28). For CP-AMPAR in ob/ob mice, all of Gyki, Naspm, and PER exhibited a significant inhibitory effect in any region of EC, CA1, CA2, CA3, and DG (Fig. 10-1A). In ob/ob mice orally administered PER, leptin significantly enhanced the function of NMDAR in any region of EC, CA1, CA2, CA3, and DG (Panel B of Fig. 10-2). Similarly, in HFD mice orally administered PER, leptin significantly enhanced the function of NMDAR in any region of EC, CA1, CA2, CA3, and DG (Panel C in Fig. 10-2). These results indicate the importance of leptin in hippocampal glutamatergic synaptic transmission. In these experiments, AMPA was used at 100 $\mu$M, CTZ was used at 100 $\mu$M, NMDA was used at 50 $\mu$M, PER was used at 100 $\mu$M, glycine was used at 10 $\mu$M, APV was used at 50 $\mu$M, Naspm was used at 10 $\mu$M, Gyki was used at 100 $\mu$M, and leptin was used at 100 nM. * indicates p < 0.05 in t-test.

A leptin deficient obesity model and the ob/ob mice as a leptin resistant obesity model were analyzed. Specifically, these leptin deficient/resistant models were analyzed for high-resolution MRI, tissue morphology in staining of MAP2, GluA1, GluA2, qRT-PCR, and behavioral analysis. This revealed whether the state of leptin deficiency/resistance status affects hippocampal morphology (Fig. 4). Elevation of $[Ca^{2+}]i$ via CP-AMPAR and the presence of NMDAR inhibition in the region of all (EC, CA1, CA2, CA3, and DG) hippocampal slices of ob/ob mice were revealed (Fig. 4A), similar to HFD-fed mice (Figs. 1A-E). The increase in $[Ca^{2+}]i$ via AMPAR was completely repressed by adding 100 $\mu$M GYKI and 20 $\mu$M NAPSM (Fig. 8). PER administration simultaneously induced significant inactivation of $Ca^{2+}$ permeability via CP-AMPAR and restoration of NMDAR function in all hippocampal subregions examined (Fig. 4B, C) (p < 0.05). These findings were similar to those observed in HFD using PER-treated mice (Figs. 1F to J). The results of qRT-PCR analysis showed a significant increase in GluA1 and GluA2 expression in the CA3 region and GluN2A and GluN2B expression in both CA1 and CA3 regions in ob/ob mice (Fig. 4D), and an increased change in $[Ca^{2+}]i$ in EC and a significant increase in $Ca^{2+}$ permeability index of AMPA type receptor in ob/ob mice compared to CD mice (N = 3) (Fig. 4D).

To apply the volumetric analysis, MRI images were acquired of ob/ob and lean littermate control wild-type (C57BL/6 background) mice, showing that the grey matter mass (0.18 ± 0.02 ml (n = 8)) of ob/ob mice was different compared to wild-type (0.28 ± 0.02 ml (n = 8)) (Fig. 4H) and was significantly reduced. The editing status of the GluA2Q/R site in ob/ob mice was unedited (Q) to 0.6% and edited (R) to 99.4% (N = 5) in the hippocampus CA2 region (Fig. 4I). The results are shown in the case of HFD mice in % of unedited GluA2Q (unedited: to 0.01%); Fig. 2D). It has been reported that about a 7-fold increase in AMPAR $Ca^{2+}$ permeability changes when type (R) GluA2 edited in AMPAR switches to unedited (Q) GluA2 by approximately 25% (29). Therefore, this fact possibly plays an important role in increasing the $Ca^{2+}$ permeability of AMPAR in ob/ob mice. The grey matter mass of wild-type mice was consistent with previous reports (30). In contrast, that of ob/ob mice was significantly smaller than that of wild-type values (Fig. 4J). To further compare the volume of individual brain regions, voxel intensities of grey matter were visualized in the t-value map (Fig. 11A) and an anatomical ROI map was created in the MRI image. In humans, blood leptin levels are directly correlated with brain volumes, including not only areas of feeding behavior but also the hippocampus (31, 32), and the left and right volumes of the hippocampus DG, hypothalamus, sensory cortex, and cerebellum (normalized intensity) of ob/ob mice are significantly smaller than those of wild-type mice (33) (Fig. 11B, C), and leptin deficiency states have a broad effect on changes in brain volume as in HFD-fed mice (Fig. 2G). The results of the five social memory analyses (Fig. 4F) and the behavioral analysis shown in the water maze test (Fig. 4G) showed significant differences compared to the wild type group ($p < 0.005$). These data ($p < 0.005$) in Figs. 4A, B, C, F, and G also suggest that CP-AMPAR and/or NMDAR in the hippocampal CA2 region play an important role in social memory as well as in HFD mice (Figs. 1 and 2G).

ob/ob mice and their littermates with wild-type traits were obtained as the offspring of ob +/- mice. These mice were fed freely and some of the ob/ob mice were fed perampanel-containing diet at 5 mg/kg body weight from 8 to 12 weeks of age. The appearance and body weight of these mice were compared at 12 weeks of age. The results were as shown in Fig. 24. As shown in Fig. 24, compared with ob/ob mice, overweight was prevented in the perampanel-administered mice, exhibiting a lean appearance close to that of the wild type. This result suggests that perampanel administration is effective for the treatment of obesity and overweight. In addition, it was suggested that the cognitive dysfunction might lead to an increase in food amount, and that the AMPA receptor antagonist administration might reduce the cognitive dysfunction, thereby inhibiting the contact amount.

Mice were then split into an HFD-fed group (n = 5) and a PER-containing HFD-fed group (n = 5) under the condition of the same feeding amount (refer to lower Panel of Fig. 25) and their body weights monitored. As shown in the upper Panel of Fig. 25, even under the condition of the same feeding amount, significant body weight loss was observed in the PER-containing HFD-fed group as compared with the HFD-fed group. This suggested that in overweight or obese subjects, the AMPA receptor antagonist produced a weight loss effect even when the amount of meal was not reduced.

Furthermore, our data (Figs. 1 and 4G) suggest that CP-AMPAR, but not the hippocampal NMDAR, plays an important role in pattern separability, as memory recall is not prevented in CA1 of NMDAR knockout mice (34, 35). Similar to the HFD mouse model, ob/ob mice had reduced dendritic arbors integrity as determined by MAP2 staining (Fig. 12). On the other hand, in ob/ob mice using PER, the integrity of the dendritic arbors determined by MAP2 staining (Fig. 12) was significantly more restored by 12 week administration than by 1 week administration. Quantitative analysis of immunoreactive regions was examined with the ZEN Intelesis software (Carl Zeiss, Germany), and PER simultaneously restored reduced GluA1 expression in CA1 and the ratio of GluA2/GluA1 in ob/ob mice (Figs. 4J and 11 to 13). In addition, the maturation of the protrusions of DCX-positive cells progressed by treatment with PER for 12 weeks (Fig. 14). As shown by the mouse obesity model, Leptin-deficient ob/ob mice fed HFD had an effect on the glutamatergic calcium dynamics of the hippocampus inducing changes in morphology, volume, and function. In order to see whether similar findings were observed in humans, first a volumetric analysis was performed in overweight and obese humans compared to normal body weight volunteers, respectively, obtained by T2 data of MRI (Discovery MR 750; General Electric, Milwaukee, WI).

Increasing body mass index adversely affects amount of grey matter in humans

To investigate the correlation of body mass index (BMI) and grey brain mass, analysis was performed by applying volumetric analysis from data obtained by T2 magnetic resonance (MR) imaging of the brain of 122 Japanese subjects (84 people (BMI: less than 25), 27 people (BMI: 25 or more and less than 30), and 11 people (BMI: 30 or more)) (Table 5).

[Table 5]

| Table 5: Number of human subject subject to MRI analysis | | | |
|---|---|---|---|
|  | Adult male | Adult female | Total |
| BMI less than 25 (normal) | 55 | 67 | 122 |
| BMI of 25 or greater and less than 30 (overweight) | 11 | 16 | 27 |
| BMI of 30 or greater | 7 | 4 | 11 |

In men, a significant negative correlation was found between BMI and grey matter mass compared to women. As for healthy subjects having a high (to 30.2) value and a low (to 21.1) value of BMI (Figs. 5A and 5B), there was a tendency that the grey matter mass (gv) decreased with an increase in BMI. In addition, a significant negative correlation was observed between "gv and age" and "gv and body weight" (Fig. 5C, E). In contrast, no significant correlation was found between "BMI and age", and a significant positive correlation was found between "BMI and body weight" (Fig. 5F). Partial correlation analysis was also used to reveal that there was a significant negative correlation between gv and BMI when the effects of age and weight were minimised (Fig. 5G).

Next, the influence of BMI elevation on human hippocampal synaptic transmission was examined by analyzing variations in blood oxygenation level-dependent (BOLD) response, hippocampal memory function of pattern completion, and pattern separability (formation of separate representations of similar inputs) by fMRI behavioral task (35, 36). BMI, pattern completion, and memory recall ability were negatively correlated. The structural network supporting pattern complementarity is based on CA3 and CA1. The central role of ensemble dynamics (dynamic synchronization) was observed in healthy volunteers in this region (37) (Fig. 5H). Importantly, no significant reduction in cognitive function was evident in the overweight group compared to the control group (Fig. 5I). On the other hand, obese people with a BMI of 30 kg/m$^2$ or more showed a significant decrease in pattern completion ability (Fig. 5J). In addition, the change ratio of the BOLD response tended to increase in the overweight group and the obese group as compared with the normal body weight group (Figs. 5H to 5J). In particular, the change ratio of BOLD generally fell within 0.9 to 1.1 in the normal body weight group, whereas the change ratio of BOLD response was less than 0.9 fold or more than 1.1 fold in the overweight group and obese group.

Human's pattern completion ability for memory recall inversely correlates with BMI

In particular, the importance of resting fMRI for biomarkers of human cognition in hippocampal memory function has increased (35). The fMRI data, which measured resting brain activity, suggests that among organized large networks, the default mode network dynamically controls salience and central executive networks of healthy individuals. In obese individuals, this network was disrupted, resulting in impaired cognitive function (Fig. 6A). Finally, a quantitative analysis of human networks based on the graph theory of normal, overweight and obese examinees was applied. A functional connectivity map for each group was created by seed-based analysis, the posterior cingulate cortex and precuneus were set as the default mode network, anterior cingulate, and the cerebellar lobules CrusI of hippocampal memory served as the regions of interest (35). Different network connections between subjects in each of the classified groups were shown in Fig. 6B. Particular alternation between greater networks and between centrality mapping associated with disruption of module autonomy was revealed in overweight and obese groups compared to normal BMI groups, resulting in failure of correct memory recall. Independent network formation of the default mode network (DMN), saliency network (SN), emotional network (EN), and cerebellar network (CBN) was observed in the normal body weight group. In the overweight group, a central executive network (CEN) was added to the network of the normal body weight group, demonstrating new connectivity between DMN and CEN. Intrinsic connectivity within EN and CBN also increased. The intrinsic connectivity of the CEN and visual network (VN) was enhanced in the obese group, and bilateral island cortex appeared in the SN. The centricity between the anterior cingulate cortex and paracingulate gyrus was enhanced over the other two groups. The connectivity between different functional networks (DMN, SN, CEN, CBN, and VN) was extended in obese groups. The total number of nodes in the normal body weight, overweight, and obese groups were estimated to be 17, 21, and 23, respectively (Fig. 6B, C).

Administration of AMPA receptor antagonists to highly obese women

The effect of an AMPA receptor antagonist on the hippocampus and body weight of a 70 year old female with a BMI value of 38 was investigated. A 70 year old female had quadriplegia and respiratory disorders due to a large foramen magnum tumor. After the tumor extraction, anti-epileptic treatment was performed. As an anti-epileptic treatment, 2 mg/day (thereafter, the dose was changed to 4 mg/day) of perampanel was orally administered. The transition of the female body weight and the Fugl-meyer assessment score of the lower limb was observed. In addition, the size of the hippocampus was evaluated from MRI of the brain of the female at 26 weeks after surgery. The results were as shown in Fig. 26. As shown in Fig. 26, the size of the hippocampus (refer to the frame in the upper photograph of the drawing) was clearly increased by PER administration. The numerical values were as shown in Table 6 below.

[Table 6]

Table 6: Effect of AMPA receptor antagonist on hippocampus in obese women

|  | gray matter/TIV | CA1/TIV | | CA3/TIV | | DG&CA4/TIV | | BMI |
|---|---|---|---|---|---|---|---|---|
|  |  | left | right | left | right | left | right |  |
| Before surgery | 0.52 | 2.76 | 2.29 | 0.27 | 0.27 | 0.79 | 1.00 | 37.7 |
| 26w after surgery | 0.50 | 2.97 | 2.22 | 0.30 | 0.30 | 0.86 | 1.01 | 33.4 |

TIV: total intracranial volume Units are in L/L for grey matter and mm$^3$ for hippocampal region.

From the above results, AMPA receptor antagonists increased hippocampal volume in women. Furthermore, as shown in Fig. 27, after administration of the AMPA receptor antagonist, the body weight of the woman decreased smoothly. This result is similar to the results shown in Figs. 24 and 25.

Furthermore, it was investigated whether inactivation of the NMDA receptor is a calcium-dependent inhibition. Calcium ion dependent NMDA receptor expression and reactivity to NMDA and glycine in mice (2 w HFD) fed with HFD for 2 weeks were confirmed. The results were as shown in Panel A of Fig. 28. Brain slices from the 2 w HFD group were treated for 60 min in the presence of 3 $\mu$M BAPTA-AM or under calcium ion-free conditions, after which the treated brain slices were subjected to calcium ion imaging. In the 2 w HFD group (n = 5), the addition of NMDA and glycine did not activate the NMDA receptor in any brain region, and the NMDA receptor was inactivated. In the BAPTA-AM-treated 2 w HFD group (2w HFD + BAPTA, n = 3), BAPTA-AM rapidly chelated calcium ions in the aqueous solution, which released the inactivation of the NMDA receptor, as indicated by the restoration of reactivity (increased calcium ion concentration) to the addition of NMDA (50 $\mu$M) and glycine (10 $\mu$M) of the NMDA receptor. This reactivity abolished when D-(-)-2-amino-5 phosphonovaleric acid (APV) (50 $\mu$M), an antagonist of the NMDA receptor, was administered simultaneously with NMDA and glycine, revealing that the increase in calcium ion concentration to the addition of NMDA and glycine is realized by the NMDA receptor. In Fig. 28, Panel B shows a comparison of maximum value of normalized fluorescence intensity (calcium ion concentration). As shown in Fig. 28, Panel B, compared to the HFD-fed group (n = 4), the BAPTA-AM treated group (n = 3) and the calcium-free treated group (n = 3) restored NMDA receptor reactivity to NMDA. A similar phenomenon was observed in brain slices treated under calcium ion-free conditions. That is, under calcium ion-free (0 mM Ca) conditions, the NMDA receptor showed reactivity to NMDA and glycine (n = 3). In addition, in the presence of APV as an NMDA receptor antagonist, the reactivity disappeared. In any of the CA1, CA2, CA3, DG, and EC regions of the hippocampus of 17 week old mice, the 2 w HFD group restored NMDA receptor responsiveness to NMDA and glycine by BAPTA-AM treatment and calcium ion-free treatment (Panel B of Fig. 28). In 14 to 16 week old mice on normal diet, NMDA receptors responded to NMDA and glycine in the presence of 2 mM calcium ions (n = 5), whereas in brain slices pretreated with 1 $\mu$M ionomycin for 10 min (n = 4), NMDA receptor reactivity due to addition of NMDA and glycine disappeared (Panels C and D of Fig. 28).

From these results, it was expected that the $Ca^{2+}$-dependent inactivation (CDI) [15, 40, 41] observed in obese mice was probably regulated by AMPAR's $Ca^{2+}$ permeability and endogenous $Ca^{2+}$ buffering capacity. Indeed, NMDAR inactivation via AMPAR disappeared in the presence of intracellular fast $Ca^{2+}$ buffer BAPTA or in $Ca^{2+}$ free extracellular solution (Panels A and B of Fig. 28). On the other hand, bathing with ionomycin (1 $\mu$M) [42] with 2 mM $Ca^{2+}$ induced CDI of the NMDAR (Fig. 28, Panels C and D). From these results, it was suggested that the functional decrease of the NMDA receptor in the hippocampus by HFD was mediated by the increase in $Ca^{2+}$ permeability of the AMPA receptor.

Central role of CP-AMPAR in obesity-related cognitive decline

HFD stimulated calcium dynamics in the hippocampus via AMPAR, and subunit structures rearranged from non-CP-AMPAR to CP-AMPAR, altering NMDAR calcium signal transduction to downregulate $Ca^{2+}$ influx. Inactivation of transmission by function of the NMDAR is thought to correspond to a decline in cognitive function. CP-AMPAR led to decreased hippocampal size and cell number (especially CA3), decreased dendritic integrity of CA1, and maturation of the spine in the DG region. It was found that ingestion of HFD for 7 days or more affects the memory circuit of the hippocampus via a calcium signal. While calcium dynamics via NMDAR was maintained with this short-term ingestion, calcium entry via AMPAR was evident in the hippocampus. Long-term ingestion for 8 weeks or more after weaning completely reduced calcium entry via NMDAR with changes in calcium kinetics via AMPAR. AMPAR and NMDAR are involved in the high-speed transmission of glutamate-based synapses and are localized in the postsynaptic membrane, and play an important role in human cognition such as learning and memory. Inhibition of calcium influx into a synapse by an NMDAR is regulated by the intracellular calcium concentration, regardless of the source of calcium, such as AMPAR, voltage-dependent calcium channels, internal storage, NMDAR (9). Notably, AMPAR is co-active with NMDAR and is responsible for excitatory synaptic responses on the 1/10 millisecond timescale, while HFD increased calcium entry of these receptors and ultimately decreased the magnitude of calcium current through NMDAR. NMDARs have been reported to play an important role in controlling appetite and dietary preferences, in addition to inducing synaptic plasticity (LTP/LTD) (36), which is believed to cause a vicious cycle of binge drinking, reaction due to dietary restriction failure, and increased obesity (37), ultimately further accelerating decline in cognitive function. Attempts to activate the NMDAR to restore neural function by AMPAR-mediated hyperthermia have also been shown to conversely promote neuronal injury (72).

Modification of glutamatergic synaptic transmission affects functional network connectivity of resting fMRI data

Higher BMI is associated with cognitive dysfunction in young people (38), and in middle age, overweight and obesity may increase the future risk of cognitive decline in old age. In the elderly, there is a paradoxical phenomenon that higher BMI results in better cognitive function and lower mortality (39). HFD induces obesity and causes hypertension, type 2 diabetes, and cardiovascular events, and patients with these diseases are at increased risk of cognitive decline (40, 41). Presumably, the inability to properly manage lifestyle, including self-regulation of dietary behavior, prevents effective treatment and disease control. In medical care, developing an effective intervention method against the decline in cognitive function associated with obesity is a key to better disease management. In the brains of an overweight person and an obese person, the correlation between the decrease in resting activity of the precuneus and posterior cingulate cortex and the decrease in activity of the dorsal lateral prefrontal area and the insular cortex occurred at the same time was broken (Fig. 6). Similar perturbations were also observed in subjects with metabolic syndrome (42). Furthermore, in the connectivity analysis of the resting fMRI data to which the graph theory is applied, the structural and functional connectivity is disturbed from the overweight brain to the obese brain, the number of networks with nodes and edges increases, and they integrally form a hierarchical network in which the right lower frontal lobe is located, whereas simple module formation without centricity was observed in the normal body weight group. A small number of distributed parallel module networks that work autonomously without top-down interference leads to high efficiency.

AMPAR is an attractive target for cognitive decline

Notably, the appearance of frequent hydrocephalus was seen among the early onset obese mouse group. In humans, obesity began in early childhood from 2 to 6 years of age (46), and even most children who were obese at that age were obese in adolescence. Appropriate and effective interventions are an urgent challenge for human health and illness. Importantly, application of HFD with PER treatment restored dysregulated calcium signal transduction via both AMPAR and NMDAR, while eliciting restoration of brain size and behavioral memory capacity. Preventing weight gain due to obesity provides health benefits. Food restriction (47), which has been reported to induce CP-AMPAR in the nucleus accumbens, usually rebounds and ends up with weight gain and binge eating. Taken together, AMPAR is suggested to be an attractive therapeutic target for cognitive decline associated with human obesity.

Action of AMPA receptor antagonists inhibits β-amyloid deposition

In the examples, using the perampanel as an AMPA receptor antagonist, it was demonstrated that the perampanel significantly increased the Aβ42/Aβ40 ratio of the culture supernatant in the neural stem cell culture system derived from the Alzheimer's type model mouse, decreased the deposition of Aβ, and inhibited the accumulation of Aβ in the hippocampus and cerebrum of the model mouse.

Biomarkers of Alzheimer's dementia include the Aβ42/Aβ40 ratio of cerebrospinal fluid (CSF) and plasma levels, phosphorylated tau (P-tau) in threonines 181 and 217, and neurofilament lite (NfL) (Curr Opin Neurol 2021 1 266-274). The Aβ42/Aβ40 ratio is considered to be able to predict Aβ accumulation in PET (Doecke JD et al., Neurology 2020). Hippocampus on 17 to 19 days of NL-G-F(-/-) mouse embryonic life was taken out and cultured by a Neurosphere method by Brewer GJ et al. (Nat Protoc 2007 2, 1490-1498), and a supernatant was measured by an EIA method using this culture system. The change in the Aβ42/Aβ40 ratio by the perampanel (50 μM/methyl cellulose 4 μL) was analyzed. In 4 μL of a 0.5 w/v% aqueous solution of methyl cellulose 400, $5 \times 10^4$ spheres smaller than 50 um in diameter were cultured in 50 ml Falcon Flask coated with poly-D-lysine/laminin, and Aβ42 and Aβ40 of the supernatant were measured by EIA method on the second day (Fig. 29A) and the fourth day (Fig. 29B) of culture. The solvent was administered 12 hours after seeding. A significant change in the Aβ42/Aβ40 ratio by the perampanel was observed in the NL-G-F(-/-) mouse. When the culture was subjected to formic acid treatment on the fourth day of culture to perform Aβ staining (Anti-Human Amyloid β (N) (82 E) Mouse IgG MoAb, 1: 100) (IBL, Japan) and background staining with DAPI, as shown in Fig. 30, a decrease in Aβ was observed in the perampanel group (PER).

The effect of perampanel administration on Aβ accumulation in the hippocampal dentate gyrus (DG) CA1 and the cerebral cortex (Cx) in the Alzheimer's dementia model mouse (NL-G-F(-/-)) is shown. The X axis indicates the intensity of fluorescence, and the Y axis indicates the fluorescence count. A shows an untreated mouse (control group), and B shows a mouse administered with perampanel (5 mg/kg) from 6 to 15 weeks of age (PER administration group). As shown in Fig. 31, a decrease in Aβ accumulation was observed in the mice to which the perampanel was administered.

Epileptic seizures develop early in Alzheimer's disease (AD)

Epilepsy occurs more frequently in AD patients than in the control population (Pascal E 2012), and the incidence of epilepsy increases to 7 to 21% (Amatniek et al., 2006; Hauser et al., 1986; Mendez and Lim, 2003) in sporadic AD patients and 30% in early-onset familial AD (FAD) (Palop and Mucke, 2009; Larner and Doran, 2006). Early latent hippocampal hyperexcitability was detected in AD patients (Lam AD et al., Nat Med 2017). As shown in Fig. 32, in the transgenic animals that promote Aβ aggregation, both NL(-/-) mice having one mutation and NL-G-F(-/-) mice having three mutations were confirmed to have enhanced epileptiform reactions as compared with wild-type (WT) mice. In particular, in NL-G-F(-/-) mice (15 to 17 weeks old), significant enhancement of epileptiform reactions in the CA1 and CA2 regions was confirmed by Bicculine (BIC) (refer to Fig. 32). These results are consistent with the clinical background behind hippocampal overactivity. In an animal model of AD in which amyloid β is accumulated, it has been reported that an epileptiform reaction is enhanced (Busche, 2008; 2012). Also in this experiment, epileptic induction by bicrine was enhanced in both NL(-/-) mice and NL-G-F(-/-) (15 to 17 weeks old (W)) as compared with control mice. In addition, since accumulation of amyloid β could not be detected in the brain of a 17 W NL(-/-) mouse, it was suggested that accumulation of amyloid β is not directly related to excitability itself of epilepsy.

The perampanel, which is a non-competitive AMPA receptor antagonist, inhibits the activation of the AMPA receptor of the postsynaptic membrane by glutamic acid, and is indicated for partial seizures (including secondary generalized seizures) like epileptic patients of 12 years old or older, tonic-clonic seizures, and partial seizures (including secondary generalized seizures) like epileptic patients of 4 years old or older, but it seems to be also indicated for epileptic seizures of the elderly and epileptic seizures accompanied by dementia in the future.

Administration of the perampanel to NL(-/-) mice almost completely abolished the epileptiform reactions induced by bicculine (refer to Fig. 33A).

AMPA receptor antagonists improve cognitive function in dementia subjects together with memantine

NL-G-F(-/-) mice, a dementia model, were administered with memantine (mema), exercise, memantine and exercise, memantine and perampanel, and perampanel alone (5 mg/kg or 10 mg/kg), and subjected to behavioral experiments. As the behavior test, a novel object recognition test and a hippocampus function-dependent fear conditioning test were performed. Memantine is an antagonist of the NMDA receptor, but has distinct properties from other NMDA receptor antagonists (refer to Hokama Y., et al., Neuro Oncology, 2022, noac162). That is, memantine is a drug that works only when glutamic acid is excessively present in the brain. Memantine also has a superior effect on the site of synaptic NMDAR relative to the non-synaptic NMDA receptor (extrasynaptic NMDAR).

As a result, as shown in Fig. 34, in the novel object recognition test, significant improvement in cognitive function was observed in the group in which memantine and perampanel were used in combination. In addition, as shown in Fig. 35, in the hippocampus function-dependent fear conditioning test, improvement in cognitive function was observed by administration of memantine alone and exercise, but remarkable improvement in cognitive function was observed by administration of perampanel alone or combination use of memantine and perampanel. It is considered that memantine opens the gate of proliferation of neural progenitor cells (DCX-positive neurons) by inhibiting the NMDAR of the interneurons that inhibit neurogenesis, restarts neurogenesis, and improves hippocampal function, but improvement of cognitive function was remarkable when used in combination with the perampanel.

The documents cited in this specification are incorporated herein by reference in their entirety.

Related documents

1. E. E. Noble, S. L. Kanoski, Curr. Opin. Behav. Sci. 9, 7 14 (2016).

2. S. L. Hargrave, S. Jones, T. L. Davidson, Curr. Opin. Behav. Sci. 9, 40-46 (2016).

3. L-L. Hwang, C-H. Wang, T-L. Li, S-D. Chang, L-C. Lin, C-P. Chen, C-T. Chen, K-C. Liang, I-K. Ho, W-S. Yang, L-C. Chiou, Obesity (Silver Spring) 18, 463-469 (2010).

4. I. Valladolid-Acebes, P. Stucchi, V. Cano, M. S. Femández-Alfonso, B. Merino, M. Gil-Ortega, A. Foie, L. Morales, M. Ruiz-Gayo, N. D. Olmo, Neurobiol. Learn. Mem. 95, 80-85 (2011).

5. M. Fotuhi, D. Do, C. Jack, Nat. Rev. Neurol. 8, 189-202 (2012).

6. N. D. Olmo, M. Ruiz-Gayo, Front. Cell. Neurosci. 12, 439 (2018).

7. M. Anai, M. Funaki, T. Ogihara, A. Kanda, Y. Onishi, H. Sakoda, K. Inukai, M. Nawano, Y. Fukushima, Y. Yazaki, M. Kikuchi, Y. Oka, T. Asano, Diabetes 48,158-169 (1999).

8. S. Hibi, K. Ueno, S. Nagato, K. Kawano, K. Ito, Y. Norimine, O. Takenaka, T. Hanada, M. Yonaga, J. Med. Chem. 55, 10584-10600 (2012).

9. A. Rozov, N. Bumashev, Cell Calcium 60, 407-414 (2016).

10. S. Ishiuchi, K. Tsuzuki, Y. Yoshida, N. Yamada, N. Hagimura, H. Okado, A. Miwa, H. Kurihara, Y. Nakazato, M. Tamura, T. Sasaki, S. Ozawa, Nat. Med. 8, 971-978 (2002).

11. M.Akamatsu, T. Yamashita, N. Hirose, S. Teramoto, S. Kwak, Sci. Rep. 6, 28649 (2016).

12. K. L. Conrad, K. Y. Tseng, J. L. Uejima, J. M. Reimers, L-J. Heng, Y. Shaham, M. Marinelli, M. E. Wolf, Nature 454, 118-121 (2008).

13. R. K. Khangura, A. Bali, A. S. Jaggi, Eur. J. Pharmacol. 795, 36-42 (2017).

14. J. A. French, C. L. Krauss, R. T. Wechsler, X-F. Wang, B. DiVentura, C. Brandt, E. Trinka, T. J. O Brien, A.

Laurenza, A. Patten, P. Bibbiani, Neurology 85, 950-957 (2015).

15. S. Cull-Candy, L. Kelly, M. Farrant, Curr. Opin. Neurobiol. 16, 288-297 (2006).

16. S. J. Liu, R. S. Zukin, Trends Neurosci. 30, 126-134 (2007).

17. S. Ozawa, J. Physiol. 587, 1861-1862 (2009).

18. E. Iwamura, K. Yamada, Y. Ichitani, Behav. Brain Res. 307, 92-99 (2016).

19. F. L. Hitti, S.A. Siegelbaum, Nature 508, 88-92 (2014).

20. B. Mei, F. Li, Y. Gu, Z. Cui, J. Z. Tsien, PLoS One 6, e19326 (2011).

21. C. Porrero, P. Rubio-Garrido, C. Avendaño, F. Clascá, Brain Res. 1345, 59-72 (2010).

22. K. Chung, J. Wallace, S-Y. Kim, S. Katyanasundamm, A. S. Andalman, T. J. Davidson, J. J. Mirzabekov, K. A. Zalocusky, J. Mattis, A. K. Denεsin, S. Pak, H. Bernstein, C. Ramakrishnan, L. Grosenick, V. Gradinatu, K. Deisseroth, Nature 497, 332-337 (2013).

23. Y. Zhang, R. Proenca, M. Maffei, M. Barone, L. Leopold, J. M. Friedman, Nature 372, 425-432 (1994).

24. C. T. Montague, I. S. Farooqi, J. P. Whitehead, M.A. Soos, H. Rau, N. J. Wareham, C. P. Sewter, J. E. Digby, S. N. Mohammed, J. A. Hurst, C. H. Cheetham, A. R. Earley, A. H. Barnett, J. B. Prins, S. O 1 Rahilly, Nature 387, 903-908 (1997).

25. L. J. Shanley, A. J. Irving, J. Harvey, J. Neurosci. 21, RC186 (2001).

26. R. Mazor, D. Friedmann-Morvinski, T. Alsaigh, O. Kleifeld, E. B. Kistler, L. Rousso-Noori, C. Huang, J. B. Li, I. M. Verma, G. W. Schmid-Schönbein, Sci. Transl. Med. 10, 6324 (2018).

27. R. Brusa, F. Zimmermann, D.-S. Koh, D. Feldmeyer, P. Gass, P. N. Seeburg, R. Sprengel, Science 270, 1677-1680 (1995).

28. S. J. Sawiak, N.I. Wood, G. E3. Williams, A. J. Morton, T. A. Carpenter, Neurobiol. Dis. 33, 20-27 (2009).

29. G.J. Paz-Filho, NeuralPlast. 2016, article ID 8528934 (2016).

30. A. V. Witte, T. Köbe, A. Graunke, J. P. Schuchardt, A. Hahn, V. A. Tesky, J. Pantel, A. Flöel, Hum. Brain Mapp. 37, 4539-4549 (2016).

31. A. E. Dorr, J. P. Lerch, S. Spring, N. Kabani, R. M. Henkelman, Neuroimage 42, 60-69 (2008).

32. A. Shiroma, M. Nishimura, II. Nagamine, T. Miyagi, Y. Hokama, T. Watanabe, S. Murayama, M. Tsutsui, D. Tominaga, S. Ishiuchi, Cerebellum 15, 645-662 (2016).

33. R. Usugi, M. Nishimura, S. Ishiuchi, Brain Behav. 10, e01878 (2020).

34. J.F. Guzowski, J. J. Knierim, E. I. Moser, Neuron 44, 581-584 (2004).

35. T. Sasaki, S. Matsui, T. Kitamura, Int. J. Mol. Sci. 17, 1081 (2016).

36. C. Lüscher, R. C. Malenka, Cold Spring Harb. Perspect. Biol. 4, a005710 (2012).

37. S. E. Kanoski, T. L. Davidson, Physiol. Behav. 103, 59-68 (2011).

38. L. B. Massing, A. K. Dahl, N. L. Pedersen, B. Johansson, Dement. Geriatr. Cogn. Disord. 29, 543-552 (2010).

39. W. Jagust, D. Harvey, D. Mungas, M. Haan, Arch. Neurol. 62, 1545-1548 (2005).

40. K. F. Yates, V. Sweat, P. L. Yau, M. M. Turchiano, A. Convit, Arterioscler. Thromb. Vase. Biol. 32, 2060-2067 (2012).

41. P. G. Kopelman, Nature 404, 635-643 (2000).

42. B. Rashid, S. I. Dev, M. Nicolette, F. Schwarz, T. Ferland, F. C. Fortenbaugh, W. P. Milberg, R. E. McGlinchey, D. H. Salat, F. C. Leritz, Brain Behav. 9, e01333 (2019).

43. M. Geserick, M. Vogel, R. Gausche, T. Lipek, U. Spielau, E. Keller, R. Pfäffle, W. Kiess, A. Körner, J. Med. 379, 1303-1312 (2018).

44. D. C. Ou-Yang, P. J. York, C. J. Kleck, V. V. Patel, J. Bone Joint Surg. Am. 99, 2027-2036 (2017).

45. T. Yonekawa, M. C. V. Malicdan, A. Cho, Y. K. Hayashi, I. Nonaka, T. Mine, T. Yamamoto, I. Nishino, S. Noguchi. Brain 137, 2670-2679 (2014).

46. H. Ageta, A. Murayama, R. Migishima, S. Kida, K. Tsuchida, M. Yokoyama, K. Inokuchi, PLoS one 3, 1869 (2008).

47. T-N. Huang, Y. P. Hsueh, Bioprotocol 4, Iss19 (2014).

48. T. Toda, Y. Noda, G. Ito, M. Maeda, T. Shimizu, J. Biomed. Biotechnol. 617974 (2011).

49. T. Nakashiba, J. D. Cushman, K.A. Polkey, C. J. McBain, M. S. Fanselow, S. Tonegawa, Cell 149, 188-201 (2012),

50. W. B. Chwang, K. J. O ' Riordan, J. M. Levenson, J. D. Sweatt, Learn. Mem. 13, 322-328 (2006).

51. J. P. P. Ullmann, C. Watson, A. L. Janke, N. D. Kurniawan, D. C. Reutens, Neuroimage 78, 196-203 (2013).

52. T.L. Richards, T. J. Grabowski, P. Boord, K. Yagle, M. Askren, Z. Mestre, P. Robinson, O. Welker, D. Gulliford, W. Nagy, V. Berninger, Neuroimage Clin. 8, 408-421 (2015).

53. S. Whitfield-Gabrieli, A. Nieto-Castanon. Brain Connect. 2, 125-141 (2012).

54. M. Rubinov, O. Sporns, Neuroimage 52, 1059-1069 (2010).

55. M. Xia, J. Wang, Y. He, PLoS One 8, e68910 (2013).

56. I. Yao, H. Takagi, H. Ageta, T. Kal)yo, S. Sato, K. Hatanaka, Y. Fukuda, T. Chiba, N. Morone, S. Yuasa, K. Inokuchi, T. Ohtsuka, G. R. Macgregor, K. Tanaka, M. Setou, Cell 130, 943-957 (2007).

57. H. Takagi, M. Setou, S. Ito, 1. Yao. Neural. Plast. 352829 (2012).

58. L. Nowak, P. Bregestovski, P. Ascher, A. Herbet, A. Prochiantz. Nature, 307:462-465 (1984)

59. M. Koike, M. Iino, S. Ozawa, Neurosci. Res. 29, 27-36 (1997).

60. A.C. Boudreau, J. M. Reimers, M. Milovanovic, M. E. Wolf, J. Neurosci. 27, 10621-10635 (2007).

61. O.H. Lowry, N. J. Rosebrough, A. L. Farr, R. J. Randall, J. Biol. Chem. 193, 265-275 (1951).

62. J. B. Treweek, K. Y. Chan, N. C. Flytzanis, B. Yang, B. E. Deverman, A. Greenbaum, A. Lignell, C. Xiao, L. Cai, M. S. Ladinsky, P. J. Bjorkman, C. C. Fowlkes, V. Gradinaru, Nat. Protoc. 10:1860-1896 (2015)

63. G. Fong, R. H. Mellor, M. Bernstein, C. Keller-Peck, Q. T. Nguyen, M. Wallace, J. M. Nerbonne, J. W. Lichtman, J. R. Sanes, Neuron 28, 41-51 (2000).

64. C. Porrero, P. Rubio-Garrido, C. Avendaño, F. Clascá, Brain Res. 1345, 59-72 (2010).

65. D. Kim, J. M. Paggi, C. Park, C. R. Bennett, S. L. Salzberg, Nat Biotechnol. 37; 907-915 (2019).

66. R. Patro, G. Duggal, M. I. Love, R. A. Irizarry, C. Kingsford, Nat Methods 14, 417-419 (2017).

67. M. Pertea, G. M. Pertea, C. M. Antonescu, T. C. Chang, J. T. Mendell, S. L. Salzberg, Nat Biotechnol. 33, 290-295 (2015).

68. M. A. DePristo, E. Banks, R, Poplin, K. V. Garimella, J. R. Maguire, C. Hart1, A. A. Philippakis, G. del Angel, M. A. Rivas, M. Hanna, A. McKenna, T. J. Fennell, A. M. Kernytsky, A. Y. Sivachenko, K. Cibulskis, S. B. Gabriel, D. Altshuler, M. J. Daly, Nat. Genet. 43, 491-498 (2011).

69. G.A. Van der Auwera, M. O. Carneiro, C. Hartl, R. Poplin, G. Del Angel., A. Levy-Moonshine, T. Jordan., K. Shakir, D. Roazen, J. Thibault, E. Banks, K. V. Garimella, D. Altshuler, S. Gabriel, M. A. DePristo, Curr. Protoc. Bioinformatics 43 (2013).

70. A. McKenna, M. Hanna, E. Banks, A. Sivachenko, K. Cibulskis, A. Kernytsky, K. Garimella, D. Altshuler, S. Gabriel, M. Daly, M. A. DePristo, Genome Res. 20, 1297-1303 (2010).

71. P. Cingolani, A. Platts, L. L. Wang, M. Coon, T. Nguyen, L. Wang, S. J. Land, X. Lu, D. M. Ruden, Fly 6, 80-92 (2012).

72. K. A. Yamada, Neurobiology of Disease, 5, 67-80 (1998)

Extraction of Sequence Listings

>SEQ ID NO: 1

atgcaaaagattatgcatatttctgtcctcctttctcctgttttatggggactgatttttggtgtctcttctaacagcatacagatagggggg
gctatttcctaggggcgccgatcaagaatacagtgcatttcgagtagggatggttcagttttccacttcggagttcagactgacacccc
acatcgacaatttggaggtggcaaacagcttcgcagtcactaatgctttctgctcccagttttcgagaggagtctatgctattttggatt
ttatgacaagaagtctgtaaataccatcacatcattttgcggaacactccacgtctccttcatcactcccagcttcccaacagatggcac
acatccatttgtcattcagatgagacccgacctcaaaggagctctccttagcttgattgaatactatcaatgggacaagtttgcatacctc
tatgacagtgacagaggcttatcaacactgcaagctgtgctggattctgctgctgaaaagaaatggcaagtgactgctatcaatgtgg
gaaacattaacaatgacaagaaagatgagatgtaccgatcacttttttcaagatctggagttaaaaaaggaacggcgtgtaattctgga
ctgtgaaagggataaagtaaacgacattgtagaccaggttattaccattggaaaacatgttaaagggtaccactacatcattgcaaatc
tgggatttactgatggagacctattaaaaatccagtttggaggtgcaaatgtctctggatttcagatagtggactatgatgattcgttggt
atctaaatttatagaaagatggtcaacactggaagaaaaagaataccctggagctcacacaacaacaattaagtatacttctgctctga
cctatgatgccgttcaagtgatgactgaagccttccgcaacctaaggaagcaaagaattgaaatctcccgaaggggggaatgcaggag
actgtctggcaaacccagcagtgccctggggacaaggtgtagaaatagaaagggccctcaaacaggttcaggttgaaggtctctcag
gaaatataaagtttgaccagaatggaaaaagaataaactatacaattaacatcatggagctcaaaactaatgggccccggaagattgg
ctactggagtgaagtggacaaaatggttgttacccttactgagctcccttctggaaatgacacctctgggcttgagaataagactgttgt
tgtcaccacaattttggaatctccgtatgttatgatgaagaaaaatcatgaaatgcttgaaggcaatgagcgctatgagggctactgtgt
tgacctggctgcagaaatcgccaaacattgtgggttcaagtacaagttgacaattgttggtgatggcaagtatggggccagggatgca
gacacgaaaatttggaatgggatggttggagaacttgtatatgggaaagctgatattgcaattgctccattaactattacccttgtgaga
gaagaggtgattgacttctcaaagcccttcatgagcctcgggatatctatcatgatcaagaagcctcagaagtccaaaccaggagtgtt
ttcctttcttgatcctttagcctatgagatctggatgtgcattgttttttgcctacattggggtcagtgtagtttattcctggtcagcagattt
agcccctacgagtggcacactgaggagtttgaagatggaagagaaacacaaagtagtgaatcaactaatgaatttgggatttttaata
gtctctggttttccttgggtgcctttatgcagcaaggatgcgatatttcgccaagatccctctctgggcgcattgttggaggtgtgtggtg
gttctttaccctgatcataatctcctcctacacggctaacttagctgccttcctgactgtagagaggatggtgtctcccatcgaaagtgct
gaggatctttctaagcaaacagaaattgcttatggaacattagactctggctccactaaagagtttttcaggagatctaaaattgcagtg
tttgataaaatgtggacctacatgcggagtgcggagccctctgtgtttgtgaggactacggccgaaggggtggctagagtgcggaag
tccaaagggaaatatgcctacttgttggagtccacgatgaacgagtacattgagcaaaggaagccttgcgacaccatgaaagttggtg
gaaacctggattccaaaggctatggcatcgcaacacctaaaggatcctcattaagaaccccagtaaatcttgcagtattgaaactcagt
gagcaaggcgtcttagacaagctgaaaaacaaatggtggtacgataaaggtgaatgtggagccaaggactctggaagtaaggaaaa
gaccagtgccctcagtctgagcaacgttgctggagtattctacatccttgtcggggggccttggtttggcaatgctggtggctttgattga
gttctgttacaagtcaaggggccgaggcgaaacgaatgaaggtggcaaagaatgcacagaatattaacccatcttcctcgcagaattca
cagaattttgcaacttataaggaaggttacaacgtatatggcatcgaaagtgttaaaatttag

>SEQ ID NO: 2

MQKIMHISVLLSPVLWGLIFGVSSNSIQIGGLFPRGADQEYSAFRVGMVQFSTSEFRLT
PHIDNLEVANSFAVTNAFCSQFSRGVYAIFGFYDKKSVNTITSFCGTLHVSFITPSFPTD

GTHPFVIQMRPDLKGALLSLIEYYQWDKFAYLYDSDRGLSTLQAVLDSAAEKKWQVT
AINVGNINNDKKDEMYRSLFQDLELKKERRVILDCERDKVNDIVDQVITIGKHVKGY
HYIIANLGFTDGDLLKIQFGGANVSGFQIVDYDDSLVSKFIERWSTLEEKEYPGAHTT
TIKYTSALTYDAVQVMTEAFRNLRKQRIEISRRGNAGDCLANPAVPWGQGVEIERALK
QVQVEGLSGNIKFDQNGKRINYTINIMELKTNGPRKIGYWSEVDKMVVTLTELPSGN
DTSGLENKTVVVTTILESPYVMMKKNHEMLEGNERYEGYCVDLAAEIAKHCGFKYKL
TIVGDGKYGARDADTKIWNGMVGELVYGKADIAIAPLTITLVREEVIDFSKPFMSLGIS
IMIKKPQKSKPGVFSFLDPLAYEIWMCIVFAYIGVSVVLFLVSRFSPYEWHTEEFEDGRE
TQSSESTNEFGIFNSLWFSLGAFMQQGCDISPRSLSGRIVGGVWWFFTLIIISSYTANLA
AFLTVERMVSPIESAEDLSKQTEIAYGTLDSGSTKEFFRRSKIAVFDKMWTYMRSAEPS
VFVRTTAEGVARVRKSKGKYAYLLESTMNEYIEQRKPCDTMKVGGNLDSKGYGIATP
KGSSLRTPVNLAVLKLSEQGVLDKLKNKWWYDKGECGAKDSGSKEKTSALSLSNVAG
VFYILVGGLGLAMLVALIEFCYKSRAEAKRMKVAKNAQNINPSSSQNSQNFATYKEGY
NVYGIESVKI

>SEQ ID NO: 3

MQKIMHISVLLSPVLWGLIFGVSSNSIQIGGLFPRGADQEYSAFRVGMVQFSTSEFRLT
PHIDNLEVANSFAVTNAFCSQFSRGVYAIFGFYDKKSVNTITSFCGTLHVSFITPSFPTD
GTHPFVIQMRPDLKGALLSLIEYYQWDKFAYLYDSDRGLSTLQAVLDSAAEKKWQVT
AINVGNINNDKKDEMYRSLFQDLELKKERRVILDCERDKVNDIVDQVITIGKHVKGY
HYIIANLGFTDGDLLKIQFGGANVSGFQIVDYDDSLVSKFIERWSTLEEKEYPGAHTT
TIKYTSALTYDAVQVMTEAFRNLRKQRIEISRRGNAGDCLANPAVPWGQGVEIERALK
QVQVEGLSGNIKFDQNGKRINYTINIMELKTNGPRKIGYWSEVDKMVVTLTELPSGN
DTSGLENKTVVVTTILESPYVMMKKNHEMLEGNERYEGYCVDLAAEIAKHCGFKYKL
TIVGDGKYGARDADTKIWNGMVGELVYGKADIAIAPLTITLVREEVIDFSKPFMSLGIS
IMIKKPQKSKPGVFSFLDPLAYEIWMCIVFAYIGVSVVLFLVSRFSPYEWHTEEFEDGRE
TQSSESTNEFGIFNSLWFSLGAFMRQGCDISPRSLSGRIVGGVWWFFTLIIISSYTANLA
AFLTVERMVSPIESAEDLSKQTEIAYGTLDSGSTKEFFRRSKIAVFDKMWTYMRSAEPS
VFVRTTAEGVARVRKSKGKYAYLLESTMNEYIEQRKPCDTMKVGGNLDSKGYGIATP
KGSSLRTPVNLAVLKLSEQGVLDKLKNKWWYDKGECGAKDSGSKEKTSALSLSNVAG
VFYILVGGLGLAMLVALIEFCYKSRAEAKRMKVAKNAQNINPSSSQNSQNFATYKEGY
NVYGIESVKI

>SEQ ID NO: 4

atgcagcacattttttgccttcttctgcaccggtttcctaggcgcggtagtaggtgccaatttccccaacaatatccagatcggggggatta
tttccaaaccagcagtcacaggaacatgctgcttttagatttgctttgtcgcaactcacagagcccccgaagctgctcccccagattgat

attgtgaacatcagcgacagctttgagatgacctatagattctgttcccagttctccaaaggagtctatgccatctttgggtttttatgaac

gtaggactgtcaacatgctgacctccttttgtgggggccctccacgtctgcttcattacgccgagctttcccgttgatacatccaatcagtt

tgtccttcagctgcgccctgaactgcaggatgccctcatcagcatcattgaccattacaagtggcagaaatttgtctacatttatgatgc

cgaccggggcttatccgtcctgcagaaagtcctggatacagctgctgagaagaactggcaggtgacagcagtcaacattttgacaac

cacagaggagggataccggatgctctttcaggacctggagaagaaaaaggagcggctggtggtggtggactgtgaatcagaacgc

ctcaatgctatcttgggccagattataaagctagagaagaatggcatcggctaccactacattcttgcaaatctgggcttcatggacatt

gacttaaacaaaattcaaggagagtggcgccaatgtgacaggtttccagctggtgaactacacagacactattccggccaagatcatgc

agcagtggaagaatagtgatgctcgagaccacacacgggtggactggaagagacccaagtacacctctgcgctcacctacgatggg

gtgaaggtgatggctgaggctttccagagcctgcggaggcagagaattgatatatctcgccggggggaatgctggggattgtctggct

aacccagctgttccctggggccaagggatcgacatccagagagctctgcagcaggtgcgatttgaaggtttaacaggaaacgtgcag

tttaatgagaaaggacgccggaccaactacacgctccacgtgattgaaatgaaacatgacggcatccgaaagattggttactggaatg

aagatgataagtttgtccctgcagccaccgatgcccaagctgggggcgataattcaagtgttcagaacagaacatacatcgtcacaac

aatcctagaagatccttatgtgatgctcaagaagaacgccaatcagtttgagggcaatgaccgttacgagggctactgtgtagagctg

gcggcagagattgccaagcacgtgggctactcctaccgtctggagattgtcagtgatggaaaatacggagcccgagaccctgacac

gaaggcctggaatggcatggtgggagagctggtctatggaagagcagatgtggctgtggctcccttaactatcactttggtccggga

agaagttatagatttctccaaaccatttatgagtttgggggatctccatcatgattaaaaaaccacagaaatccaagccgggtgtcttctc

cttccttgatcctttggcttatgagatttggatgtgcattgttttttgcctacattggagtgagtgttgtcctcttcctggtcagccgcttcag

tccctatgaatggcacagtgaagagtttgaggaaggacgggaccagacaaccagtgaccagtccaatgagtttgggatattcaacag

tttgtggttctccctgggagccttcatgcagcaaggatgtgacatttctcccaggtccctgtctggtcgcatcgttggtggcgtctggtg

gttcttcaccttaatcatcatctcctcatatacagccaatctggccgccttcctgaccgtggagaggatggtgtctcccattgagagtgc

agaggacctagcgaagcagacagaaattgcctacgggacgctggaagcaggatctactaaggagttcttcaggaggtctaaaattgc

tgtgtttgagaagatgtggacatacatgaagtcagcagagccatcagtttttgtgcggaccacagaggaggggatgattcgagtgag

gaaatccaaaggcaaatatgcctacctcctggagtccaccatgaatgagtacattgagcagcggaaaccctgtgacaccatgaaggt

gggaggtaacttggattccaaaggctatggcattgcaacacccaaggggtctgccctgagaaatccagtaaacctggcagtgttaaa

actgaacgagcaggggctttggacaaattgaaaaacaaatggtggtacgacaagggcgagtgcggcagcggggggaggtgattcc

aaggacaagacaagcgctctgagcctcagcaatgtggcaggcgtgttctacatcctgatcggaggacttggactagccatgctggtt

gccttaatcgagttctgctacaaatcccgtagtgaatccaagcggatgaaggggtttttgtttgatcccacagcaatccatcaacgaagc

catacggacatcgaccctcccccgcaacagcggggcaggagccagcagcggcggcagtggagagaatggtcgggtggtcagccat

gacttccccaagtccatgcaatcgattccttgcatgagccacagttcagggatgcccttgggagccacgggattgtaa

>SEQ ID NO: 5

MQHIFAFFCTGFLGAVVGANFPNNIQIGGLFPNQQSQEHAAFRFALSQLTEPPKLLPQ
IDIVNISDSFEMTYRFCSQFSKGVYAIFGFYERRTVNMLTSFCGALHVCFITPSFPVDTS
NQFVLQLRPELQDALISIIDHYKWQKFVYIYDADRGLSVLQKVLDTAAEKNWQVTAV
NILTTTEEGYRMLFQDLEKKKERLVVVDCESERLNAILGQIIKLEKNGIGYHYILANLG
FMDIDLNKFKESGANVTGFQLVNYTDTIPAKIMQQWKNSDARDHTRVDWKRPKYTS

ALTYDGVKVMAEAFQSLRRQRIDISRRGNAGDCLANPAVPWGQGIDIQRALQQVRFE
GLTGNVQFNEKGRRTNYTLHVIEMKHDGIRKIGYWNEDDKFVPAATDAQAGGDNSS
VQNRTYIVTTILEDPYVMLKKNANQFEGNDRYEGYCVELAAEIAKHVGYSYRLEIVSD
GKYGARDPDTKAWNGMVGELVYGRADVAVAPLTITLVREEVIDFSKPFMSLGISIMIK
KPQKSKPGVFSFLDPLAYEIWMCIVFAYIGVSVVLFLVSRFSPYEWHSEEFEEGRDQTT
SDQSNEFGIFNSLWFSLGAFMQQGCDISPRSLSGRIVGGVWWFFTLIIISSYTANLAAFL
TVERMVSPIESAEDLAKQTEIAYGTLEAGSTKEFFRRSKIAVFEKMWTYMKSAEPSVFV
RTTEEGMIRVRKSKGKYAYLLESTMNEYIEQRKPCDTMKVGGNLDSKGYGIATPKGS
ALRNPVNLAVLKLNEQGLLDKLKNKWWYDKGECGSGGGDSKDKTSALSLSNVAGVF
YILIGGLGLAMLVALIEFCYKSRSESKRMKGFCLIPQQSINEAIRTSTLPRNSGAGASSG
GSGENGRVVSHDFPKSMQSIPCMSHSSGMPLGATGL

>SEQ ID NO: 6

atggccaggcagaagaaaatggggcaaagcgtgctccgggcggtcttcttttttagtcctggggctttttgggtcattctcacggaggat
tccccaacaccatcagcataggtggacttttcatgagaaacacagtgcaggagcacagcgctttccgctttgccgtgcagttatacaac
accaaccagaacaccaccgagaagcccttccatttgaattaccacgtagatcacttggattcctccaatagttttccgtgacaaatgct
ttctgctcccagttctcgagagggggtgtatgccatctttggattctatgaccagatgtcaatgaacaccctgacctccttctgtggggcc
ctgcacacatcctttgttacgcctagcttccccactgacgcagatgtgcagtttgtcatccagatgcgcccagccttgaagggcgctatt
ctgagtcttctgggtcattacaagtggggagaagtttgtgtacctctatgacacagaacgaggattttccatcctccaagcgattatggaa
gcagcagtgcaaaacaactggcaagtaacagcaaggtctgtgggaaacataaaggacgtccaagaattcaggcgcatcattgaaga
aatggacaggaggcaggaaaagcgatacttgattgactgcgaagtcgaaaggattaacacaattttggaacaggttgtgatcctagg
gaaacactcaagaggttatcactacatgctcgctaacctgggtttttactgatattttactggaaagagtcatgcatggggggagccaaca
ttacaggtttccagattgtcaacaatgaaaaccctatggttcagcagttcatacagcgctgggtgaggctggatgaaagggaattccct
gaagccaagaatgcaccactaaagtatacatctgcattgacacacgacgcaatactggtcatagcagaagctttccgctacctgagga
ggcagcgagtagatgtgtcccggagaggaagtgctggagactgcttagcaaatcctgctgtgccctggagtcaaggaattgatattg
agagagctctgaaaatggtgcaagtacaaggaatgactggaaatattcaatttgacacttatggacgtaggacaaattataccatcgat
gtgtatgaaatgaaagtcagtggctctcgaaaagctggctactggaatgagtatgaaaggtttgtgcctttctcagatcagcaaatcag
caatgacagtgcatcctcagagaatcggaccatagtagtgactaccattctggaatcaccatatgtaatgtacaagaagaaccatgag
caactggaaggaaatgaacgatatgaaggctattgtgtagacctagcctatgaaatagccaaacatgtaaggatcaaatacaaattgt
ccatcgttggtgacgggaaatatggtgcaagggatccagagactaaaatatggaacggcatggttggggaacttgtctatgggagag
ctgatatagctgttgctccactcactataacattggtccgtgaagaagtcatagatttttcaaagccattcatgagcctgggcatctccat
catgataaagaagcctcagaaatcaaaaccaggcgtattctcatttctggatcccctggcttatgaaatctggatgtgcattgtctttgct
tacattggagtcagcgtagttctttttcctagtcagcaggttcagtccttatgaatggcacttggaagacaacaatgaagaacctcgtgac
ccacaaagtcctcctgatcctccaaatgaatttggaatatttaacagtctttggttttccttgggtgcctttatgcagcaaggatgtgatat
ttctccaagatcactctccgggcgcattgttggagggggtttggtggttcttcaccctgatcataatttcttcctatactgccaatctcgctg
ctttcctgactgtggagaggatggtttctcccatagagagtgctgaagacttagctaaacagactgaaattgcatatgggaccctggac

tccggttcaacaaaagaattttttcagaagatccaaaattgctgtgtacgagaaaatgtggtcttacatgaaatcagcggagccatctgt

gtttaccaaaacaacagcagacggagtggcccgagtgcgaaagtccaagggaaagttcgccttcctgctggagtcaaccatgaatga

gtacattgagcagagaaaaccatgtgatacgatgaaagttggtggaaatctggattccaaaggctatggtgtggcaacccctaaagg

ctcagcattaagaacgcctgtaaaccttgcagtattgaaactcagtgaacaaggcatcttagacaagctgaaaaacaaatggtggtac

gataagggggaatgtggagccaaggactccgggagtaaggacaagaccagcgctctgagcctgagcaatgtggcaggcgttttcta

tatacttgtcggaggtctgggggctggccatgatggtggcttttgatagaattctgttacaaatcacgggcagagtccaaacgcatgaaac

tcacaaagaacacccaaaactttaagcctgctcctgccaccaacactcagaattatgctacatacagagaaggctacaacgtgtatgg

aacagagagtgttaagatctag

>SEQ ID NO: 7

MARQKKMGQSVLRAVFFLVLGLLGHSHGGFPNTISIGGLFMRNTVQEHSAFRFAVQL
YNTNQNTTEKPFHLNYHVDHLDSSNSFSVTNAFCSQFSRGVYAIFGFYDQMSMNTLT
SFCGALHTSFVTPSFPTDADVQFVIQMRPALKGAILSLLGHYKWEKFVYLYDTERGFSI
LQAIMEAAVQNNWQVTARSVGNIKDVQEFRRIIEEMDRRQEKRYLIDCEVERINTILE
QVVILGKHSRGYHYMLANLGFTDILLERVMHGGANITGFQIVNNENPMVQQFIQRW
VRLDEREFPEAKNAPLKYTSALTHDAILVIAEAFRYLRRQRVDVSRRGSAGDCLANPA
VPWSQGIDIERALKMVQVQGMTGNIQFDTYGRRTNYTIDVYEMKVSGSRKAGYWNE
YERFVPFSDQQISNDSASSENRTIVVTTILESPYVMYKKNHEQLEGNERYEGYCVDLAY
EIAKHVRIKYKLSIVGDGKYGARDPETKIWNGMVGELVYGRADIAVAPLTITLVREEVI
DFSKPFMSLGISIMIKKPQKSKPGVFSFLDPLAYEIWMCIVFAYIGVSVVLFLVSRFSPYE
WHLEDNNEEPRDPQSPPDPPNEFGIFNSLWFSLGAFMQQGCDISPRSLSGRIVGGVW
WFFTLIIISSYTANLAAFLTVERMVSPIESAEDLAKQTEIAYGTLDSGSTKEFFRRSKIAV
YEKMWSYMKSAEPSVFTKTTADGVARVRKSKGKFAFLLESTMNEYIEQRKPCDTMKV
GGNLDSKGYGVATPKGSALRTPVNLAVLKLSEQGILDKLKNKWWYDKGECGAKDSG
SKDKTSALSLSNVAGVFYILVGGLGLAMMVALIEFCYKSRAESKRMKLTKNTQNFKPA
PATNTQNYATYREGYNVYGTESVKI

>SEQ ID NO: 8

atgaggattatttccagacagattgtcttgttattttctggattttggggactcgccatgggagcctttccgagcagcgtgcaaataggt

ggtctcttcatccgaaacacagatcaggaatacactgcttttcgattagcaattttttcttcataacaccagccccaatgcgtcggaagct

ccttttaatttggtacctcatgtggacaacattgagacagccaacagtttttgctgtaacaaacgccttctgttcccagtattctagaggag

tatttgccatttttggactctatgataagaggtcggtacataccttgacctcattctgcagcgccttacatatctccctcatcacaccaagt

ttccctactgaggggggagagccagtttgtgctgcaactaagaccttcgttacgaggagcactcttgagtttgctggatcactacgaatg

gaactgttttgtcttcctgtatgacacagacaggggatactcgatactccaagctattatggaaaaagcaggacaaatggttggcatg

tcagcgctatatgtgtggaaaattttaatgatgtcagctataggcaacttctagaagaacttgacagaagacaagagaagaagtttgta

atagactgtgagatagagagacttcaaaacatattagaacagattgtaagtgttggaaaagcatgttaaaggctaccattatatcattgca

aacttgggattcaaggatatttctcttgagaggtttatacatggtggagccaatgttactggattccagttggtggattttaatacacctat

ggtaatcaaactaatggatcgctggaagaaactagatcagagagagtatccaggatctgagactcctccaaagtacacctctgctctg

acttatgatggagtccttgtgatggctgaaactttccgaagtcttaggaggcagaaaattgatatctcaaggagaggaaatgctgggg

attgtctggcaaatcctgctgctccatgggggccagggaattgacatggagaggacactcaaacaggttcgaattcaagggctgacag

ggaatgttcagtttgaccactatggacgtagagtcaattacacaatggatgtgtttgagctgaaaagcacaggacctagaaaggttggt

tactggaatgatatggataagttagtcttgattcaagatgtaccaactcttggcaatgacacagctgctattgagaacagaacagtggtt

gtaaccacaattatggaatccccatatgttatgtacaagaaaaatcatgaaatgtttgaaggaaatgacaagtatgaaggatactgtgt

agatttggcatctgaaattgcaaaacatattggtatcaagtataaaattgccattgtccctgatggaaaatatggagcaagggatgcag

acacaaaaatctggaatgggatggtaggagaacttgtttatgggaaagcagagattgctattgcccctctgacaatcactttggtacga

gaggaggtcattgacttttctaagcccttcatgagtttgggcatatctatcatgatcaaaaagcctcagaaatccaaaccaggagtgttt

tccttcttggatcctctggcctatgagatttggatgtgcatagtctttgcctacattggtgtcagcgtggtcttattcctagttagtagattt

agtccatatgagtggcacacagaagagccagaggacggaaaggaaggacccagcgaccagcctcccaatgagtttggcatctttaa

cagcctctggttttccctgggtgcttttatgcagcaaggatgtgacatttcacccagatccctctcaggtcgaattgttggaggtgtttgg

tggttctttacactcatcattatatcatcttatactgctaacctcgctgctttcctgacggttgagcgaatggtctctcccatagaaagtgc

agaagacctggccaaacaaacagaaattgcctatggaacactggattcaggatcaacaaaagaattcttcagaagatcaaaaatagc

agtgtatgaaaagatgtggacctacatgcgatcagcagagccatcagtattcactaggactacagctgagggagtagctcgtgtccgc

aaatccaagggcaaatttgcctttctcctggagtccactatgaatgaatacattgagcagcgaaagccatgtgacacgatgaaagtgg

gaggaaatctggattccaaaggctatggagtagcaacgcccaagggttcctcattaagaactcctgtaaaccttgccgttttgaaactc

agtgaggcaggcgtcttagacaagctgaaaaacaaatggtggtacgataaaggtgaatgtggacccaaggactctggaagcaagga

caagacgagtgccttgagcctgagcaatgtagcaggcgtcttctacattctggttggcggcttgggcttggcaatgctggtggctttga

tagagttctgttacaagtccagggcagaagcgaagagaatgaagctgaccttttctgaagccataagaaacaaagccagattatccat

cactgggagtgtgggagagaatggccgcgtcttgacgcctgactgcccaaaggctgtacacactggaactgcaatcagacaaagttc

aggattggctgtcattgcatcggacctaccataa

**Claims**

1. A pharmaceutical composition for use in treating a subject having overweight or obesity, comprising an AMPA receptor antagonist.

2. The pharmaceutical composition according to claim 1, for delaying, inhibiting, or arresting a development of overweight or obesity in a subject having overweight or obesity.

3. The pharmaceutical composition according to claim 1, for use in reducing body weight in a subject having overweight or obesity.

4. The pharmaceutical composition according to claim 1 or 3, for use in reducing food intake in a subject having overweight or obesity.

5. The pharmaceutical composition according to any one of claims 1 to 4, for use in inhibiting an incidence of diabetes, dyslipidemia, atherosclerosis, and cardiovascular disease in a subject having overweight or obese.

6. The pharmaceutical composition according to any one of claims 1 to 4, wherein the AMPA receptor antagonist is perampanel.

# FIG. 1-1

# FIG. 1-2

**\*\*:p<0.01;\*:p<0.05**

## FIG. 1-3

# FIG. 2-1

FIG. 2-2

# FIG. 3

# FIG. 4

# FIG. 5

# FIG. 6

# FIG. 7

# FIG. 8

# FIG. 9

# FIG. 10-1

# FIG. 10-2

B  *ob/ob* plus PER mouse

C  *HFD* plus PER mouse

## FIG. 11

## FIG. 12

# FIG. 13

# FIG. 14

# FIG. 15

# FIG. 16

# FIG. 17

## FIG. 18

## FIG 19

# FIG. 20

# FIG. 21

## FIG. 22

A

B

C

D

## FIG. 23

# FIG. 24

Wt 12w
litter mouse of *obob*

*obob* 12w
with PER treatment for 4w

*obob* 12w

# FIG. 25

CHANGE IN BODY WEIGHT

—▲—ob/ob (N=5)
—●—ob/ob with PER (N=5)

\*:p<0.05

FOOD INTAKE

—●—ob/ob(n=5)
—●—ob/ob with PER (n=5)

## FIG. 26

BEFORE PER
ADMINISTRATION

AFTER PER
ADMINISTRATION

## FIG. 27

# FIG. 28

## FIG. 29

A

**2DIV ratio Aβ42 /40**

B

**4DIV ratio Aβ42 /40**

## FIG. 30

100 μm

# FIG. 31

FIG. 32

# FIG. 33

A **NL homo with PER**

B **CP-AMPA**

C

*:p<0.05

# FIG. 34

## Novel object

Legend:
- ☐ Familiar oblect
- ▨ **Novel object**

| | |
|---|---|
| control | n=5 |
| mema | n=5 |
| exercise | n=4 |
| mema+exercise | n=5 |
| PER+mema | n=4 |
| PER  5mg/ kg | n=5 |
| PER 10mg/kg | n=4 |

*P<0.05

# FIG. 35

## Fear conditioning

** p<0.01

| | |
|---|---|
| control | n=5 |
| mema | n=4 |
| exercise | n=4 |
| mema+exercise | n=5 |
| PER+mema | n=4 |
| PER  5mg/kg | n=7 |
| PER 10mg/kg | n=4 |

<table>
<tr><td colspan="2" align="center">**INTERNATIONAL SEARCH REPORT**</td><td>International application No.<br><br>**PCT/JP2022/034601**</td></tr>
</table>

**A.   CLASSIFICATION OF SUBJECT MATTER**

*A61K 45/00*(2006.01)i; *A61P 3/04*(2006.01)i; *A61K 31/444*(2006.01)i
FI:   A61K45/00; A61K31/444; A61P3/04

According to International Patent Classification (IPC) or to both national classification and IPC

**B.   FIELDS SEARCHED**

Minimum documentation searched (classification system followed by classification symbols)

A61K45/00; A61P3/04; A61K31/444

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C.   DOCUMENTS CONSIDERED TO BE RELEVANT**

| Category* | Citation of document, with indication, where appropriate, of the relevant passages | Relevant to claim No. |
|---|---|---|
| X | US 6191117 B1 (KOZACHUK, Walter, E.) 20 February 2001 (2001-02-20)<br>      claims, column 1, lines 14-18, column 2, lines 3-5 | 1-5 |
| Y | | 6 |
| Y | JP 2010-520154 A (EISAI R & D MANAGEMENT CO., LTD.) 10 June 2010 (2010-06-10)<br>      paragraphs [0054]-[0055], [0077] | 6 |
| Y | JP 2008-538216 A (EISAI R & D MANAGEMENT CO., LTD.) 16 October 2008<br>(2008-10-16)<br>      claims | 6 |

☐ Further documents are listed in the continuation of Box C.          ☑ See patent family annex.

| | |
|---|---|
| *      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **28 October 2022** | **08 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/034601**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    The "form of an Annex C/ST.25 text file" is replaced with the "form of ST.26."

Form PCT/ISA/210 (continuation of first sheet) (January 2015)

<div align="center">

**INTERNATIONAL SEARCH REPORT**
Information on patent family members

</div>

| | International application No. |
|---|---|
| | **PCT/JP2022/034601** |

| Patent document cited in search report | | | Publication date (day/month/year) | Patent family member(s) | | | Publication date (day/month/year) |
|---|---|---|---|---|---|---|---|
| US | 6191117 | B1 | 20 February 2001 | WO | 2002/003915 | A2 | |
| | | | | EP | 1305034 | A2 | |
| JP | 2010-520154 | A | 10 June 2010 | US | 2010/0099714 | A1 | |
| | | | | paragraphs [0077]-[0078], [0100] | | | |
| | | | | WO | 2008/111590 | A2 | |
| | | | | EP | 2131828 | A2 | |
| JP | 2008-538216 | A | 16 October 2008 | US | 2006/0270709 | A1 | |
| | | | | claims | | | |
| | | | | WO | 2006/107859 | A2 | |
| | | | | EP | 1871368 | A2 | |
| | | | | KR | 10-2008-0007233 | A | |
| | | | | CN | 101511186 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6949571 B
- WO 9610023 A
- WO 2003082332 A
- WO 9512594 A
- WO 817652 A
- WO 9732858 A
- WO 9906408 A
- US 5639751 A
- WO 9615100 A
- EP 283959 A
- WO 9521842 A
- WO 9907707 A
- WO 9207847 A
- US 2017015851 W

**Non-patent literature cited in the description**

- **PATERNAIN et al.** *Neuron,* 1995, vol. 14, 185-189
- *Neuropharmacology,* 1998, vol. 37, 1211-1222
- *Neuropharmacology,* 2011, vol. 61 (5-6), 1033-47
- **SAITO et al.** Single App knock-in mouse models of Alzheimer's disease. *Nat. Neurosci.,* 2014, vol. 17, 661-4
- *Nat. Neurosci.,* 2014, vol. 17, 661-4
- *Curr Opin Neurol,* 2021, vol. 1, 266-274
- **DOECKE JD et al.** *Neurology,* 2020
- **BREWER GJ et al.** *Nat Protoc,* 2007, vol. 2, 1490-1498
- **LAM AD et al.** *Nat Med,* 2017
- **HOKAMA Y et al.** *Neuro Oncology,* 2022
- **E. E. NOBLE ; S. L. KANOSKI.** *Curr. Opin. Behav. Sci.,* 2016, vol. 9, 7-14
- **S. L. HARGRAVE ; S. JONES ; T. L. DAVIDSON.** *Curr. Opin. Behav. Sci.,* 2016, vol. 9, 40-46
- **L-L. HWANG ; C-H. WANG ; T-L. LI ; S-D. CHANG ; L-C. LIN ; C-P. CHEN ; C-T. CHEN ; K-C. LIANG ; I-K. HO ; W-S. YANG.** *Obesity (Silver Spring),* 2010, vol. 18, 463-469
- **I. VALLADOLID-ACEBES ; P. STUCCHI ; V. CANO ; M. S. FEMÁNDEZ-ALFONSO ; B. MERINO ; M. GIL-ORTEGA ; A. FOIE ; L. MORALES ; M. RUIZ-GAYO ; N. D. OLMO.** *Neurobiol. Learn. Mem.,* 2011, vol. 95, 80-85
- **M. FOTUHI ; D. DO ; C. JACK.** *Nat. Rev. Neurol.,* 2012, vol. 8, 189-202
- **N. D. OLMO ; M. RUIZ-GAYO.** *Front. Cell. Neurosci.,* 2018, vol. 12, 439
- **M. ANAI ; M. FUNAKI ; T. OGIHARA ; A. KANDA ; Y. ONISHI ; H. SAKODA ; K. INUKAI ; M. NAWANO ; Y. FUKUSHIMA ; Y. YAZAKI.** *Diabetes,* 1999, vol. 48, 158-169
- **S. HIBI ; K. UENO ; S. NAGATO ; K. KAWANO ; K. ITO ; Y. NORIMINE ; O. TAKENAKA ; T. HANADA ; M. YONAGA.** *J. Med. Chem.,* 2012, vol. 55, 10584-10600
- **A. ROZOV ; N. BUMASHEV.** *Cell Calcium,* 2016, vol. 60, 407-414
- **S. ISHIUCHI ; K. TSUZUKI ; Y. YOSHIDA ; N. YAMADA ; N. HAGIMURA ; H. OKADO ; A. MIWA ; H. KURIHARA ; Y. NAKAZATO ; M. TAMURA.** *Nat. Med.,* 2002, vol. 8, 971-978
- **M. AKAMATSU ; T. YAMASHITA ; N. HIROSE ; S. TERAMOTO ; S. KWAK.** *Sci. Rep.,* 2016, vol. 6, 28649
- **K. L. CONRAD ; K. Y. TSENG ; J. L. UEJIMA ; J. M. REIMERS ; L-J. HENG ; Y. SHAHAM ; M. MARINELLI ; M. E. WOLF.** *Nature,* 2008, vol. 454, 118-121
- **R. K. KHANGURA ; A. BALI ; A. S. JAGGI.** *Eur. J. Pharmacol.,* 2017, vol. 795, 36-42
- **J. A. FRENCH ; C. L. KRAUSS ; R. T. WECHSLER ; X-F. WANG ; B. DIVENTURA ; C. BRANDT ; E. TRINKA ; T. J. O BRIEN ; A. LAURENZA ; A. PATTEN.** *Neurology,* 2015, vol. 85, 950-957
- **S. CULL-CANDY ; L. KELLY ; M. FARRANT.** *Curr. Opin. Neurobiol.,* 2006, vol. 16, 288-297
- **S. J. LIU ; R. S. ZUKIN.** *Trends Neurosci.,* 2007, vol. 30, 126-134
- **S. OZAWA.** *J. Physiol.,* 2009, vol. 587, 1861-1862
- **E. IWAMURA ; K. YAMADA ; Y. ICHITANI.** *Behav. Brain Res.,* 2016, vol. 307, 92-99
- **F. L. HITTI ; S.A. SIEGELBAUM.** *Nature,* 2014, vol. 508, 88-92
- **B. MEI ; F. LI ; Y. GU ; Z. CUI ; J. Z. TSIEN.** *PLoS One,* 2011, vol. 6, e19326
- **C. PORRERO ; P. RUBIO-GARRIDO ; C. AVENDAÑO ; F. CLASCÁ.** *Brain Res.,* 2010, vol. 1345, 59-72

- K. CHUNG ; J. WALLACE ; S-Y. KIM ; S. KATYANASUNDAMM ; A. S. ANDALMAN ; T. J. DAVIDSON ; J. J. MIRZABEKOV ; K. A. ZALOCUSKY ; J. MATTIS ; A. K. DENεSIN. *Nature,* 2013, vol. 497, 332-337
- Y. ZHANG ; R. PROENCA ; M. MAFFEI ; M. BARONE ; L. LEOPOLD ; J. M. FRIEDMAN. *Nature,* 1994, vol. 372, 425-432
- C. T. MONTAGUE ; I. S. FAROOQI ; J. P. WHITEHEAD ; M.A. SOOS ; H. RAU ; N. J. WAREHAM ; C. P. SEWTER ; J. E. DIGBY ; S. N. MOHAMMED ; J. A. HURST. *Nature,* 1997, vol. 387, 903-908
- L. J. SHANLEY ; A. J. IRVING ; J. HARVEY. *J. Neurosci.,* 2001, vol. 21, RC186
- R. MAZOR ; D. FRIEDMANN-MORVINSKI ; T. ALSAIGH ; O. KLEIFELD ; E. B. KISTLER ; L. ROUSSO-NOORI ; C. HUANG ; J. B. LI ; I. M. VERMA ; G. W. SCHMID-SCHÖNBEIN. *Sci. Transl. Med.,* 2018, vol. 10, 6324
- R. BRUSA ; F. ZIMMERMANN ; D.-S. KOH ; D. FELDMEYER ; P. GASS ; P. N. SEEBURG ; R. SPRENGEL. *Science,* 1995, vol. 270, 1677-1680
- S. J. SAWIAK ; N.I. WOOD ; G. E3. WILLIAMS ; A. J. MORTON ; T. A. CARPENTER. *Neurobiol. Dis.,* 2009, vol. 33, 20-27
- G.J. PAZ-FILHO. *NeuralPlast.,* 2016
- A. V. WITTE ; T. KÖBE ; A. GRAUNKE ; J. P. SCHUCHARDT ; A. HAHN ; V. A. TESKY ; J. PANTEL ; A. FLÖEL. *Hum. Brain Mapp.,* 2016, vol. 37, 4539-4549
- A. E. DORR ; J. P. LERCH ; S. SPRING ; N. KABANI ; R. M. HENKELMAN. *Neuroimage,* 2008, vol. 42, 60-69
- A. SHIROMA ; M. NISHIMURA ; II. NAGAMINE ; T. MIYAGI ; Y. HOKAMA ; T. WATANABE ; S. MURAYAMA ; M. TSUTSUI ; D. TOMINAGA ; S. ISHIUCHI. *Cerebellum,* 2016, vol. 15, 645-662
- R. USUGI ; M. NISHIMURA ; S. ISHIUCHI. *Brain Behav.,* 2020, vol. 10, e01878
- J.F. GUZOWSKI ; J. J. KNIERIM ; E. I. MOSER. *Neuron,* 2004, vol. 44, 581-584
- T. SASAKI ; S. MATSUI ; T. KITAMURA. *Int. J. Mol. Sci.,* 2016, vol. 17, 1081
- C. LÜSCHER ; R. C. MALENKA. *Cold Spring Harb. Perspect. Biol.,* 2012, vol. 4, a005710
- S. E. KANOSKI ; T. L. DAVIDSON. *Physiol. Behav.,* 2011, vol. 103, 59-68
- L. B. MASSING ; A. K. DAHL ; N. L. PEDERSEN ; B. JOHANSSON. *Dement. Geriatr. Cogn. Disord.,* 2010, vol. 29, 543-552
- W. JAGUST ; D. HARVEY ; D. MUNGAS ; M. HAAN. *Arch. Neurol.,* 2005, vol. 62, 1545-1548
- K. F. YATES ; V. SWEAT ; P. L. YAU ; M. M. TURCHIANO ; A. CONVIT. *Arterioscler. Thromb. Vase. Biol.,* 2012, vol. 32, 2060-2067
- P. G. KOPELMAN. *Nature,* 2000, vol. 404, 635-643
- B. RASHID ; S. I. DEV ; M. NICOLETTE ; F. SCHWARZ ; T. FERLAND ; F. C. FORTENBAUGH ; W. P. MILBERG ; R. E. MCGLINCHEY ; D. H. SALAT ; F. C. LERITZ. *Brain Behav.,* 2019, vol. 9, e01333
- M. GESERICK ; M. VOGEL ; R. GAUSCHE ; T. LIPEK ; U. SPIELAU ; E. KELLER ; R. PFÄFFLE ; W. KIESS ; A. KÖRNER. *J. Med.,* 2018, vol. 379, 1303-1312
- D. C. OU-YANG ; P. J. YORK ; C. J. KLECK ; V. V. PATEL. *J. Bone Joint Surg. Am.,* 2017, vol. 99, 2027-2036
- T. YONEKAWA ; M. C. V. MALICDAN ; A. CHO ; Y. K. HAYASHI ; I. NONAKA ; T. MINE ; T. YAMAMOTO ; I. NISHINO ; S. NOGUCHI. *Brain,* 2014, vol. 137, 2670-2679
- H. AGETA ; A. MURAYAMA ; R. MIGISHIMA ; S. KIDA ; K. TSUCHIDA ; M. YOKOYAMA ; K. IN-OKUCHI. *PLoS one,* 2008, vol. 3, 1869
- T-N. HUANG ; Y. P. HSUEH. *Bioprotocol,* 2014, vol. 4 (19
- T. TODA ; Y. NODA ; G. ITO ; M. MAEDA ; T. SHIMI-ZU. *J. Biomed. Biotechnol.,* 2011, vol. 617974
- T. NAKASHIBA ; J. D. CUSHMAN ; K.A. POLKEY ; C. J. MCBAIN ; M. S. FANSELOW ; S. TON-EGAWA. *Cell,* 2012, vol. 149, 188-201
- W. B. CHWANG ; K. J. O ' RIORDAN ; J. M. LEVENSON ; J. D. SWEATT. *Learn. Mem.,* 2006, vol. 13, 322-328
- J. P. P. ULLMANN ; C. WATSON ; A. L. JANKE ; N. D. KURNIAWAN ; D. C. REUTENS. *Neuroimage,* 2013, vol. 78, 196-203
- T.L. RICHARDS ; T. J. GRABOWSKI ; P. BOORD ; K. YAGLE ; M. ASKREN ; Z. MESTRE ; P. ROBINSON ; O. WELKER ; D. GULLIFORD ; W. NAGY. *Neuroimage Clin.,* 2015, vol. 8, 408-421
- S. WHITFIELD-GABRIELI ; A. NIETO-CASTANON. *Brain Connect.,* 2012, vol. 2, 125-141
- M. RUBINOV ; O. SPORNS. *Neuroimage,* 2010, vol. 52, 1059-1069
- M. XIA ; J. WANG ; Y. HE. *PLoS One,* 2013, vol. 8, e68910
- I. YAO ; H. TAKAGI ; H. AGETA ; T. KAL)YO ; S. SATO ; K. HATANAKA ; Y. FUKUDA ; T. CHIBA ; N. MORONE ; S. YUASA. *Cell,* 2007, vol. 130, 943-957
- H. TAKAGI ; M. SETOU ; S. ITO. *Yao. Neural. Plast.,* 2012, vol. 1, 352829
- L. NOWAK ; P. BREGESTOVSKI ; P. ASCHER ; A. HERBET ; A. PROCHIANTZ. *Nature,* 1984, vol. 307, 462-465

- **M. KOIKE ; M. IINO ; S. OZAWA.** *Neurosci. Res.,* 1997, vol. 29, 27-36
- **A.C. BOUDREAU ; J. M. REIMERS ; M. MILOVANOVIC ; M. E. WOLF.** *J. Neurosci.,* 2007, vol. 27, 10621-10635
- **H. LOWRY ; N. J. ROSEBROUGH ; A. L. FARR ; R. J. RANDALL.** *J. Biol. Chem.,* 1951, vol. 193, 265-275
- **J. B. TREWEEK ; K. Y. CHAN ; N. C. FLYTZANIS ; B. YANG ; B. E. DEVERMAN ; A. GREENBAUM ; A. LIGNELL ; C. XIAO ; L. CAI ; M. S. LADINSKY.** *Nat. Protoc.,* 2015, vol. 10, 1860-1896
- **G. FONG ; R. H. MELLOR ; M. BERNSTEIN ; C. KELLER-PECK ; Q. T. NGUYEN ; M. WALLACE ; J. M. NERBONNE ; J. W. LICHTMAN ; J. R. SANES.** *Neuron,* 2000, vol. 28, 41-51
- **C. PORRERO ; P. RUBIO-GARRIDO ; C. AVENDAÑO ; F. CLASCÁ.** *Brain Res.,* 2010, vol. 1345, 59-72
- **D. KIM ; J. M. PAGGI ; C. PARK ; C. R. BENNETT ; S. L. SALZBERG.** *Nat Biotechnol.,* 2019, vol. 37, 907-915
- **R. PATRO ; G. DUGGAL ; M. I. LOVE ; R. A. IRIZARRY ; C. KINGSFORD.** *Nat Methods,* 2017, vol. 14, 417-419
- **M. PERTEA ; G. M. PERTEA ; C. M. ANTONESCU ; T. C. CHANG ; J. T. MENDELL ; S. L. SALZBERG.** *Nat Biotechnol.,* 2015, vol. 33, 290-295
- **M. A. DEPRISTO ; E. BANKS ; R, POPLIN ; K. V. GARIMELLA ; J. R. MAGUIRE ; C. HART1 ; A. A. PHILIPPAKIS ; G. DEL ANGEL ; M. A. RIVAS ; M. HANNA.** *Nat. Genet.,* 2011, vol. 43, 491-498
- **G.A. VAN DER AUWERA ; M. O. CARNEIRO ; C. HARTL ; R. POPLIN ; G. DEL ANGEL. ; A. LEVY-MOONSHINE ; T. JORDAN. ; K. SHAKIR ; D. ROAZEN ; J. THIBAULT.** *Curr. Protoc. Bioinformatics,* 2013, vol. 43
- **A. MCKENNA ; M. HANNA ; E. BANKS ; A. SIVACHENKO ; K. CIBULSKIS ; A. KERNYTSKY ; K. GARIMELLA ; D. ALTSHULER ; S. GABRIEL ; M. DALY.** *Genome Res,* 2010, vol. 20, 1297-1303
- **P. CINGOLANI ; A. PLATTS ; L. L. WANG ; M. COON ; T. NGUYEN ; L. WANG ; S. J. LAND ; X. LU ; D. M. RUDEN.** *Fly,* 2012, vol. 6, 80-92
- **K. A. YAMADA.** *Neurobiology of Disease,* 1998, vol. 5, 67-80